# EUROPÄISCHE PATENTSCHRIFT

(11) **EP 1 419 259 B1**
(45) Veröffentlichungstag und Bekanntmachung des Hinweises auf die Patenterteilung: **17.07.2013**
(21) Anmeldenummer: 02764846.8
(22) Anmeldetag: 13.08.2002
(51) Int. Cl.: C12N 15/10, C12N 15/67

(54) **VERFAHREN ZUR REPARATUR EINER MUTIERTEN RNA AUS EINER GENDEFEKTEN DNA UND ZUM GEZIELTEN ABTÖTEN VON TUMORZELLEN DURCH RNA-TRANSSPLEISSEN SOWIE VERFAHREN ZUM NACHWEIS VON NATÜRLICH-TRANSGESPLEISSTER ZELLULÄRER RNA**
METHOD FOR REPAIRING A MUTANT RNA FROM A DNA WITH GENETIC DEFECTS AND FOR THE PROGRAMMED DEATH OF TUMOUR CELLS BY RNA TRANS-SPLICING AS WELL AS A METHOD FOR IDENTIFYING NATURALLY TRANS-SPLICED CELLULAR RNA
PROCEDE DESTINE A LA REPARATION D'UN ARN MUTANT, A PARTIR D'UN ADN PRESENTANT DES DEFAUTS GENETIQUES, ET A LA MORT PROGRAMMEE DE CELLULES TUMORALES PAR TRANS-EPISSAGE D'ARN ET PROCEDE D'IDENTIFICATION D'ARN CELLULAIRE NATURELLEMENT TRANS-EPISSE

(30) Priorität: 13.08.2001 DE 10139492
(43) Veröffentlichungstag der Anmeldung: 19.05.2004
(73) Patentinhaber: Trans RNA Technology Unternehmergesellschaft (haftungsbeschränkt), 14163 Berlin (DE)
(72) Erfinder: EUL, Joachim, 12045 Berlin (DE)
(86) Internationale Anmeldenummer: PCT/EP2002/009082
(87) Internationale Veröffentlichungsnummer: WO 2003/016537

(56) Entgegenhaltungen:
- WO-A-02/053581
- WO-A1-97/22250
- WO-A2-00/09734
- CAUDEVILLA CONCHA ET AL: "Localization of an exonic splicing enhancer responsible for mammalian natural trans-splicing." NUCLEIC ACIDS RESEARCH, Bd. 29, Nr. 14, 15. Juli 2001 (2001-07-15), Seiten 3108-3115, XP002251616 ISSN: 0305-1048
- CONRAD R ET AL: "INSERTION OF PART OF AN INTRON INTO THE 5' UNTRANSLATED REGION OF A CAENORHABDITIS ELEGANS GENE CONVERTS ITS INTO A TRANS-SPLICED GENE" MOLECULAR AND CELLULAR BIOLOGY, AMERICAN SOCIETY FOR MICROBIOLOGY, WASHINGTON, US, Bd. 11, Nr. 4, April 1991 (1991-04), Seiten 1921-1926, XP001073410 ISSN: 0270-7306
- EUL JOACHIM ET AL: "Experimental evidence for RNA trans-splicing in mammalian cells." EMBO (EUROPEAN MOLECULAR BIOLOGY ORGANIZATION) JOURNAL, Bd. 14, Nr. 13, 1995, Seiten 3226-3235, XP001154331 ISSN: 0261-4189
- EUL J ET AL: "Trans-splicing and alternative-tandem-cis-splicing: Two ways by which mammalian cells generate a truncated SV40 T-antigen." NUCLEIC ACIDS RESEARCH, Bd. 24, Nr. 9, 1996, Seiten 1653-1661, XP002251617 ISSN: 0305-1048
- PUTTARAJU M. ET AL: "Spliceosome-mediated RNA trans-splicing as a tool for gene therapy", NATURE BIOTECHNOLOGY, vol. 17, no. 3, 1999, pages 246-252, UK
- MANSFIELD S.G. ET AL: "Repair of CFTR mRNA by spliceosome-mediated RNA trans-splicing", GENE THERAPY, vol. 7, no. 22, 2000, pages 1885-1895, UK
- PUTTARAJU M. ET AL: "Messenger RNA repair and restoration of protein function by spliceosome-mediated RNa trans-splicing", MOLECULAR THERAPY, vol. 4, no. 2, August 2001 (2001-08), pages 104-114,

## Beschreibung

Die Erfindung betrifft ein Verfahren zur mikrochirurgischen, zellulären Reparatur von mutierten Abschnitten einer RNA und zum gezielten Abtöten von Tumorzellen mit Hilfe einer einer eingebrachten, transspleißfähigen RNA unter Verwendung der natürlich vorkommenden Spleißkomponenten in der Zelle sowie ein Screening-Verfahren zum Nachweis von natürlichtransgespleißter zellulärer RNA.

In Säugerzellen können Spleißprozesse innerhalb einer RNA-Sequenz - das Cis-Spleißen - und zwischen verschiedenen RNA-Sequenzen - das Transspleißen - stattfinden. Transspleißen heißt, dass Verbindungen innerhalb eines RNA-Moleküls gelöst und neue Bindungen mit anderen RNA-Molekülen eingegangen werden und so ein neues RNA- Molekül entsteht. Derartige RNA-Transspleißprozesse verändern daher die genetische Information und können zu veränderten RNA-Molekülen bzw. zu pathogenen Mutationen führen, die z.B. Tumoren auslösen. Es ist daher von großer Bedeutung ein Verfahren zur Identifizierung von transgespleißter RNA bereitzustellen. Aber Transspleißprozesse können jedoch auch gezielt eingesetzt werden, um ein imitiertes Gen zu reparieren oder etwa um Tumorzellen spezifisch abzutöten. Die Erfinder streben aus diesem Grund Patentschutz für ein Grundverfahren sowie ein Erzeugnis an, mit dem je nach Anwendungsfall transgespleißte RNA zu therapeutischen Zwecken erzeugt wird, bzw. mit dem eventuell pathogene, zellulär-transgespleißte RNA diagnostiziert werden kann.

Diese Anwendungen basieren auf dem Verfahrensgrundprinzip, dass mittels einer in die lebenden Zellen eingebrachter DNA eine künstliche pre-mRNA erzeugt wird, die sich in ihrer Stuktur wesentlich von allen natürlichen zellulären pre-mRNA-Molekülen unterscheidet: Diese künstliche pre-mRNA besteht nämlich nur aus 1 oder maximal 2 Spleißstellen, entsprechend einem Exon, welches an seinen beiden Enden von einer oder zwei "Intronhälften", hier definiert als Outrons umgeben ist. Dieses Exon dient dann je nach Anwendungsfall dazu, ein gendefektes Exon einer natürlichen zellulären RNA über ein Austauschverfahren zu ersetzen/reparieren (Anwendungsprinzip 1) , oder etwa in Tumorzellen nach Ankoppelung an ein Exon aus einer tumorspezifischen RNA eine mRNA entstehen zu lassen, die für ein Protein codiert, das direkt oder indirekt zum Zelltod der Tumorzelle führt (Anwendungsprinzip 2) , oder dieses Exon ist Bestandteil einer RNA-Sonde, mit der zunächst transspleißfähige zelluläre pre-mRNAs indentifiziert werden, welche ggf. zu diagnostizierbaren neuen Hybrid-mRNA-Molekülen in der Zelle interagieren (Anwendungsfall 3) , die wiederum eventuell pathogene Prozesse auslösen.

Die in allen drei Anwendungsfällen eingesetzte RNA, die entsprechend ein oder zwei Spleißstellen enthält, muss zudem in allen Fällen bestimmte Bedingungen zur Gewährleistung einer ausreichenden Funktionseffiziens erfüllen: Damit diese Spleißstellen eine hohe Potenz zu einem intermolekularen Transspleiß aufweisen, müssen sie Sequenzen enthalten, die eine optimale Bindung von Spleiß (hilfs) - proteinen an diese RNA-Sequenzen gestatten. Im Falle einer therapeutischen Verwendung dieser RNA (Anwendungsfall 1 oder 2) ist zudem eine spezifische Anbindung zwischen der eingebrachten transspleißfahigen RNA und der zellulären Target-RNA erforderlich, was durch eine künstlich erzeugte AntisensStruktur auf der eingebrachten RNA, die in diesem Bereich spezifisch zur zellulären Target-RNA paart, erreicht wird. Durch die hierdurch entstandene ionischchemische Bindung (über Wasserstoffionenbrücken) zwischen der eingebrachten RNA und der zellulären Target-RNA wird zudem die Transspleißeffizienz erheblich gesteigert.

In Bezug auf das Anwendungsprinzip 1, nämlich dem Einsatz dieser eingebrachten RNA als Reparatur-RNA, ist auszuführen, dass eine sehr große Anzahl von Krankheiten wie Alzheimer, Parkinson, Diabetes, Hämophilie B, erblich bedingter Bluthochdruck etc.durch angeborene oder später erworbene singuläre Gendefekte bzw. Mutationen ausgelöst werden. Diese monogenetisch bedingten Krankheiten werden zur Zeit in der Regel mit Medikamenten in der Weise behandelt, dass diese Medikamente rein symptombezogen physiologisch wirken, ohne dass jedoch die eigentlichen Ursachen des Krankheitsbildes angegangen werden.

Sofern sich der Gendefekt nur in bestimmten Zelltypen oder Organen mit besonderen Aufgaben auswirkt, ist alternativ auch ein Austausch und Ersatz dieser Zellen oder Organe über eine Transplantation möglich. Aufgrund der noch ungelösten Probleme der Immunabstoßung von Fremdtransplantaten und Gefahren der Virenübertragung sind diese Möglichkeiten aber nur begrenzt einsetzbar.

Nur in wenigen Fällen ist bislang eine zumindest kausale Behandlung gegeben, indem das - bedingt durch den Gendefekt - fehlende oder funktionsunfähige Protein extern zugeführt wird. Beispiele sind die tägliche Injektion von Insulin bei Diabetikern oder von bestimmten Blutgerinnungsfaktoren bei Hämophilie.

Im Unterschied zu einer symptomatischen oder kausalen Behandlung kann eine echte Heilung genetisch bedingter Erkrankungen im Prinzip nur dadurch erfolgen, dass das defekte oder mutierte Gen in den Zellen in seiner Funktion wiederhergestellt wird. Da auf molekularer Ebene eine mikrochirurgische Genrepa^ratur durch gezielten Austausch der defekten Genteile in der Zelle nach heutigem Stand der Technikicht möglich ist, wird nach den etablierten gentherapeutischen Verfahren das defekte Gen durch Einschleusen eines homologen, intakten Genes in seiner Funktion ersetzt.

Auf Grund vielfacher technischer Probleme, wie der begrenzten Aufnahmegröße viraler Genshuttles, wird beim Ersatz des Gens jedoch nicht das eigentliche Gen, bestehend aus vielen Exon- und Intronbereichen mit z.T. 100 kb oder mehr Größe, verwendet. Das in die Zellen hineingebrachte Transgen ist vielmehr die um bis zu 95 % verkürzte cDNA des Gens, welche nur aus den Protein-codierenden Exonteilen besteht, und die nach reverser Transkription der mRNA erhalten wird. N-terminal ist an diese cDNA ein geeigneter Promotor und C-terminal eine Erkennungsstelle zur RNA-Polyadenylierung, z.B. aus SV40, durch einen gentechnischen Eingriff gekoppelt. Die Polyadenylierung ist vor allem für den Schutz der mRNA vor cytoplasmatisehen RNAsen und damit für deren Stabilität verantwortlich. Diese gentherapeutischen cDNA-Konstrukte erlauben eine konstante und hohe RNA-Expression. Der Ersatz des defekten Genes mit seiner komplexen Struktur, insbesondere mit seinen Intronanteilen und regulatorischen Sequenzen durch sein komprimiertes cDNA-Homolog hat aber auch einige gravierende Nachteile:
a) Eine sehr hohe Anzahl (bzw. %-Satz) der zellulären primären Genabschriften - der pre- RNAs -codiert nicht nur für eine einzige mRNA bzw. nur ein Protein, sondern wird über alternative Spleißvorgänge zu sehr unterschiedlichen mRNAs und Proteinen zusammengestellt. Bei zellulärem Ersatz eines Genes auf cDNAanstelle der DNA-Ebene entfällt der Mechanismus der RNA-Reifung über das Spleißen. Für jede benötigte Version dieser mRNAs muss deshalb eine andere cDNA gentherapeutisch in die Zelle gebracht werden.
b) Weiterhin wird die Auswahl der Exone und damit die Art der gebildeten Proteine im alternativen RNA-Spleißen in der Zelle durch sehr komplexe Mechanismen kontrolliert, welche wiederum durch verschiedene externe Faktoren gesteuert werden. Insbesondere Hilfsproteine binden an regulatorische RNA-Sequenzen und beeinflussen so die Auswahl der Exone bei alternativen Spleißvorgängen und damit das molare Verhältnis der verschiedenen möglichen, alternativen Spleißprodukte zueinander. Das Verhältnis der alternativen Spleißprodukte untereinander kann zudem einer komplexen zeitlichen Ablaufsteuerung unterliegen, wie etwa die verschiedenen Spleißformern der Immunoglobuline (IgM, IgG etc.). Diese regulatorischen Sequenzen für alternative Spleißvorgänge sind aber oft gerade innerhalb der Introns enthalten. Selbst wenn also alle möglichen alternativen mRNA-Formen durch unterschiedliche eingebrachte cDNA-Konstrukte zur Verfügung stehen würden (siehe a) , so ist doch eine physiologische und oft auch zeitlich gesteuerte Mengenregulation dieser mRNAs untereinander nicht gegeben, da diese regulierenden Intronsequenzen in diesen cDNAs nicht enthalten sind. Ein Ersatz alternativ spleißbarer, defekter Gene über die herkömmlichen Verfahren durch eine cDNA ohne diese Intronstukturen ist deshalb nachteilig.
c) Ein weiterer wesentlicher Aspekt ist, dass sich regulierende Sequenzen zur Transkriptionskontrolle wie Enhancer etc. zuweilen sehr weit N-terminal entfernt vom Promotor oder sogar in Intronbereichen befinden. Die artifiziellen cDNA-Genkonstrukte haben diesen natürlichen Kontext jedoch nicht mehr, es fehlen die Intronbereiche (s.o.) sowie auch regulatorische Sequenzen, die oft weit vom Promoter entfernt auf der nicht transkribierten DNA liegen. Selbst bei Verwendung des authentischen Promotors ist bei cDNA-Konstrukten also die zelluläre Fein-Regulation der Transkription nicht mehr gegeben; so kann es zu unphysiologischen Über- oder Unterexpressionen von Proteinen kommen, die wiederum zellschädlich sein können.
   Eine künstlich eingebrachte, komprimierte Ersatz-cDNA, versehen mit einem Promotor und einer neuen Polyadenylierungsstelle, hat also aufgrund großer fehlender DNA-Anteile (fehlende Introns und sonstige Bereiche) niemals die Komplexität des homologen natürlichen gendefekten Genes, und kann deshalb auch keinen physiologisch vollwertigen Ersatz darstellen.
d) Sofern große gendefekte mRNAs bzw. Proteine (150-200 kD Molekulargewicht) zu ersetzen sind, werden selbst bei Verwendung einer komprimierten cDNA Transgen-Größen von 5 kB und mehr Länge benötigt. Hierdurch können sich durch dieses Größe erhebliche Einschränkungen bei der Verwendung bestimmter Genshuttels (z.b. bei Adeno-assoziiertes-Viren (AAV)) sowie insbesondere bei einem nicht Viren-vermittelten, direkten 'Gentransfer ergeben.

Aufgabe der Erfindung ist es daher, ein Verfahren bereitzustellen, welches die obigen Nachteile vermeidet und es gestattet, das defekte bzw. mutierte Gen inklusive seiner natürlichen Umgebung weiterhin als zelluläre Ausgangsbasis zu verwenden, wobei das defekte Gen nicht komplett durch ein artifizielles Austauschprodukt (als cDNA) ersetzt, sondern das mutierte Gen spezifisch an der defekten Stelle mikrochirurgisch repariert wird.

Die mikrochirurgische Reparatur des Gendefektes erfolgt hierbei jedoch nicht auf der unmittelbaren DNA-Ebene, was nach heutigem Stand der Technik und in (naher) Zukunft (noch) nicht möglich ist. Die Reparatur des Gendefektes erfolgt vielmehr auf der Stufe der Abschrift der DNA, also der RNA, welche letztlich auch für die Proteinsynthese erst verantwortlich ist. Im Unterschied zur DNA ist die RNA nämlich in kleinere Untereinheiten (sog. Exone) untergliedert, die prinzipiell frei kombinierbar und deshalb auch untereinander austauschbar sind. Auf der primären Genabschrift, der pre-mRNA, sind diese Exone noch durch dazwischen liegende, nicht codierende RNA-Sequenzen (Intronbereiche) getrennt. Durch einen sehr komplexen Mechanismus im Zellkern, genannt (Cis) Spleißen, werden alle Intronbereiche herausgeschnitten und die Exone werden so direkt perlschnurartig miteinander verknüpft. Die so entstandene RNA (mRNA) verlässt dann den Zellkern, um danach im Zytoplasma der Proteinsynthese zur Verfügung zu stehen. Das Primärtransskript (pre mRNA) kann hierbei einige kb bis 100 kb Länge aufweisen (durchschnittlich 6 kb Länge) , insbesondere Intronlängen bis zu 50 kb Länge sind beschrieben. Die Exone sind hingegen relativ kurz; die nicht N- oder C-terminalen, also mittleren Exone haben nur eine Größe von durchschnittlich ca. 100-150 b Länge. Die Exonzahl schwankt je nach Gen von 2 bis über 50 (durchschnittlich ca. 6-10) , die durchschnittliche Größe aller aufsummierten Exone einer Säugerzellen-mRNA beträgt etwa 1,5 kb .

Gendefekte, die zu funktionsunfähigen Proteinen führen, betreffen oft nur eine oder sehr wenige Stellen des Genes, sie liegen also prinzipiell nur in einem der vielen 2 bis 50 Gen-Exone. Es ist daher, was Grundlage dieser Erfindung ist, ausreichend, anstelle des kompletten Genes, nur den mutierten Exonteil des Genes auszutauschen. Dieser Austausch erfolgt hierbei auf der RNA-Ebene.

Neben dem Cis-Spleißvorgang, der vor ca. 25 Jahren entdeckt wurde, und der verschiedene Exone desselben pre-mRNA-Moleküls verbindet, gibt es in der Säugerzelle auch sog. Transspleiß-prozesse, die als in vivo real existierend erstmals Mitte der 90er Jahre vom Entwickler der nachfolgend beschriebenen Erfindung und Anmelder dieses Patentes nachgewiesen und publiziert wurde. Bei diesen Trans-Spleißprozessen werden unterschiedliche pre-mRNAs bzw. Exone aus zwei oder mehr pre-mRNA-Molekülen miteinander kombiniert. Ein Reparatur-Austausch eines defekten Exons gegen ein intaktes Exon ist also möglich, indem man sich die natürliche RNA-Transspleißkapazität der Säugerzelle zu Hilfe nimmt.

Die Abbildung B1, B2 zeigt zunächst, wie aus einer defekten pre-mRNA nach erfolgtem, üblichen Cis-Spleiß anschließend eine mutierte, gendefekte zelluläre mRNA entsteht, die für ein funktionsuntüchtiges Protein codiert, sofern es sich um keine sogenannte stille Punktmutation handelt, und sofern die veränderte Aminosäure zu einer Funktionsbeeinträchtigung des Proteins führt.

Die Abbildung C1, C2 zeigt, wie im Prinzip ein nicht N-oder C-terminales gendefektes Exon auf einer pre-mRNA (Stufe 1) durch einen Transspleiß mit Hilfe einer Reparatur-RNA gegen das intakte Exon homolog ausgetauscht werden kann, sodass letztlich wieder eine intakte mRNA (Stufe 2) entsteht. Die Reparatur-RNA ist auch eine pre-mRNA und besteht im Prinzip aus dem auszutauschenden Exon, das an beiden Enden je eine "Intronhälfte" also einem Intron mit nur einer Spleißstelle, hier definiert als "Outron^{w}, trägt. Die Reparatur-pre-mRNA wird dabei dadurch in der Säugerzelle hergestellt, indem eine codierende DNA über ein DNA-Gentransferverfahren einmalig in die Zelle eingeschleust wird.

Die Erfindung löst das technische Problem einer mikrochirurgischen Reparatur eines defekten Genes, welche einem Genersatz auf cDNA-Basis vorzuziehen ist, also durch die Bereitstellung eines Verfahrens zur Reparatur eines mutierten Exons einer pre-mRNA in einer Säugerzelle, wobei in die Säugerzelle eine DNA eingebracht wird, die für eine Reparatur-RNA codiert, die ein nicht-mutiertes homologes Exon mit flankierenden Outrons umfasst. Das mutierte Exon der gendefekten RNA wird dann durch das nicht-mutierte Exon der Reparatur-RNA mittels der in der Zelle natürlich vorkommenden Spleißkomponenten über einen RNA-Transspleiß ausgetauscht, und so die RNA in ihrem defekten Anteil repariert .

Zum Austausch eines internen (nicht terminalen) mutierten Exons ist folglich im Regelfall ein zweifacher Transspleiß notwendig; die Reparatur-RNA enthält also neben dem Reparatur-Exon zwei Spleißstellen, d.h. ein proximales (5') und ein distales (3') Outron (s. Fig. C) . Zum Austausch eines terminalen, mutierten und teilweise Protein-codierenden Exons ist hingegen nur ein einfacher Transsspleiß notwendig; zur Reparatur eines codierenden Gendefektes in einem N-terminalen Exon besteht die Reparatur-RNA folglich aus einem Reparatur-Exon und einem distalen (3') Outron (s. Fig. D) ; bei einem codierenden Gendefekt in einem C-terminalen Exon besteht die Reparatur-RNA entsprechend aus einem proximalen (5') Outron gefolgt vom Reparatur-Exon (s. Fig. E). Ein einfacher (anstelle zweifacher) Transpleiß und folglich nur ein Outron in der Reparatur-RNA ist auch nur notwendig, wenn in dem transzuspleißendnen Repartur-Exon die genetische Information mehrerer terminaler Exone in Form einer cDNA enthalten ist (s. Fig. G und H) ; auf diesen Sonderfall soll in einem späteren Textabschnitt nochmals genauer eingegangen werden.

In der Grundvariante der Erfindung liegt also ein zentrales Reparatur-Exon vor, welches von einem proximalen 5' Outron und einem distalen 3' Outron umgeben ist. Die Übergangsregionen oder Grenzregionen zwischen Exon und Outron (bzw. Intron), die Spleißstelle, haben dabei stets eine gewisse Struktur.

Am hinteren Ende des Exons zum hinteren 3' Outron hin liegt die 5' Spleißstelle. 5' Spleißstellen sind Folgen von 9 RNA-Basen mit der Säugerzell-Konsensussequenz A/C-A-G-G-U-A/G-A-G-U. ZU einer 5' Spleißstelle gehören sowohl die letzten drei Nucleotide des Exons als auch die ersten sechs Nucleotide des Outrons/Introns . Die Basen G-U sind die ersten beiden Basen des Introns bzw. Outrons und zudem essentiell für eine normale 5' Spleißstelle, andere Basen können auch variieren. Die Basen der 5' Spleißstelle paaren in einer sehr frühen Phase (E-Komplex) des Spleißablaufes über H-Brücken mit der UₓsnRNA, die zum U^nRNP (einem Proteinkomplex) gehört (s. Fig. AI). Die Bindung zwischen der pre-mRNA und der U^nRNA bzw. dem U-Proteinkomplex ist dabei umso besser, je mehr Watson-Crick-Basenpaarungen eingegangen werden (maximal 9) ; eine optimale Paarung zur UⱼSnRNA wird hierbei durch die 9-Basenfolge (A/G) - (A/G) -G-G-U- (A/G) - (A/G) -G-U auf einer pre-mRNA erreicht. Die Bindung des U^nRNP an die 5' Spleißstelle wird noch weiter stabilisiert durch sog. Serin/Arginin-reiche Proteine (S/R-Proteine) (s. Fig. A 12). S/R-Proteine binden zum einen oft an Purinbasen-reiche RNA-Abschnitte vornehmlich in Exonbereichen. Die an die RNA in Exonbereichen (Exonic Splice Enhancer, ESE) gebundenen SR-Proteine (z.B. ASF/SF2) assoziieren in einem anderem Bereich dieser Proteine dann mit U₁snRNP und tragen so zur weiteren U₁SnRNP-Stabilisation zur pre-mRNA bei (s. Fig. I1 - 13).

Am vorderen Ende des Exons zum vorderen 5' Outron hin liegt die 3' Spleißstelle. Im Unterschied zur 5' Spleißstelle sind an der 3' Spleißstelle nur Nucleotide des Outrons bzw. Introns, also keine Nucleotide des Exons, beteiligt. Die 3' Spleißstelle ist komplexer aufgebaut als die 5' Spleißstelle und besteht aus 3 verschiedenen essentiellen Nucleotidkomponenten, die teilweise auch räumlich voneinder getrennt sind. Als diese drei Komponenten einer 3' Spleißstelle fungieren: eine sog. Branch-A-Stelle, ca. 20-50 Nucleotide downstream davon ein Pyrimidinbasen-Stretch und unmittelbar daran anschliessend ein essentielles AG-Dinucleotid. Das AG-Dinucleotid stellt zugleich das hintere Ende des Outrons/Introns an der Grenze zum nachfolgenden Exon dar.

Die relative Stärke einer 3' Spleißstelle einer pre-mRNA der Säugerzelle wird sehr stark durch die Qualität des Polypyrimidinbasen-Stretches beinflusst. Konkret ist der relative (U/C) Anteil der Basenfolge, die 2 bis ca. 15-19 Nucleotide upstream des AG-Dinucleotides liegt, von Bedeutung. Eine durchgehende U/C-Folge liegt in vivo nur selten vor, oft ist jedes 5. oder 6. Nucleotid ein G oder seltener ein A. Die höchste Spleißsignalstärke ist aber bei einem ununterbrochenen U/C-Stretch von mindestens 9-12 Nucleotiden gegeben. An den Polypyrimidinstretch bindet in einer sehr frühen Phase des Spleißprozesses (E-Komplex) der Proteinkomplex U2AF (U₂snRNP-Auxilary-Factor) über dessen Proteinanteil U2AF⁶⁵ (s. Fig. AI, Jl - J3). Die Konsensus-RNA-Bindungsstelle für U2AF⁶⁵ ist im übrigen 19 Nucleotide lang mit der Basenfolge U₆ (U/C) CC (C/u) U₇CC. Der zweite Anteil von U2AF, nämlich U2AF³⁵ kann an weitere SR-Proteine binden (Fig. Jl - J3) , die wiederum zu Purinreichen Regionen im nachfolgenden Exon (Exonic Splice Enhancer (ESE) Regionen) assoziieren (s. Fig. J1 - J3). Diese ESE-Regionen begünstigen also ebenfalls die Bindung von U2AF, wie im Falle der U-_{L}snRNP-Stabilisation an der 5' Spleißstelle.

Das dritte Element der 3' Spleißstelle, die sogenannte Branch-A-Site, die ca. 20-50 Nucleotide upstream des Poly-(U/C) -Stretches liegt (s. auch Fig. Jl - J3) , ist hingegen nicht so eindeutig über eine bestimmte Basenfolge definiert. Die Säugerkonsensussequenz hat die 7-Nucleotid-Folge (C/U) -N-C-U- (A/G) -A- (C/U). Essentiell ist insbesondere das sog. Branch A, (das unterstrichen ist) , welches in einer sehr späten Spleißphase (C-Komplex) über die 2' OH Gruppe seiner Ribose die Bildung des Lariats ermöglicht.

Die Branch-Sequenz der pre-mRNA paart in der auf den E-Komplex folgenden Spleißphase (= A- omplex) und im nachfolgenden B-Komplex (s. Fig. A2) mit der U₂snRNA, die zum Proteinkomplex U₂snRNP gehört (s. Fig. Jl - J3).

Wichtig kann auch hier wieder eine möglichst starke Watson-Crick-H-Bindung zwischen der pre-mRNA und der U₂snRNA sein, aber auch, dass das Branch-A in dem zur U₂snRNA gebundenem RNA-Stretch "herausragt", d.h. dieses Adenosin sollte insbesondere nicht zu einer Base (d.h. einem U) der U₂snRNA binden (s. Fig. J) . Die Bindung von U₂snRNP an die pre-mRNA wird dabei durch das bereits im E-Komplex gebundene U2AF unterstützt. Als Bindeglied zwischen U2AF und U₂snRNP können hierbei noch weitere Proteine (SF3a, SF3b) fungieren.

Beim Spleißen findet prinzipiell stets eine Assoziation einer 5' Spleißstelle mit einer 3' Spleißstelle statt. Beim Cis-Spleißen liegen diese beiden Spleißstellen auf dem selben RNA-Molekül, beim Trans-Spleißen entsprechend auf zwei verschiedenen RNA-Molekülen. Eine verbindende Nucleotidfolge zwischen den beiden Spleißstellen (gegeben immer bei Cis-Spleißprozessen innerhalb des selben RNA-Moleküls) ist zwar stark förderlich, aber nicht unbedingt notwendig für den Spleißprozess . Die eigentliche Assoziation zwischen den beiden Spleißstellen wird vielmehr durch Proteinkomplexe erreicht, die zuvor je an der 9-mer-Basenfolge der 5' Spleißstelle einerseits sowie am Polypyrimidinbasenstretch und der Branch-A-Stelle der 3' Spleißstelle andererseits gebunden sind. Der Kontakt der beiden Spleißstellen erfolgt also über eine Protein-Protein-Assoziation jener, an der RNA gebundenen?, Proteine. Diese Assoziation zwischen zwei Spleißstellen über gebundene Proteine erfolgt hierbei in mehreren Stufen: Im frühen E-Komplex des Spleißprozesses erfolgt eine Assoziation zwischen dem Poly(U/C) -Stretch der 3' Spleißstelle zu der 5' Spleißstelle. Hierbei wird an dem Poly(U/C) -Stretch der 3' Spleißstelle der Proteinkomplex U2AF^{S5}U2AF³⁵ gebunden, der wiederum über ein Brücken-SR-Protein (SC35) zum U^nRNP paart, was zuvor an der 5' Spleißstelle gebunden ist (s. Fig. AI). Im späteren B-Komplex findet hingegen eine Assoziation zwischen der Branch-A-Site der 3' Spleißstelle und der 5' Spleißstelle statt, indem die U₂snRNA des U₂snRNP in einem snRNA-Anteil zur Branch-A-Stelle und in einem andern snRNA-Anteil zur U₆snRNA des U₆snRNP paart. Die U₆snRNA wiederum paart mit Basen des Intronbereiches der 5' Spleißstelle der pre-mRNA. (Das vorher (im E und A-Komplex) hier gebundene UiSnRNP hat die Spleißstelle zu gunsten des U₆snRNP verlassen und auch U2AF ist hier nicht mehr am U/C-Stretch gebunden.) (s. Fig. A2). In einer noch späteren Phase assoziiert der Proteinkomplex U₅snRNP zunächst in einem Anteil zum Exonanteil der 5' Spleißstelle und danach in einem anderen Anteil auch zum Exonanteil der 3' Spleißstelle (s. Fig. A2) , gefolgt von der Lariatbildung der RNA (nicht mehr dargestellt) . Durch diese assoziierenden Strukturen tritt also ein sehr naher räumlicher Kontakt zwischen den später zu ligierenden Exonen ein.

Da die exakte Assoziation der Spleißstellen durch die an die pre-mRNA gebundenen Proteine bewerkstelligt wird, besteht die Aufgabe der verbindenden Nucleotidkette eines Introns von bis zu 50 kB Grosse beim Cis-Spleißen also nicht darin, die 5' und 3' Spleißstelle eines Introns in den erforderlichen räumlichen Zusammenhang (Kontext) zum Spleißen zu bringen; dies gilt im wesentlichen nur für sehr kurze Introns mit ca. 100 Basen Länge. Die Nucleotidkette des Introns hat vielmehr die Aufgabe, die zugehörige 5' und 3 ' Spleißstelle in einem nicht zu großem maximalen Abstand (ca. 100-500 nm, je nach Intronlänge) verglichen mit der Zellkerngröße von 10.000 nm zu halten, sodass sich die beiden mit den Spleißproteinen beladenen Spleißstellen während der thermisch bedingten ständigen Schwingungs- und Bewegungsvorgänge der RNA im Zellkern mit hoher Wahrscheinlichkeit irgendwann treffen können, wonach der Spleißprozess (über die Spleißstufen E, A, B usw.) eingeleitet wird.

Trans-Spleißprozesse der Säugerzelle laufen nach gleichem Muster unter Benutzung der selben Proteinkomplexe wie Cis-Spleißprozesse ab; der einzige Unterschied ist, dass eine verbindende RNA-Nucleotidkette zwischen den beiden Spleißstellen von Natur aus nicht vorhanden ist (s. Fig. A1 und A2 : Die gestrichelte Linie ist dort in der Abbildung wegzudenken) .

Aufgrund dieses Umstandes einer fehlenden kovalenten Bindung zwischen den beiden Spleißstellen sind Transspleiß-prozesse zwischen zwei ganz bestimmten RNA-Molekülsorten - statistisch gesehen - zunächst einmal recht seltene Vorgänge, vergleicht man'den großen Zellkemdurchmesser (ca. 10.000 nm) mit dem kleinen Durchmesser (ca. 100 nm) der Proteinkomplexe, welche an den 5' und 3' Spleißstellen auf zwei unterschiedlichen RNA-Molekülen gebunden sind.

Die an sich sehr geringe Transspleißwahrscheinlichkeit zwischen zwei spezifischen RNA-Molekülsorten lässt sich jedoch gegeben durch zufällige natürliche Umstände (oder aber gewollt durch externe Eingriffe) um mehrere Zehnerpotenzen steigern, wenn folgende Bedingungen erfüllt sind:
(i) Die transzuspleißenden pre-mRNA-Moleküle sollten relativ abbaustabil sein und in hoher Konzentration im Zellkern vorliegen. Abbaustabilität bedeutet, dass diese RNA-Moleküle u.a. am hinteren Ende eine Polyadenylkette aufweisen; die DNA-Vorlage dieser RNA muss also die beiden Signalsequenzen zur Polyadenylierung (AATAAA-Region upstream und eine GT-reiche Region downstream der Polyadenylierungsstelle) aufweisen (s. z.B. Fig. FI, Gl, Hl). Eine hohe RNA-Konzentration wird dadurch verursacht, dass eine große Zahl von pre-mRNA-Molekülen von dem entsprechenden DNA-Template abgeschrieben werden, was wiederum durch einen starken Promotor auf der entsprechenden DNA erreicht wird. So gibt es in vivo, bedingt durch unterschiedliche Promotoren in der Säugerzelle, etwa 11.000 Gene die nur durchschnittlich 15 mRNA-Kopien, 500 Gene, die durchschnittlich 300 Kopien und nur 4 Gene, die durchschnittlich 12.000 mRNA-Kopien im Zellkern enthalten. Im • Falle des durch die Patentanmelder erstmals nachgewiesenen Säugerzellen-Transspleißes in vivo, wurde beispielsweise eine sehr große Zahl von Primärtranskripten, die mit einer quantitativen PCR nachgewiesen wurden, bedingt durch einen starken SV40 Promotor hergestellt.
(ii) Förderlich für einen intermolekularen Transspleißprozess kann es ferner sein, wenn die beiden transzuspleißenden RNA-Moleküle über einen längeren Basenbereich in Antisens-Strukturen über Watson-Crick-H-Brücken miteinander verbunden sind. Im Falle des durch den Patentanmelder nachgewiesenen in vivo Transspleißes bei Säugerzellen erfolgte diese RNA-Paarung vermutlich durch eine 13 Basen lange natürlich vorkommende Antisensregion zwischen dem N-terminalen Teil des einen RNA-Moleküls und dem C-terminalen Teil des zweiten RNA-Moleküls. Auf RNA-Ebene ist dabei neben der starken Basen-Paarung G-C und der mittelstarken Paarung A-U auch die etwas schwächere Basen-Paarung G-U zu berücksichtigen. Je nach G/C-Anteil sind bei einer Temperatur von 37°C ca. 10-16 durchgehende, d.h. hintereinanderliegende Basenpaarungen für eine stabile RNA-RNA-Assoziation ausreichend. Die Verbindung der beiden RNA-Moleküle über diese Antisens-Basenpaarungs-"Brücke" ist also der Funktion der Nucleotidkette des Introns bei Cis zu spleißenden, gewöhnlichen pre-mRNAs analog: Die beiden Spleißstellen werden durch eine verbindende NucleotidKette in einem bestimmten nicht zu großen, maximalen Abstand gehalten, und der Eintritt eines Spleißprozesses nach Assoziation der Proteinbeladenen Spleißstellen wird dadurch wesentlich wahrscheinlicher.
(iii) Aber auch wenn die beiden potentiellen RNA-Transspleißpartner in enger Nähe zueinander liegen, z.B. bedingt etwa über Antisens-H-Brücken, ist immer noch nicht ausreichend sichergestellt, dass hinreichend oder überwiegend intermolekulare Transspleiß-Prozesse stattfinden: Eine bestimmte 5' Spleißstelle beispielsweise auf dem ersten RNA-Molekül kann nämlich nicht nur in einer Transspleißreaktion zu einer 3' Spleißstelle auf einem zweiten, nahe liegendem RNA-Molekül, sondern auch weiterhin mit der gegenüberliegenden intronischen 3' Cis-Spleißstelle des selben ersten Moleküls in einem gewöhnlichen Cis-SpleißProzess interagieren. Der letztere Fall wird meist sogar wahrscheinlicher sein. Eine relative Bevorzugung des Trans-Spleißes zu ungunsten des Cis-Spleißes kann aber insbesondere dadurch auftreten, dass die entsprechende Cis-Spleiß-Stelle eine nicht so hohe Spleißstellen-Attraktivität (weniger angenähert an die Konsensussequenzen etc.) wie die konkurrierende Transspleißstelle aufweist. Doch auch dann können noch beide Reaktionen (Trans- und Cis-Spleiß) nebeneinander stattfinden. Nur wenn die konkurrierende Cis-Spleißstelle durch bestimmte weitere natürliche Faktoren oder externe (erfinderische) Eingriffe funktionsunfähig gemacht wird, ist mit einem ausschließlichen Transspleiß zu rechnen.

Speziell in Bezug auf eine Reparatur-RNA, die über einen RNA-Transspieiß ein gendefektes Exon ersetzen soll, müssen zwei prinzipielle Grundbedingungen erfüllt werden: (1) Der RNA-Transspleiß muss effektiv ablaufen, was konkret heißt, dass (je nach Art des Gendefektes) 5-50 % der gendefekten RNA-Moleküle repariert werden. Und: (2) Der RNA-Transspleiß soll spezifisch nur zur gendefekten RNA (und speziell zum gendefekten Exon) , nicht jedoch zu anderen RNA-Molekülsorten der Zelle ablaufen.

Um (1) eine relativ hohe Transspleißrate (von 5-50 %) und (2) eine hohe Substratspezifität zu erreichen, müssen in Bezug auf eine künstlich geschaffene Reparatur-RNA (dieser Anwendungsfall) oder auch in Bezug des Transspleißes einer unvollständigen "Zelltod-RNA" zu einer Tumorzellenspezifischen RNA (siehe der nächste genauer beschriebene Anwendungsfall 2) unter Beachtung der obigen Ausführungen folgende Kriterien erfüllt sein:
1.) Die die transzuspleißende RNA (Reparatur-RNA etc.) codierende DNA sollte insbesondere einen starken RNA Polymerase-II-Promotor enthalten, z.B. SV40 early T-Antigen Promotor oder andere mit einem starken Enhancer (s. Fig. FI) , damit eine hohe Zahl von transspleißfähigen RNA-Transskripten hergestellt werden kann. Die Effizienz einer Transspleißreaktion ist wesentlich von der Konzentration der transzuspleißenden RNA-Moleküle (= Substrat) im Zellkern abhängig.
2.) Die die transzuspleißende RNA (Reparatur-RNA etc.) codierende DNA sollte z.B. am 3' Ende die SignalSequenzen zur pre-mRNA-Polyadenylierung aufweisen, da die Polyadenylierung u.a. für die Stabilität einer RNA verantwortlich ist. Als eine sehr potente Polyadenylierungsregion kann hier z.B. auch die entsprechende Region der early SV40 DNA-Region genutzt werden.
3.) Ansonsten sollte das transgenische Produkt, welches die Transspleiß-RNA codiert, in geeignete Vektoren integriert sein, die u.a. eine selbständige Replikation des Transgens erlauben, eine Integration dieser DNA in die chromosomale DNA eher ausschließen und die keine eventuelle Immunabwehr über sonstige codierende Proteine des Vektors ermöglichen.
4.) Die transzuspleißende RNA (Reparatur-RNA, unvollständige "Zelltod-RNA" etc.) sollte über ionische Bindungen (H-Brücken) mit den Transspleißpartner (gendefekte RNA, tumorzellspezifische RNA etc.) verbunden sein, um die Transspleißeffizienz zu steigern. Dies ist dadurch zu erreichen, dass die Reparatur-RNA etc. eine längere, hintereinander liegende Folge von Nucleotiden in den Outron-RNA-Teilen aufweist, die über Antisens-H-Brücken zu einem entsprechenden Bereich auf der zu reparierenden RNA etc . paart .Dies wird dadurch erzielt, dass in der diese Transspleiß-RNA (Reparatur-RNA etc.) codierenden DNA eine entsprechende Antisenssequenz erzeugt wird.

Damit unter physiologischen Bedingungen der Zelle (37°C, neutraler pH-Wert, 150 mM NaCl-Konzentation etc.) eine stabile RNA-RNA-Hybridisierung eintritt, muss die Hybridisierungszone der Watson-Crick-H-Brücken eine minimale Länge aufweisen. Als sehr grober Richtwert gilt für die Annealing-Temperatur, dass die Basenpaarung C - G um 3°C, die Paarung A - U bzw. A - T um 2°C und die Paarung G - U um 1°C die Annealing Temperatur über 0°C erhöht. Eine 12 Basen (mer) lange ununterbrochene RNA-RNA-Hybridisierungszone bestehend aus statistisch verteilt: 6 Bindungspaaren C - G und 6 Bindungspaaren A - U besteht also insgesamt aus 30 H-Brücken mit annähernd 30°C Schmelztemperatur, wobei dies ein Minimalwert ist; tatsächlich liegt der Wert einige Celcius-Grade höher, eine genaue Berechnung ist über entsprechende EDV-Programme möglich. Unter dem Gesichtspunkt der Annealingtemperatur unter zellphysiologishen Bedingungen ist also eine mind. 12-14 Basen lange Hybridisierungszone für eine feste RNA-RNA-Bindung bei einer linearen RNA ausreichend.

Besondere Sekundärstrukturen der transzuspleißenden externen RNA (Reparatur-RNA etc.) und des zellulären RNA-Transspleißpartners (gendefekte RNA etc.) sind bei der Auswahl der willkürlichen Nucleotidfolge auf der Reparatur-RNA, die hybridisieren soll, jedoch zu berücksichtigen. RNAs liegen in der Regel nämlich nicht als eine lange, lineare Nucleotidkette vor, sondern es können auch intramolekulare Hybridisierungszonen auftreten, wodurch über einen bestimmten RNA-Bereich eine doppelsträngige RNA - mit der Ausbildung von stem loops, hairpins etc. - entsteht. Mit Hilfe geeigneter Computerprogramme kann aber aufzeigt werden, welche RNA-Bereiche innerhalb eines RNA-Moleküls unter physiologischen Bedingungen miteinander paaren. Stellt man also fest, dass eine künstlich geschaffene RNA-Sequenz innerhalb einer Reparatur-RNA etc. von z.B. 12 oder mehr Nucleotiden überwiegend mit anderen Regionen dieser RNA eine intramolekulare RNA-Paarung eingeht, so ist diese Sequenz zu verwerfen und eine abgeänderte Sequenz, die natürlich wiederum zur gendefekten RNA etc. paaren soll, auszuwählen Entsprechend ist auch der zu paarende Bereich auf der gendefekten RNA etc. zu analysieren, auch dieser sollte tatsächlich für eine inter-molekulare Paarung mit der Reparatur-RNA etc. zur Verfügung stehen, er darf also ebenfalls nicht durch eine intra-molekulare Paarung zu anderen Bereichen der gendefekten RNA etc. blockiert sein. Die Auswahl von hier geeigneten RNA-Sequenzen kann auch wieder über entsprechende EDV-Programme erfolgen.
5.) Die externe transzuspleißende RNA (Reparatur-RNA, unvollständige "Zelltod-RNA" etc.) sollte sehr stringente 5' und 3' Spleißstellen aufweisen. Die 5' Spleißstelle bzw. die 3' Spleißstelle z.B. auf der Reparatur-RNA sollten insbesondere attraktiver für die Spleißenzyme (Spliceosomen und Hil sproteine) sein als die authentischen, analogen Cis-Spleißstellen auf der gendefekten zellulären RNA, damit nicht jene analogen Cis-Spleißstellen verwendet werden, was zu einer gendefekten RNA führen würde, sondern die Trans-Spleißstellen auf der Reparatur-RNA verwendet werden, wodurch eine reparierte mRNA entsteht (s. Fig. B, C).
   a) Die 5' Spleißstelle (s. Fig. I1 - I3) der Transspleiß-Reparatur-RNA sollte also insbesondere eine höhere Attraktivität für die Proteine des Spleißapparates - insbesondere das U₁snRNP - haben als die analoge, unerwünschte 5' Cis-Spleißstelle auf der gendefekten RNA. Diese Bedingung ist in Regel erfüllbar, da die natürlichen zellulären Cis-Spleißstellen, also auch die entsprechende 5' Cis-Spleißstelle der gendefekten RNA, im Regelfall zur U₁-snRNA des Spleißproteinkoplexes U^nR P nicht optimal paaren, d.h. von der optimalen 5' -Spleißstellensequenz geringfügig abweichen (s. Fig. I). Anzustreben ist also über gentechnologische Eingriffe (auf Ebene der codierenden DNA) die Schaffung einer spleißoptimalen 9-mer langen Sequenz in der 5' Spleiß-Stelle der Reparatur RNA. Im 6 Basen umfassenden Intronteil bzw. Outronteil der 5' Spleißstelle der Reparatur-RNA etc. bedeutet dies die Schaffung einer Sequenz, welche die Folge G-U-(A/G) - (A/G) -G-U enthält, die bevorzugte Folge hier ist: G-U-A-A-G-U; diese Basenfolgen paart optimal (grösste H-Brücken-Zahl) mit der U₁snRNA. Unter dem Ziel, eine optimale Spleißstellensequenz zu erreichen sind Willkürliche Eingriffe im 3 Basen umfassenden exonischen Teil der 5' Spleißstelle der Reparatur-RNA hingegen nur sehr begrenzt möglich, da durch eine Basenveränderung gegenüber der authentischen Spleißstelle auf der gendefekten RNA keine andere Aminosäure codiert werden darf. Hier sind jedoch gewisse Variationsmöglichkeiten gegeben, da in der Regel eine bestimmte Aminosäure duch 2, 4 oder 6 verschiedene Basentripletts codiert wird. Als ein zulässiges Beispiel einer solchen Veränderung sei die Überführung des Basentripletts CGG (Fig. I1) durch einen künstlichen Eingriff in das Basentriplett AGG (Fig. I2) aufgeführt. Die neue Basenfolge AGG auf der Reparatur-RNA paart besser als die analoge wt-Folge CGG in der gendefekten RNA zur U^nRNA des U₁snRNPs (Fig. II und 12), beide codieren aber nach wie vor für die gleiche Aminosäure, nämlich Arginin (Arg).
      Die Attraktivität der 5' Spleißstelle auf der Reparatur-RNA lässt sich noch weiter erhöhen, wenn die Anlagerung des U₁snRNP an diese RNA durch zusätzliche Spleiß-Hilfsproteine unterstützt wird. Diese Spleißhilfsproteine sind zumeist Arginin-Serin-reiche Proteine (SR-Proteine) , die über eine spezielle Bindungsdomäne zu Purinreichen Sequenzen oder zu anderen spezielle Sequenzen innerhalb der RNA binden. Sehr oft liegen diese A/G-reichen Sequenzen, die als "Splice-Enhancer" fungieren, im zur Spleißstelle zugehörigen Exon ("Exonic-Splice-Enhancer, " = ESE). Im Falle der Reparatur-RNA sollte versucht werden, durch den gentechnischen Austausch einzelner Basen - auf der die RNA codierenden DNA - einen möglichst langen bzw. A/G-reichen Nucleotid-Stretch zu erhalten (Fig. I1, I2) ;Voraussetzung ist auch hier, dass bei Veränderungen der Nucleotide im Exon keine Codons für andere Aminosäuren entstehen dürfen. Beispielsweise kann die Basenfolge GGU im Exon der authentischen wt-RNA durch die Basenfolge GGA in der Reparatur-RNA ersetzt werden (Fig. I1, I2) ; hierdurch wird ein durchgehender, längerer A/G-Stretch erreicht, ohne dass eine Aminosäurenveränderung auftritt, denn GGU und GGA codieren beide für Glycin. Im speziellen Falle des Spleiß-Hilfsproteins ASF/SF2, das die Bindung von U₁SnRNP unterstützt, ist die anzustrebende RNA-Folge zur Bindung dieses Proteins z.B. AGAAGAAC oder GGAAGAAC, oder andere Sequenzen, die nur A, G sowie C aber kein U enthalten. Die ASF/SF2 bindenden RNA-Regionen können anstelle im Exon aber auch im Intronteil/Outronteil der 5' Spleißstelle liegen ("Intronic-Splice-Enhancer, " = ISE).
b) Auch die 3' Trans-Spleißstelle (s. Fig. J) auf der Reparatur-RNA sollte durch gentechnologische Eingriffe auf der die Reparatur-RNA codierenden DNA eine höhere Attraktivität als die entsprechende analoge, konkurrierende, unerwünschte 3' Cis-Spleißstelle auf der gendefekten RNA aufweisen. Primärer Angriffspunkt der Attraktivitäts-Steigerung ist hierbei der Poly(U/C)-Stretch unmittelbar vor der Folge AG am distalen Ende des Introns bzw. Outrons. Günstigerweise sind durchgehende Pyrimidinstretches mit 9 oder mehr aufeinanderfolgenden U- oder C-Basen in den authentischen Spleißstellen von natürlichen (wildtyp) RNAs nur sehr selten, so dass hier durch gezielte willkürliche, gentechnologische Überführung einzelner G- oder A-Basen in C- oder U-Basen im Pyrimidinbasenstrech auf der entsprechenden Reparatur-RNA (vgl. Fig. Jl und J2) ein durchgehender, langer U/C-Stretch von 12-18 Basen geschaffen werden kann. Da der Angriffspunkt des Poly (U/C) -Stretches primär das Protein U2AF⁶⁵ ist, dessen RNA-Bindungskonsensussequenz U₆ (U/C) CC (C/U) U₇CC ist, sollte diese spezielle Sequenz über eine willkürliche Veränderung in der 3' Spleißstelle der Reparatur-RNA angestrebt werden. Prinzipiell ist auch eine Veränderung der Nucleotidfolge in der Branch-A-Stelle der 3' Spleißstelle der trans-zuspleißenden RNA (Reparatur-RNA etc.) in Richtung zu einer optimalen Paarung zur U₂snRNA des Spleißproteinkoplexes U₂snRNP möglich. Dies wäre eine anzustrebende Nucleotidfolge: U- (A/G) -C-U- (A/G) -A-(C/U) auf der transzuspleißenden RNA innerhelb der Branch-A-Stelle (s. Fig. Jl und J2). Die Verhältnisse sind jedoch in Bezug auf die Branch-A-Stelle sehr komplex; zudem ist die genaue Stelle der Branch-A-Site auf einer pre-mRNA oft nicht experimentell bestimmt, sondern es wird eine solche Branch-A-Stelle dort vermutet, wenn die Branch-A-Konsensussequenz annähernd gegeben ist. Diverse Gene, z.B. das SV40. T-Antigen-Intron, tragen zudem oberhalb des gemeinsamen Poly-Pyrimidinstretches gleich mehrere Branch-A-Stellen zur Auswahl. Willkürliche Veränderungen der Branch-A-Stelle auf der Reparatur-RNA im Vergleich zur gleichen Stelle auf der gendefekten RNA sind nur bedingt vorzunehmen, vorteilhafter ist eher eine Veränderung des Polypyrimidinbasenstretch.es (s.o.).

Die Attraktivität auch der 3' Spleißstelle auf der Reparatur-RNA lässt sich noch weiter erhöhen, wenn die Anlagerung des U2AF-Proteinkomplexes an den Polypyrimidinbasenstretch der 3' Spleißstelle dieser RNA ebenfalls durch zusätzliche Spleiß-Hilfsproteine unterstützt wird. Diese Spleißhilfsproteine können wiederum S/R-Proteine sein, die z.B. an A/G-reiche Sequenzen der RNA binden. Diese A/G-reichen Sequenzen können auch wiederum durch gentechnologische Veränderungen einzelner Basen im Repartur-Exon erzeugt werden (Fig. Jl, J2) , wobei auch hier durch diese Basenänderungen keine andere Aminosäure codiert werden darf (s.o.)
6.) Um die zum gendefekten Exon hin unerwünschten Cis-Spleißreaktionen, die zur gendefekten mRNA führen (s. Fig. B) , weiter auszuschließen, sind diese Cis-Spleißstellen zu inaktivieren. Die Inaktivierung erfolgt am besten über eine RNA, die künstlich geschaffene Antisensregionen zu diesen 5' und 3' Cis-Spleißstellen aufweist, wodurch die Cis-Spleißstellen blockiert und inaktiviert werden. Im einfachsten Falle enthält die Reparatur-RNA selber diese Antisensregionen (s. Fig. C, D, E). Die Blockierung der analogen, konkurrierenden unerwünschten 5' -Cis-Spleißstelle mit einer 9-mer-Basenfolge auf der gendefekten RNA erfolgt am besten durch eine hierzu vollständig oder überwiegend paarende, künstlich geschaffene Antisenssequenz im Outronteil der transzuspleißenden RNA (s. Fig. I3, oben). Die Blockierung der analogen, konkurrierenden unerwünschten 3 ' -Cis-Spleißstelle auf der gendefekten RNA wird entsprechend durch eine entsprechende Antisensstruktur zwischen der transzuspleißenden RNA und dem Polypyrimidinbasenstretch der 3' -Cis-Spleißstelle erreicht (s. Fig. J3 , oben).
   Die Antisensregionen der Reparatur-RNA zur Target-RNA erfüllen somit zwei Funktionen: a) Herstellung einer stabilen Bindung zur Reparatur-RNA und b) Blockierung der unerwünschten Cis-Spleißstellen auf der gendefekten RNA.
7.) Die Antisensstrukur zwischen der transzuspleißenden RNA (Reparatur-RNA etc.) und der Target-RNA hat aber auch noch eine wesentliche dritte Funktion: Sie sorgt für die erforderliche Selektion des richtigen RNA-Targets, da der RNA-Transspleiß spezifisch nur zur gendefekten RNA (und speziell zum gendefekten Exon) , nicht jedoch zu anderen RNA-Molekülsorten der Zelle ablaufen soll.

Die Wahscheinlichkeit (W) , mit welcher eine RNA-Nucleotidfolge bestehend aus den 4 Nucleotiden G, C, A, U mit einer enstprechenden Antisensfolge auf einer anderen RNA komplett paart, ist statistisch gesehen: W = 1 zu 4ⁿ (n = Anzahl der Nucleotide im Basenstretch) . Bei einem 18 mer einer DNA wäre eine vollständige Paarungswahrscheinlichkeit von zwei DNA-Hälften demnach 1 zu 4¹⁸ = 1 zu 68.719.476.736 (68 Mrd.); wegen der noch zusätzlich möglichen RNA-Basen-Paarung: U - G bzw. G - U liegt die Wahrscheinlichkeit, mit der eine 18 merige RNA-Folge mit 9 A oder C und 9 U oder G in Antisens irgendwo zu einer anderen RNA-Folge paart, jedoch darunter, nämlich bei 1 zu 4⁹ x 2^{s} = 1 zu 262.144 x 512 = 1 zu 134.217.728 (134 Mio.). Die überschlägige Berechnung der Gesamt-Nucleotidzahl der Säugerzell-RNA ergibt bei ca. 25.000 verschiedenen pre-mRNAs und sonstigen RNAs (ribosomale RNA etc.) bei einer durchschnittlichen pre-mRNA-Länge von 6.000 Basen einen Gesamtwert von ca. 150 Mio. RNA-Nucleotiden. Die Wahrscheinlichkeit, dass eine speziell geschaffene 18 mer Basenfolge mit 9 A/C und 9 U/G einer transzuspleißenden RNA (Reparatur-RNA etc.) nicht nur zur speziellen Target-RNA (gendefekte RNA etc.) , sondern noch zu einer anderen RNA mit einer identischen AntisensStruktur paart, ist also statistisch gesehen 150 Mio zu 134 Mio, d.h. die Wahrscheinlichkeit liegt bei ca. 1 zu 1 ; bei einem 20 mer ist der Wert also nur noch ca. 1 zu 8. Statistische Überlegungen alleine sind jedoch nicht ausreichend: Auch hier ist mit Hilfe von geeigneten, erhältlichen Computerprogrammen zu überprüfen, ob eine unerwünschte vollständige Hybridisierung einer bestimmten 18-20 mer-Folge zu anderen, ungewollten RNA-Mólekülen auszuschließen ist. Falls eine solche möglich ist, ist die zu paarende Basenfolge auf der Reparatur-RNA entsprechend zu modifizieren, und die Prüfung erneut zu vollziehen. Es sei jedoch in diesem Zusammenhang angemerkt, dass eine Paarung der Reparatur-RNA zu einer anderen als der vorgesehenen Target-RNA (gendefekte RNA) nicht gleichbedeutend damit ist, dass diese Reparatur-RNA dann einen zu dieser RNA nicht erwünschten Transspleiß vollzieht, da eine RNA-RNA-Paarung alleine noch keine Gewähr für einen RNA-Transspleiß darstellt; hier müssen insbesondere auch Bedingungen vorliegen, die einen Transspleiß über Blockierung etc etwaiger Cis-Spleißstellen fördern (s.v.). Ob solche unerwünschten Transspleißreaktionen wirklich stattfinden, kann z.B. über geeignete cDNA-PCR-Verfahren analysiert werden (s. auch letzter beschriebener Anwendungsfall 3).

Nachdem eine DNA, die z.B. eine Reparatur-RNA codiert, hergestellt und über geeignete Methoden in entsprechend gendefekte Zellen eingebracht wurde, so dass in diesen Zellen permanent diese Reparatur-RNA produziert wird, ist im nachfolgenden Schritt die Transspleißeffiziens zur gendefekten RNA hin zu analysieren. Für therapeutische Zwecke reicht es, wenn, je nach Anwendungs/Krankheitsfall, 5 - 50 % intakte entsprechende mRNAs bzw. Proteine im Vergleich zur nicht gendefekten Zelle hergestellt werden.

Diese Analysen erfolgen zunächst in praeklinischen Studien zuerst mit Zellkulturen und dann im Tierversuch. Die Zellkultur kann von einem Menschen abstammen, der einen monogenetischen Erbdefekt hat, der im einfachsten Fall aus einer einzigen Basenmutation besteht, welche dann für ein fehlerhaftes Protein codiert. Im angegebenen Musterbeispiel (s. Fig. F) könnte ein Gendefekt auf RNA Ebene aus der Basenfolge GAGC bestehen, wohingegen die intakte wt-Sequenz, welche die Reparatur-RNA trägt, die Basenfolge GAUC umfasst (Fig. F3) . Die Analyse der Transspleißeffizienz - dem Verhältnis von trans-gespleißter zu cisgespleißter RNA - ist dann beispielsweise über eine spezifische cDNA-PCR aus der Gesamt-RNA der Zellen gefolgt von einer "Sequenzanalyseⁿ der cDNA-PCR-Produkte möglich. Die beiden PCR-Primer würden zunächst so gewählt, dass diese im wesentlichen die cis-gespleißten, defekten RNA-Moleküle (Fig. F4b) oder trans-gespleißten, reparierten/ intakten mRNAs (Fig. F4a) erfassen, nicht jedoch die entsprechenden noch nicht cis- oder transgespleißten pre-mRNAs. Dies wird z.B. dadurch erreicht, dass die Primer so gewählt werden, dass diese genau auf der Mitte der Spleißverbindungsstelle (Splice junction) des interessierenden Exons zu dem up-stream und dem down-stream Exon der ds-cDNA in der nachfolgenden PCR binden (Fig. F5a und F5b) ; bei einem 18-mer PCR-Primer etwa würden je 9 Nucleotide auf die beiden Exonenden fallen.

In dieser cDNA-PCR werden also zunächst zwei äußerlich nicht unterscheidbare, gleichgroße PCR-Produkte erhalten, die entweder von der cis-gespleißten mRNA (Fig. F6b) oder der transgespleißten mRNA (Fig. F6a) abstammen.

Die beiden PCR-Produkte können jedoch wie folgt getrennt und unterschieden werden: Im Reparatur-Exon sind im Unterschied zum gendefekten Exon ein oder mehrere Nucleotide verändert. Durch den Austausch nur eines Nucleotides oder mehrerer Nucleotide ergibt sich im Regelfall bei der Vielzahl der bekannten Restriktionsenzyme automatisch eine neue cDNA-Restriktionsschnittstelle oder eine vorherige Restriktions-Schnittstelle verschwindet. In dem hier aufgezeigten exemplarischen Fall enthält das PCR-Produkt abstammend von der transgespleißten, reparierten mRNA die Sequenz GATC, welche eine Mbo I Restriktionsenzymschnittstelle darstellt. Sofern ausschließlich transgespleißte cDNA-PCR-Produkte vorliegen, wird das PCR-Produkt nach Mbo I Verdau also vollständig in zwei kleinere Fragmente gespalten (Fig. F7a). PCR-Produkte, z.B. mit der Sequenz GAGC, die von der cis-gespleißten, defekten mRNA abstammen, werden durch Mbo I jedoch nicht gespalten (Fig. F7b). Der Anteil der Mbo-I-gesplaltenen PCR-Produkte in Relation zu den ungespaltenen PCR-Produkten gibt also die Effizienz der Transspleißreaktion in der Zelle wieder. Das Verfahren beschreibt folglich ein vereinfachtes Sequenzierungsverfahren über eine PCR-Restriktionsfragmentanalyse.

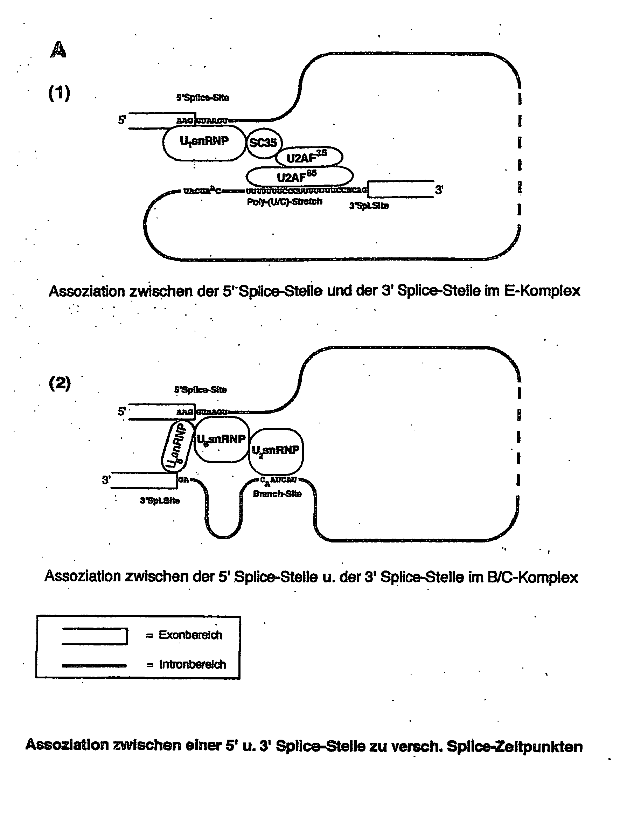

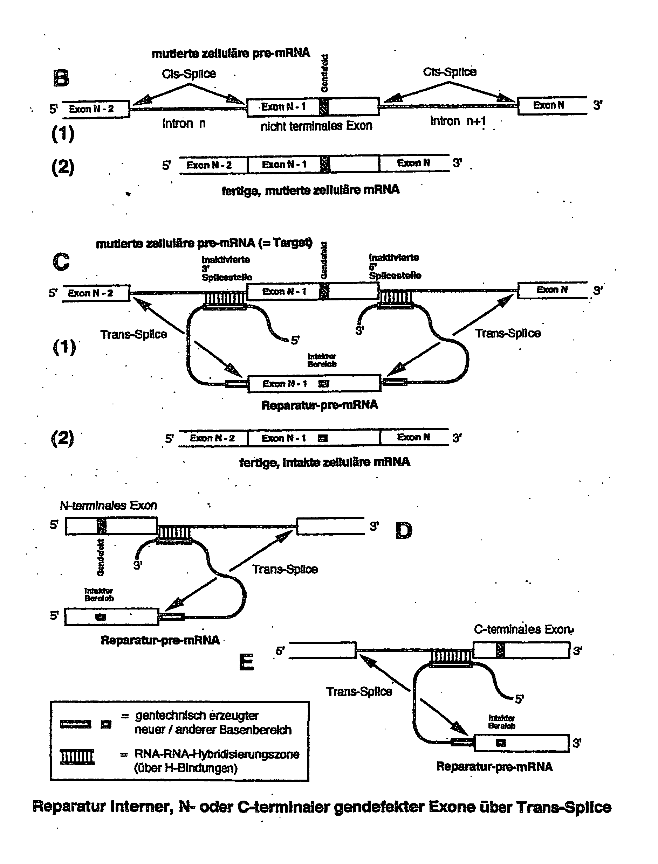

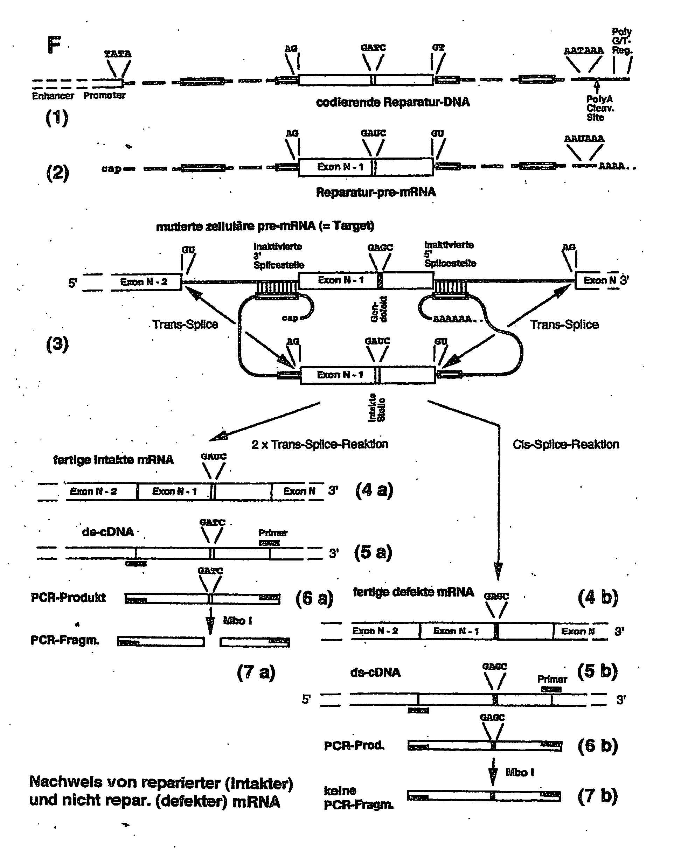

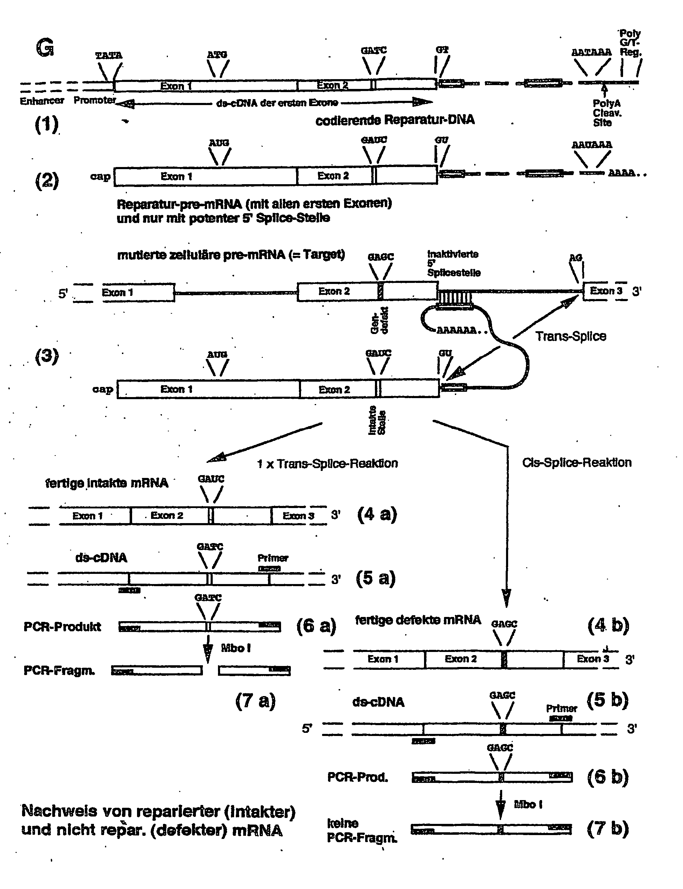

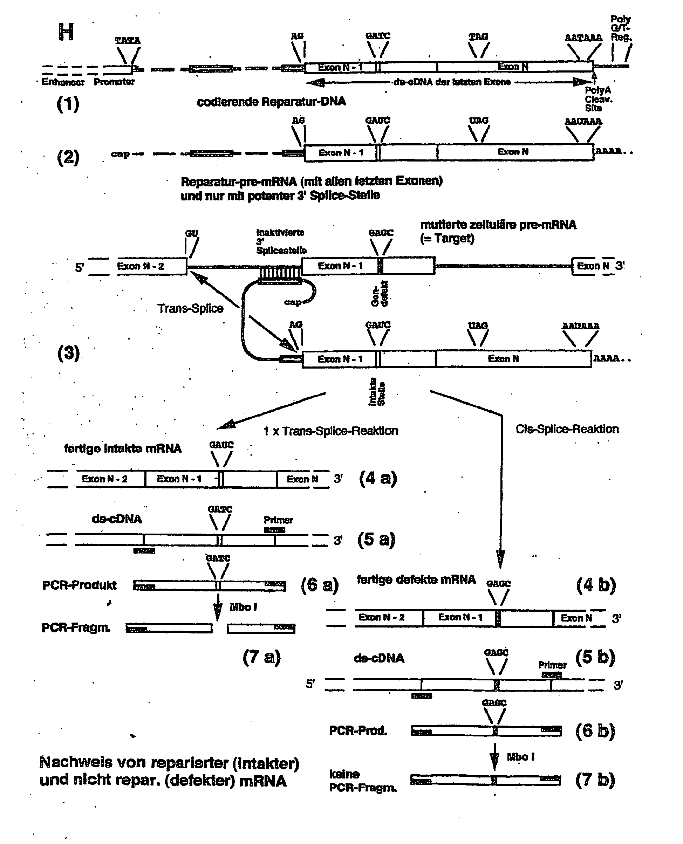

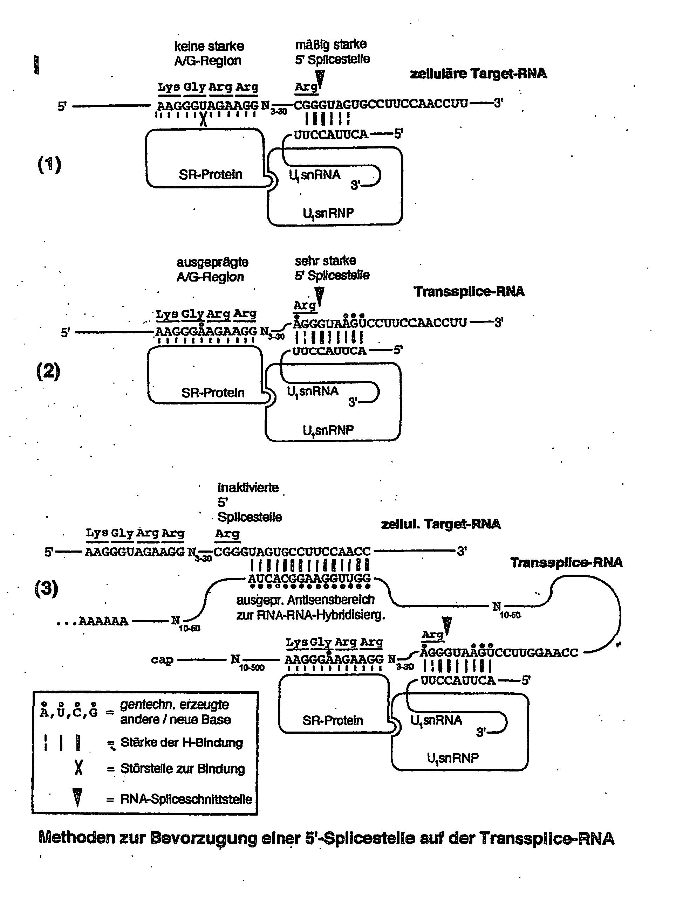

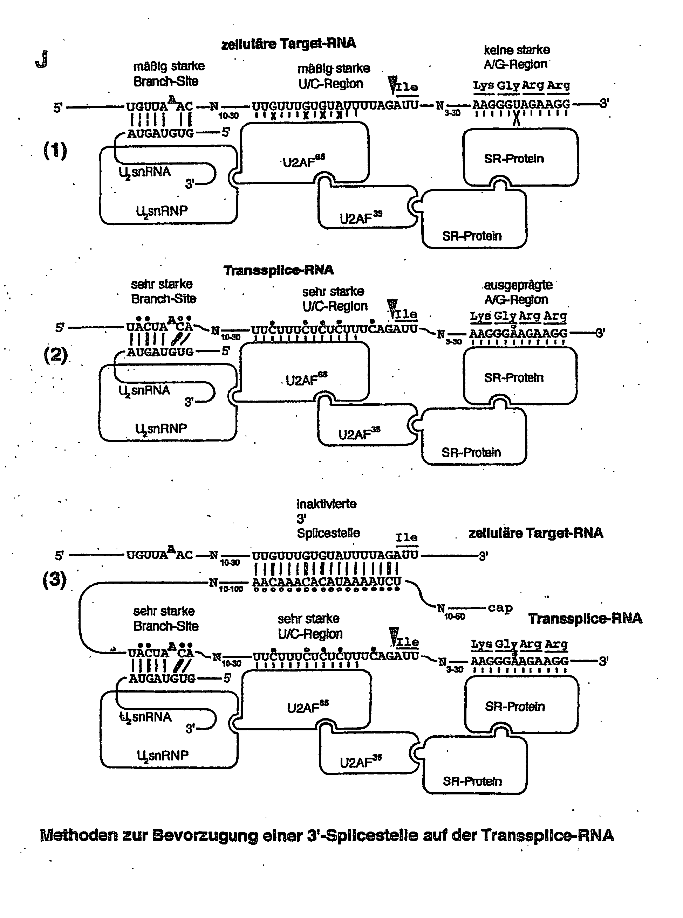

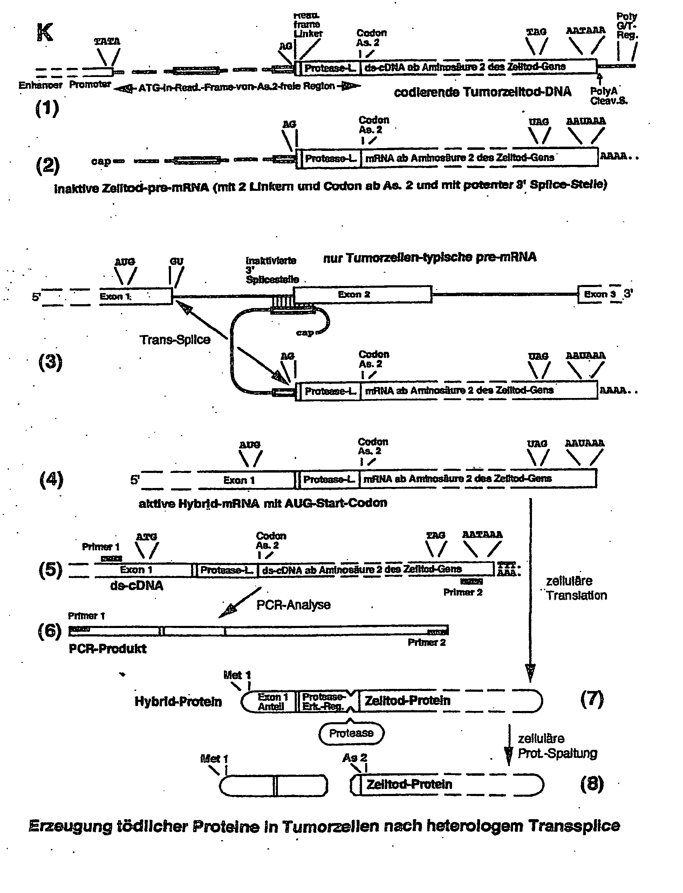

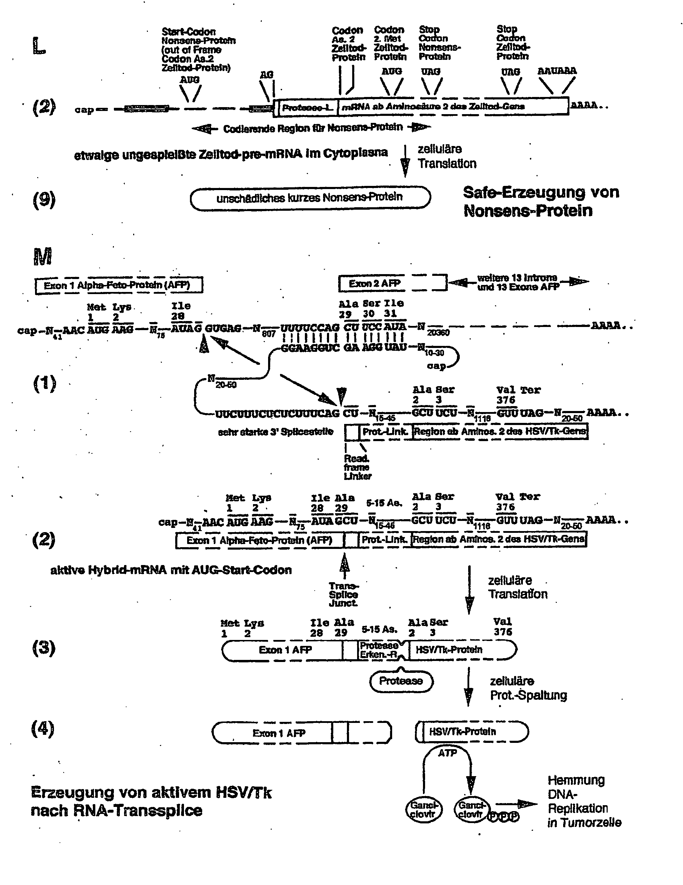

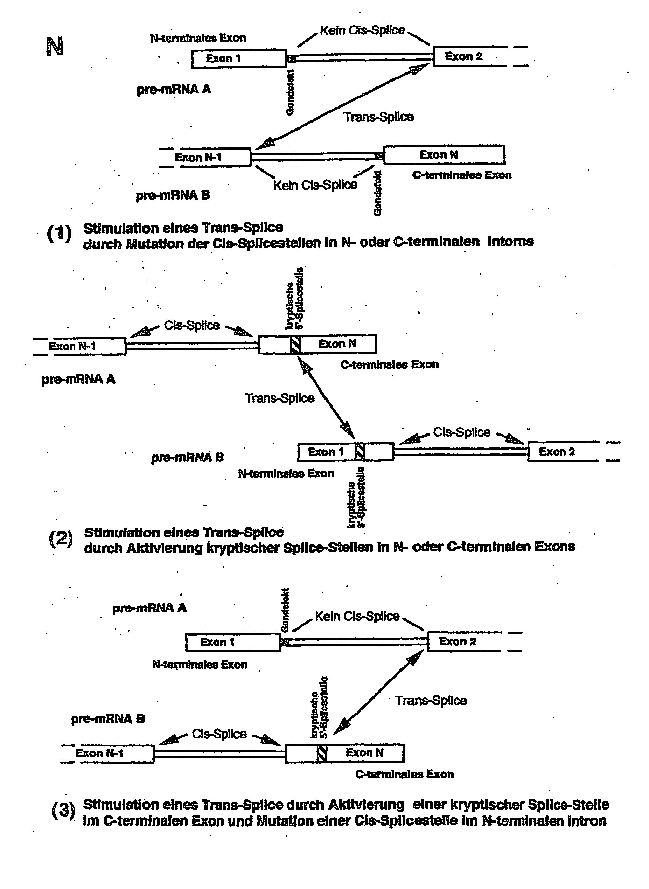

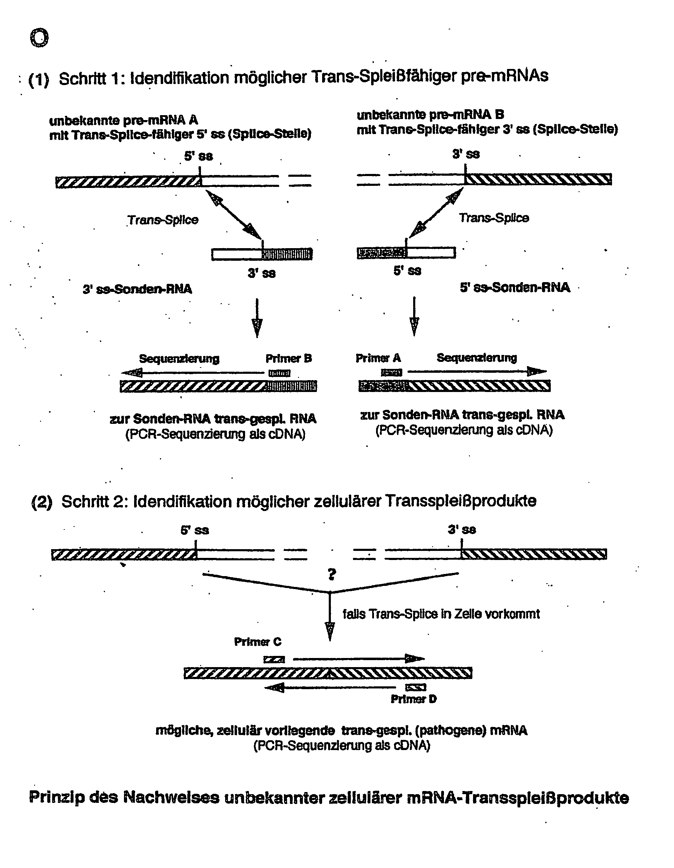

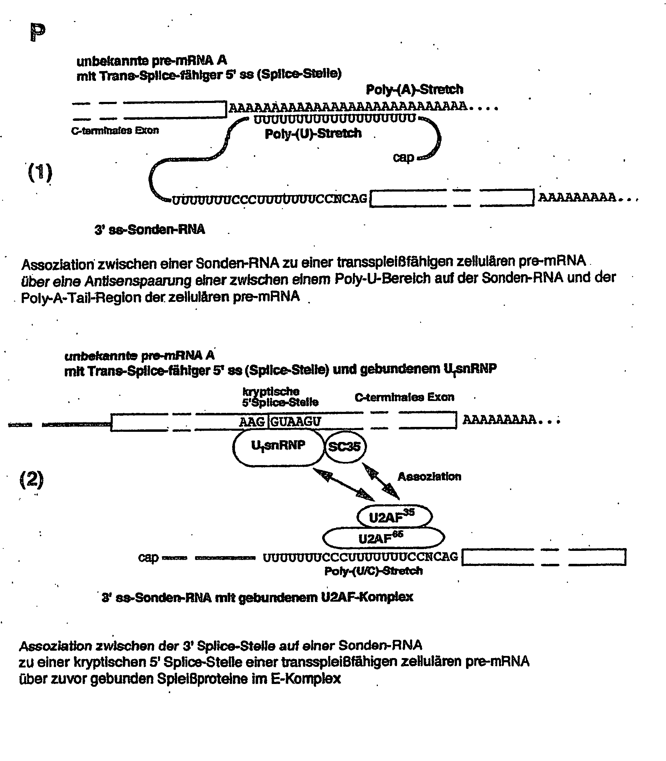

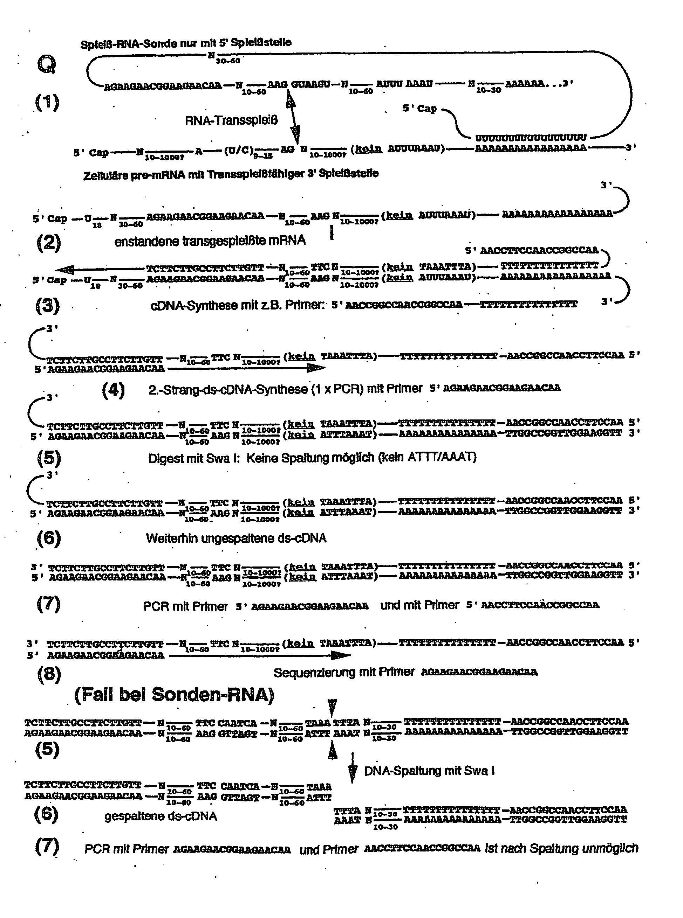

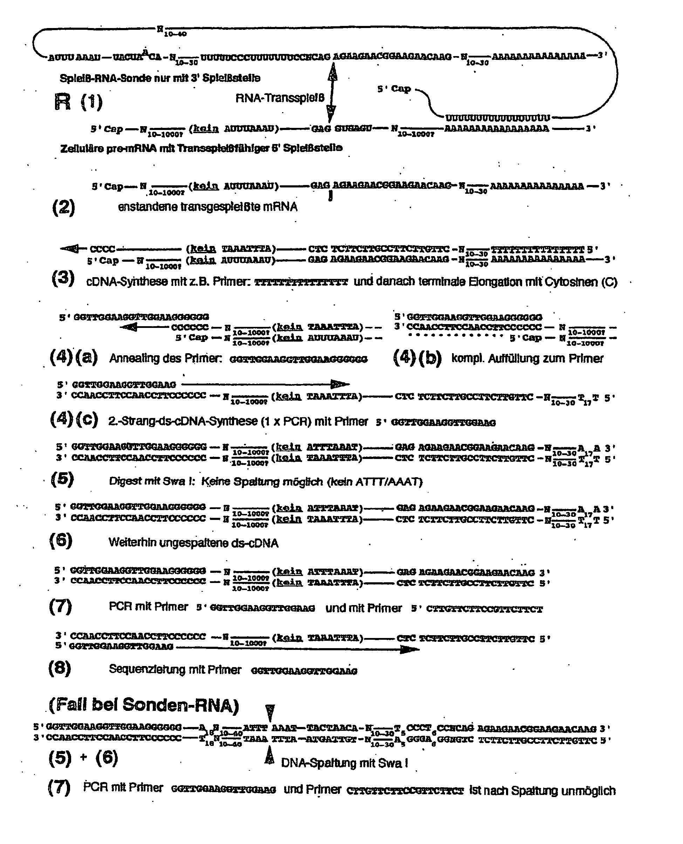

Die Reparatur-RNA könnte im unerwünschten Falle zu anderen nicht gewünschten zellulären pre-mRNAs über Antisensstrukturen annealen und eventuell dann dort auch Transspleißreaktionen ausführen (s.o.); was aber auch nach Annealing eher unwahrscheinlich ist, da die Reparatur-RNA z.B. an dieser anderen RNA höchst wahrscheinlich keine spezifische Blockierung der 5' und 3' Cis-Spleißstellen über AntisensStrukturen in diesem Bereich durchführt. Auch der Nachweis eventueller unerwünschter Transspleißprodukte erfolgt über eine cDNA-PCR: Nachdem zuvor über eine Computeranalyse mögliche Antisens-Bindungsstellen der Reparatur-RNA zu sonstigen RNA-Molekülen der Zelle aufgezeigt wurden (s.v.), geschieht die Analyse mittels einer cDNA-PCR, bei welcher ein PCR-Primer zum Exon der Reparatur-RNA und der andere Primer entsprechend auf dieser anderen zellulären RNA bzw. der abgeleiteten cDNA bindet (vgl hierzu genauer: Anwendungsfall 3 bzw. Fig. 01). Nur nach erfolgtem Transspleiß mit Bildung einer chimeren mRNA - abstammend aus der Repartur-RNA und einer ganz anderen RNA der Zelle - werden dann entsprechende cDNA-PCR-Produkte nachweisbar sein.

Je nachdem, ob ein internes oder ein terminales gendefektes Exon zu reparieren ist, hat die Reparatur-RNA, bzw. die die RNA codierende DNA, einen unterschiedlichen Grundaufbau:

Das zentrale Element der Reparatur-RNA ist immer das Reparatur-Exon, welches die richtige, korrigierte Geninformation trägt. Dieses Exon wird noch von ein oder zwei Intronhälften, genannt Outrons umgeben. Die Nucleotidsequenz der Outrons kann hierbei im wesentlichen der Nucleotidsequenz der entsprechenden Intronbereiche, die an der Grenze zum gendefekten Exon der zellulären RNA liegen, homolog sein. Basenveränderungen im Outron betreffen im wesentlichen nur Veränderungen in den Sequenzen der Spleißstellen sowie die künstliche Schaffung einer AntisensStruktur im Outron, damit die eingebrachte RNA hier im Outron zur gendefekten RNA paaren kann. Die Outrons enthalten den größeren Anteil der 5' Spleißsstellensequenz (bei einem hinteren (3') Outron) sowie alle Elemente der 3 ' Spleißstelle (bei einem vorderen (5') Outron) . Über diese Spleißstellen, die eine hohe Attraktivität für die Spleißproteine haben sollen, erfolgt durch eine einfache bzw. zweifache Transspleißreaktion der Austausch des Reparatur-Exons mit dem entsprechenden mutierten Exon der gendefekten zellulären RNA. Aufgabe dieser Outrons ist ferner über Antisensstrukturen im Outronbereich zur gendefekten RNA hin die spezifische Anbindung zwischen Reparatur-RNA und gendefeker RNA (Selektion) . Durch diese Antisens-RNA-RNA-Bindung wird darüber hinaus die Transspleißeffizienz wesentlich gesteigert und durch eine gleichzeitige Antisensblockierung der analogen Cis-Spleißstellen, der unerwünschte Cis-Spleiß, der zu einer gendefekten RNA führt, effektiv blockiert (s. Fig B, C, D, E).
A) Die Struktur der Reparatur-RNA zur Reparatur eines nicht terminalen gendefekten Exons (Übersicht s. Fig. C und F) hat demnach vorteilhafterweise folgenden Aufbau:
   (i) Struktur des vorderen (5') Outrons: Am 5' -Kopfende - also zu Beginn des vorderen Outrons - der Reparatur-RNA liegt eine cap-Sequenz, welche von der Zelle selbst erzeugt wird. Hieran schließt sich nach einer Spacerregion von n = ca. 10-50 Nucleotiden eine künstlich erzeugte Nucleotidregion an, die -aus Selektionsgründen - über eine Länge von mindestens 18 aufeinanderfolgenden Basen zur gendefekten RNA paart und dabei gleichzeitig die unerwünschte 3' CIS-Spleißstelle der gendefekten RNA über eine Blockierung des AG-Nucleotides und des Polypyrimidinbasenstretches inaktiviert (Fig. C und J3). Nach einer weiteren Spacerregion von n = ca. 10-100 Nucleotiden (Fig. J3) folgt eine künstlich erzeugte, sehr starke 3' Spleißregion. Diese besteht zunächst N-terminal aus einer starken Branch-A-Region, welche die Sequenz U-(A/G) -C-U- (A/G) -A enthält (s. Fig. J3). Nach einer weiteren Spacerregion von n = ca. 10-30 Nucleotiden folgt eine künstlich erzeugte, sehr starke Poly(U/C)-Region mit optimal 18 hintereinander liegenden U- oder C-Basen, gefolgt von der RNA-Nucleotidfolge AG, die das Ende der 3' -Spleißstelle und des vorderen Outrons der tranzuspleißenden RNA (Repartur-RNA etc.) darstellt.
   (ii) Struktur des Reparatur-Exons : Der RNA-Exonteil trägt über einen künstlichen Eingriff (auf der codierenden DNA) die entsprechende Mutation nicht mehr, das Exon weist also prinzipiell die komplette Wild-typ- (wt) - Sequenz der homologen RNA auf. Das Reparaturexon kann jedoch, abweichend von der wt-Sequenz der homoigen RNA, andere Basen im Austausch enthalten, sofern hierdurch keine anderen Aminosäuren codiert werden. Förderlich und anzustreben sind über gentechnologische Methoden auf Ebene der codierenden DNA erzeugte Sequenzen, die als sog. Exonische Splice Enhancer (ESE) fungieren und so die (Trans) Spleißeffizienz anheben. Als solche ESE-Sequenzen dienen z.B. Purin(A/G) -reiche Basenfolgen, welche zu Arginin-Serin (SR) -reichen-Spleißhilfsproteinen (SR-Proteine) binden können. Fig. I1 und I2 bzw. J1 und J2 zeigt beispielhaft, wie durch Basenaustausch nur eines Nucleotides (U zu A) ein solcher durchgehender A/G-Stretch als ESE-Sequenz erzeugt wird, ohne dass hierdurch andere Aminosäuren codiert werden.
      Sofern wie hier, auf das Exon ein weiteres Outron mit einer 5' Spleißstelle folgt, ist ferner im exonischen Anteil der 5' Spleißstelle (= letzte 3 Basen des Exons) durch Austausch von 1 - 2 Nucleotiden die Basenfolge (A/G) - (A/G) -G anzustreben, sofern hierdurch keine andere Aminosäure codiert wird. Die obige Basenfolge paart optimal zur U-₁nRNA des U₁nRNP und fördert deshalb eine (Trans) Spleißreaktion. Im Beispielfall der Fig. I1 wurde eine Base C durch einen gentechnologischen Eingriff durch eine Base A (s. Fig. I2) ersetzt, ohne dass hierdurch eine andere Aminosäure (stets Arg) codiert wird.
   (iii) Struktur des hinteren, distalen (3') Outrons: Das nachfolgende distale Outron beginnt zunächst mit den 6 intronischen Nucleotiden der 5' Spleißstelle. Hier ist durch einen gentechnologischen Eingriff auf der codierenden DNA die RNA-Nucleotidfolge G-U-A-A-G-U anzustreben (vgl. Fig I1 und I2), die optimal zur U₁SnRNA des U₁nRNP paart, wodurch eine hohe (Trans) Spleißeffizienz ereicht wird. Da die Basen des Outrons keine Aminosäuren codieren, sind hier alle willkürlichen Basenveränderungen im Vergleich zur Basenfolge des homologen Introns der gendefekten RNA möglich (vgl. Fig I1 und 12). An die 5' Spleißstelle schließt sich nach einer Spacerregion von n = ca. 10-100 Nucleotiden eine künstlich erzeugte Anitisens-Region an, die - aus Selektionsgründen - über eine Länge von mindestens 18 aufeinanderfolgenden Basen zur gendefekten RNA paart und dabei gleichzeitig die unerwünschte 5' Spleißstelle der gendefekten RNA über eine RNA-Antisensblockierung inaktiviert (Fig. C und 13). An diese Antisensregion schließt nach einer weiteren Spacerregion von n = ca. 10-50 Nucleotiden eine Polyadenylierungsstelle an. Die codierende DNA trägt hier die Erkennungsregionen zur Polyadenylierung, z.B. aus dem SV40 Early Gen, welche aus einer Sequenz AATAAA upstream zur Poly-A-Schnittstelle und einer GT-reichen Sequenz downstream der Poly-A-Schnittstelle besteht (s. Fig F1).
B) Die Struktur der Reparatur-RNA zur Reparatur eines nicht internen, N-terminalen codierenden, gendefekten Exons (s. Fig. D) besteht aus einem Reparaturexon gefolgt von einem Outron. Das Reparaturexon codiert - homolog dem gendefekten Exon - nur im hinteren Bereich für Aminosäuren, d.h. es enthält das Translationsstartcodon AUG. Für die Feinstruktur dieser beiden RNA-Elemente Exon und Outron gelten die vorherigen Ausführungen wie unter (ii) und (iii) beschrieben. Da die Reparatur-RNA nur eine Spleißstelle enthält, erfolgt hier nur eine einfache (statt zweifache) Transspleißreaktion hin zur gendefekten homologen zellulären RNA.
C) Die Reparatur-RNA zur Reparatur eines C-terminalen codierenden, gendefekten Exons (s. Fig. E) besteht umgekehrt aus einem vorderen Outron gefolgt von dem Reparaturexon. Für die Feinstruktur dieser beiden RNA-Elemente gelten ebenfalls die vorherigen Ausführungen unter (i) und (ii) mit der Ausnahme, dass das Reparaturexon entsprechend am hinteren Ende keine Nucleotide einer 5' Spleißstelle enthält. Das Reparaturexon codiert - homolog dem gendefekten Exon - nur im vorderen Bereich für Aminosäuren, d.h. es enthält einen Translationsstopcodon wie UAG etc . Das Reparturexon bzw. die hierzu codierende DNA enthält ferner am distalen Ende eine Erkennungsregion zur Polyadenylierung, wie dies unter (iii) ausgeführt ist (vgl. ähnl. Fig H1 und H2).
D) Neben dem Fall, dass das Reparaturexon nur ein authentisches, gendefektes Exon ersetzen muss, ist jedoch auch noch der Fall denkbar, dass die zu reparierende, zelluläre RNA in mehreren Exonen gendefekt ist. Sofern etwa zwei benachbarte Exone einer RNA je einen Gendefekt haben, könnte das Reparaturexon entsprechend aus einem einzigen neuen Exon bestehen, welches die beiden Geninformationen dieser beiden Exone enthält. Die das Reparaturexon codierende DNA leitet sich also aus der cDNA der mRNA nur aus diesen beiden Exonen ab. Das cDNA-abgeleitete Reparaturexon enthält dann wiederum ebenfalls zwei Transspleißstellen respektive ein proximales und ein distales Outron, oder nur eine Transspleißstelle respektive nur ein proximales oder distales Outron.

Ein besonderer Fall, der auch den Einsatz eines solchen cDNA-Reparaturexons begründen würde, liegt dann vor, wenn ein einziges Exon mutiert ist, das im vorderen oder hinteren Bereich der gendefekten RNA liegt, ohne das erste bzw. letzte codierende Exon zu sein. Im ersteren Fall könnte das entsprechende mutierte Exon der zellulären RNA z.B. das zweite codierende Exon sein (vgl. Fig. G) ; das entsprechende cDNA-Reparaturexon enthält dann die cDNA aus dem ersten und zweiten Exon. Nach dem cDNA-Exon folgt ein Outron mit einer 5' (Trans) Spleißstelle . Durch diese Konstruktion über eine cDNA im Reparaturexon wird quasi ein künstliches N-terminales Exon geschaffen (s. Fig. D) , es wird also nur ein einfacher Transspleiß (anstelle eines zweifachen Transspleißes) zur RNA-Reparatur notwendig. Dies bedeutet eine weitere Steigerung der Ausbeute an reparierten RNA-Molekülen, die naheliegend höher ist, wenn anstelle einer zweifachen (üblicher Fall bei der Reparatur eines internen Exons) nur eine einfache Transspleißreaktion zur Erzeugung einer intakten RNA notwendig ist. Der Transspleiß erfolgt hierbei zu demjenigen Exon der zellulären RNA, welches dem letzten Exon innnerhalb des cDNA-Exons der Reparatur-RNA folgt.

Der umgekehrte Fall gilt analog auch für einen Gendefekt etwa im zweit-letzten codierenden Exon (s. Fig. H) ; hier enthält das cDNA-Reparaturexon die zusammengefasste Geninformation aus dem vorletzten und letzten Exon. Upstream dieses cDNA-Exons liegt ein Outron, das alle Elemente einer 3' Spleißstelle enthält. Der singuläre Transspleiß findet also in diesem Fall zwischen dem vor-vor-letzten Exon der zellulären RNA und dem cDNA-Exon, welches mit dem vorletzten Exon beginnt, statt. Aufgrund des nur singulären Transspleißes ist auch hier mit einer Erhöhung der Ausbeute an intakten reparierten zelullären RNAs im Vergleich zu einem ansonsten zweifachen Transspleiß zu rechnen.

Eine Fortführung der Beispiele aus Fig. G oder H wäre, wenn das cDNA-Reparatur-Exon (fast) die gesamte mRNA-Information (und nicht nur einen kleineren Bereich) enthielte. Dieser Fall ist auch prinzipiell für eine RNA-Reparatur denkbar: In einer Ausführungsform A enthält das N-terminale Reparaturexon dann die cDNA-Folge aus der mRNA der codierenden Exone 1 bis N-1 (vor-letztes Exon) , der singuläre Transspleiß erfolgt zum nicht gendefekten letzten Exon N der zellulären RNA. In einer alternativen Ausführungsform B enthält das C-terminale Reparaturexon dann die cDNA-Folge aus der mRNA der codierenden Exone 2 bis N (letztes Exon) , der singuläre Transspleiß erfolgt zum nicht gendefekten ersten Exon 1 der zellulären RNA. Der Vorteil einer nur singulären Transspleißreaktion wird jedoch durch eine längere erforderliche DNA (s.u.) zur Erzeugung dieser langen Reparatur-RNA zumindest teilweise kompensiert.

Die Vorteile der Verwendung einer Transspleiß-Reparatur-RNA unter Ausnutzung der natürlichen zellulären Spleißproteine gegenüber dem herkömmlichen gentechnologischem Ersatz des kompletten Genes sind:

Bei einer Reparatur wird prinzipiell nur der defekte Teil ersetzt, während die nicht defekten Teile erhalten bleiben. Eine Reparatur ist also ökonomisch günstiger als ein vollständiger Ersatz, dies gilt nicht nur für hochwertige technische Geräte etc. sondern im medizinischen Bereich prinzipiell auch bei Reparaturen auf Organebene oder zellulärer Ebene. Wegweisend für die hier vorgestellte Technologie ist, dass ein defektes Gen nicht mehr komplett durch ein in diö Zellen zu schleusendes artifizielles Genprodukt (da u.a. auf cDNA-Ebene) ersetzt wird, wie bei der herkömmlichen somatischen DNA-Gentherapie. Das defekte Gen bleibt vielmehr funktioneil erhalten und wird nur an seiner defekten Stelle, quasi über ein mikrochirurgisches Verfahren repariert. Dieses Verfahren beinhaltet das Einbringen einer DNA mit speziellen Anforderungen (als Erzeugnis), die eine RNA codiert, welche über einen RNA-Transspleiß das defekte Gen speziell nur in seinem defekten Exon-Anteil (bzw. Exonanteilen) auf der Stufe der GenAbschrift, also der RNA repariert.

Aufgrund der weiteren Nutzung des defekten Genes bleiben auch dessen regulatorische Sequenzen erhalten, die cDNA-Gen-Konstrukte aus einer herkömmlichen Gentherapie nicht innehaben. Zu erwähnen sind hier vor allem die Intronbereiche, welche beim natürlichen Gen alternative Spleißmöglichkeiten zulassen, wobei das Verhältnis der mRNA-Spleißprodukte untereinander auch wiederum durch regulatorische Sequenzen innerhalb der Intronbereiche selber geregelt wird. Diese Intronbereiche des natürlichen Genes sowie andere Bereiche, die oft weit entfernt etwa vom Promotor liegen, können zudem regulatorische Sequenzen besitzen, die eine differenzierte RNA-Expression im zellulär physiologischen Kontext gestatten, die jedoch bei cDNA-Gen-Ersatz-Konstrukten fehlen.

Neben der Aufrechterhaltung der natürlichen Genregulation bei einer Beibelassung des defekten Genes und nur einer Reparatur des defekten Genanteiles hat die hier beschriebene Technologie gegenüber herkömmlichen gentherapeutischen DNA-Verfahren noch einen weiteren Vorteil: Die DNAs, welche die Reparatur-RNAs mit nur einem Exon und zwei endständigen Outrons codieren, können recht klein bleiben: ca. 300 bis 500 Nucleotide Länge inklusive Polyadenylierungsstelle zuzüglich der Größe des Promotors (ca. 300-500 Nucleotide) . Diese DNAs, die eine Reparatur-RNA codieren, sind damit wesentlich kürzer selbst als eine komprimierte cDNA eines Genes (ca. 1,5 - 2,5 kb Länge), was die Möglichkeiten bzw. Effizienzen des Gentransfers in die Säugerzellen stark erhöht. Als Beispiele seien genannt der Gentransfer über Adenovirus assoziierte Viren (AAV) , die bevorzugt eher kurze Transgene aufnehmen, sowie auch Möglichkeiten des direkten Gentransfers ohne die Zuhilfenahme viraler Vektoren (z.B. über elektrische Felder etc.): Insbesondere die letzteren Verfahren sind nur effizient bei kleineren, in die Zellen zu bringenden Partikeln, also bei relativ kurzen DNAs.

Ein prinzipiell anderes Einsatzgebiet von cDNA-Exonen (s.o.) aus einer eingebrachten RNA in Transspleißreaktionen liegt jedoch vor, wenn hierüber nicht eine Genreparatur über ein homologe, intakte RNA durchgeführt werden soll. Das transzuspleißende Exon kann demnach auch Gensequenzen aufweisen, die aus völlig anderen Genen der Säugerzelle oder aus Genen von niederen Lebewesen abstammen oder gar viralen Ursprunges sind. Ein konkreter Anwendungsfall hierzu wäre, dass das nach Transspleiß entstandene Hybrid-mRNA-Produkt, bzw. das hieraus entstandene Protein, irgendwelche neue Aufgaben in der Zelle übernimmt. Die potentiellen Möglichkeiten des Einsatzes der Transspleißtechnologie zur Schaffung derartiger, nicht natürlich vorkommender Hybridproteine, die eventuell zur

Lösung bestimmter Aufgaben dienen können, sind noch nicht übersehbar. Als Beispielfall (Anwendungsfall 2 der Erfindung) können nach einem derartigen RNA-Transspleiß funktioneile Proteine codiert werden, die z.B. spezifisch die Abtötung von Tumorzellen bewirken.

Die spezifische Abtötung oder Eliminierung von Tumorzellen ohne Normalzellen zu sehr zu schädigen, ist ein noch nicht zufriedenstellend gelöstes P oblem. Eine chirurgische Tumorentfernung gilt nur bei noch nicht ausgebildeten Metastasen als möglicherweise erfolgreich. Ansonsten oder bei inoperablen Tumoren (z.B. Hirntumore) sind zur Zeit nur eine Strahlentherapie oder eine Chemotherapie gängige Anwendungsverfahren. Beide Verfahren schädigen auch Normalzellen, bei der Chemotherapie werden insbesondere alle schnell-teilenden Zellen abgetötet, das sind neben Tumorzellen vor allem Blutstammzellen etc., so dass nach einer Hochdosis-Chemotherapie in der Regel eine Spende von Knochenmark mit neuen BlutStammzellen usw. erforderlich ist. Erfolgversprechende Alternativen etwa zur Chemotherapie sind wiederum gentechnologische Methoden. Vom Lösungsansatz her gibt es hier drei Prinzipien: A) Beseitigung des tumorauslösenden Gendefektes, B) Ausschaltung von aktiven Onkogenen, C) Selektive Abtötung der Tumorzellen. Zu A) Insbesondere die bei Tumorerkrankungen sehr häufigen Gendefekte im p53-Gen werden durch Einschleusen eines intakten p53-Gens kurativ kompensiert; hier ist, wie üblich, das eingebrachte p53-Gen aber wiederum die cDNA dieses Genes mit den zuvor beschriebenen Nachteilen einer komprimierten cDNA. Auch bei diesen Tumorursachen bietet eine gezielte Transspleiß-Reparatur etwa nur des gendefekten Exons mittels einer spezifischen Reparatur-RNA entsprechende Vorteile (s.v.) . Zu B) Bei der Ausschaltung aktiver Onkogene gibt es verschiedene Möglichkeiten, wie eine Blockierung der entsprechenden Promotoren oder eine Inaktivierung/Eliminierung der gebildeten Onkogen-RNAs. Zu C) Der dritte, eliminierende Absatz zielt hingegen darauf ab, Tumorzellen gezielt abzutöten, ohne Normalzellen dabei in Mitleidenschaft zu ziehen. Wegen dieser Randbedingung ist dieser Ansatz gentechnologisch nur schwierig zu lösen. Im folgenden Anwendungsfall 2 der vorgestellten Erfindung wird eine Problemlösung vorgestellt, welche wiederum auf der Ausnützung der RNA-Transspleißkapazität aller Säugerzellen (Normalzellen und Tumorzellen) basiert und bei welcher die eingebrachte transzuspleißende RNA, wie im Anwendungsfall 1 beschrieben, aus einem Outron und einem Exon besteht, wobei das Exon wiederum selektiv transgespleißt wird.

Tumorzellen unterscheiden sich von Normalzellen dadurch, dass diese bestimmte Proteine ausbilden, die zum bösartigen Zellwachstum führten. Diese Proteine werden demzufolge von bestimmten RNAs in der Zelle codiert, welche entsprechend nur in Tumorzellen, nicht aber in Normalzellen vorliegen. Als Beispiele seien hier genannt die RNAs der Onkogene Ras, Myc usw., sowie z.B. die RNA des alpha-Fetoproteins (AFP) (erhöhte Gehalte im Hepatocellulärem Karzinom) oder die RNA des Carcino-embryonischen Antigens (CEA) (erhöhte Konzentrationen in Lebermetastasen) .

Im Nachfolgenden wird ein Verfahren vorgestellt, wie diese nur in den Tumorzellen vorkommenden RNAs letztlich zum selektiven Zelltod der Tumorzellen führen, ohne dass die Normalzellen, die jene RNAs nicht ausbilden, hierbei geschädigt werden. Das Verfahren beruht im Prinzip darauf, in alle Zellen (da eine Selektion zwischen Tumorzellen und Normalzellen nicht möglich ist) eine DNA einzubringen, die eine RNA' eines Proteins codiert, welches direkt oder indirekt zum Zelltod führt. Wesentlich ist jedoch, dass diese eingebrachten Zelltod-RNAs künstlich verkürzt sind, aus welchen noch kein funktionsfähiges Zelltodprotein entstehen kann. Nur wenn also diese unvollständigen Zelltod-RNAs ergänzt bzw. verlängert werden, kommt es zum Tod der Zelle. Als Mittel zur Ergänzung dient hierbei wiederum eine tumorspezifische RNA (AFP, CEA etc.), die Ergänzung selbst erfolgt über einen RNA-Transspleißmechanismus unter Ausnutzung der üblichen Spleißproteine etc. der Säugerzelle. Die Selektivität zur Tumorzellen-spezifischen RNA wird hierbei, wie zuvor beschrieben, über entsprechende, künstlich geschaffene Antisensstrukturen auf der unvollständigen Zelltod-pre-mRNA erreicht. Die nach Transspleiß-Ergänzung erhaltene vollständige Zelltod-mRNA codiert dann für das entsprechende Zelltodprotein, das auf direktem Wege oder indirektem Wege selektiv zum Tod der Tumorzellen führt.

Als direkte oder indirekte "Zeiltodproteine" kommen hierbei prinzipiell in Frage: a) direkte Toxine, die zum Absterben der Zellen führen (z.B. das Diphtherie-Toxin etc.), b) Proteine, die einen Zelltod über einen Apoptose-Kaskadenmechanismus auslösen (z.B. Caspase 8) und c) Enzyme, die in den Stoffwechsel der Zelle eingreifen, und bei Vorliegen eines betreffenden Substrates (als "Medikament" extern zuführbar) das spezifische Substrat so verwenden, dass dadurch letztlich der Zelltod ausgelöst wird.

Im letzten, obigen Fall kann das Zelltodprotein beispielsweise das virale Enzym HSV-Thymidin-Kinase (HSV-TK) sein, welches zunächst untoxisch.es Ganciclovir (Diazothymidin) , das als "Medikament" zugeführt wird, phosphoryliert . Nur HSV-TK, nicht jedoch die normale Thymidinkinase der Säugerzelle kann Ganciclovir in das aktive phosphorylierte Derivat umsetzen. Das phosphorylierte Ganciclovir wird dann bei der Replikation der Gesamt-DNA der Tumorzelle in die replizierte DNA-Kette eingebaut; dort führt es jedoch wegen der Diazogruppe zum Kettenabbruch, was die Einstellung der DNA-Replikation und damit den Zelltod der Tumorzelle bedeutet (s. Fig. M4).

Die Unvollständigkeit der Zeiltod-pre-mRNA kann über verschiedene Wege erreicht werden, z.B. indem diese RNA infolge einer fehlenden Poly-A-Erkennungsregion nicht polyadenliert werden kann, und deshalb u.a. sofort abgebaut wird bevor es zur Proteinbiosynthese (Translation) kommt. Die Unvollständigkeit der Zelltod-pre-mRNA beruht in der hier näher ausgeführten Form darin, dass diese RNA zwar die Codons der Aminosäuren 2 bis zur letzten Aminosäure des Zelltodproteines trägt, die erste Aminosäure (Met) und damit insbesondere der Translations-Startcodon AUG zur Synthese des funktionellen Zelltodproteins fehlt jedoch auf dieser RNA. Dieses Startcodon AUG wird über einen spezifischen RNA-Transspleiß von einer entsprechenden Tumorzellen-spezifischen pre-mRNA bezogen. Der RNA-Transspleiß vollzieht sich dabei zwischen einer 3' Spleißstelle upstream des Codonstranges ab Aminosäure 2 des Zelltodproteins auf der unvollständigen Zelltod-pre-mRNA und der 5' Spleißstelle am Ende des ersten codierenden Exons auf der Tumorzellspezifischen RNA, welches den Startcon AUG trägt (s. Fig. K3).

Die erforderliche Spezifität zu dieser Spleißstelle auf der Tumorzellen-spezifischen-pre-mRNA (z.B. AFP-RNA) wird dadurch erreicht, dass die transzuspleißende Zelltod-pre-mRNA in ihrem Outronanteil oberhalb ihrer 3' Spleißstelle (d.h. oberhalb der Poly/-U/C-Region bzw. der Branch-A-Region) eine Folge von 18 oder mehr Nucleotiden trägt, die in Antisens spezifisch zur Tumorzellen-pre-mRNA (nicht jedoch zu anderen zellulären pre-mRNAs) paart. Günstigerweise liegt die betreffende Antisens-Hybridisierungsregion im Bereich der 3 ' Spleißstelle des codierenden Exons 2 der Tumorzeil-pre-mRNA, sodass hierduch diese 3' Cis-Spleißstelle blockiert wird, womit keine mögliche Cis-Spleißreaktion zwischen Exon 1 und Exon 2 der Tumorzellpre-mRNA mehr stattfindet, sondern nur die gewünschte Trans-Spleißreaktion zur konkurrierenden 3' Spleißstelle auf der Zelltod-pre-mRNA (s. Fig. M1, K3).

Nach dem Transspleiß wird dann eine Hybrid-mRNA (abstammend aus 2 Gensequenzen) erhalten (s. Fig. K4, M2). Diese enthält nun das translatierbare erste Exon aus der Tumorzellen-spezifischen pre-mRNA (z.B. der AFP-RNA) - und damit den Startcodon AUG zur Proteinsynthese - sowie das Exon aus der unvollständigen Zelltod-pre-mRNA, welches abgeleitet aus einer cDNA die Codon-Sequenzen der Aminosäure Nr. 2 bis zu letzten Aminosäure des Zelltodproteins (z.B. des HSV-TK-Proteins) trägt.

Die transspleißbare unvollständige Zelltod-pre-mRNA besteht in ihrer Grundstruktur demnach - wie schon zur Beschreibung im Anwendungsfall 1 zur Reparatur-RNA prinzipiell ausgeführt - aus einem distalen Exon und einem vorderen Outron und enthält folglich nur eine Spleißstelle in Form einer 3' Spleißstelle.

Die wesentlichen Strukturelemente der unvollständigen Zelltod-pre-mRNA sind hier ein codierendes Exon, welches - abgeleitet von einer cDNA - die Aminosäure 2 bis zur letzten Aminosäure des Zelltodproteins und distal davon die Erkennungsregion zur Polyadenylierung der RNA enthält; d.h. die codierende DNA beinhaltet die Folge AATAAA upstream und eine GT-reiche Sequenz downstream (z.B. aus SV 40) der RNA-Polyadenylierungsstelle . Sofern hierdurch keine anderen Aminosäuren codiert werden, können durch Basenaustausch einzelner Nucleotide durch gentechnologische Methoden wiederum z.B. A/G-reiche oder andere spezielle Regionen im Exon geschaffen werden, die über Bindung von S/R-Proteinen etc. als Spleißenhancer (ESE) dienen (vgl. Fig. J).

Das proximale Outron enthält insbesondere eine hochaktive 3' Spleißstelle, die eine Folge von 15-18 U/C-Basen unmittelbar upstream des Dinucleotides AG umfasst, sowie upstream des Polypryrimidinbasenstretch.es eine starke Branch-A-Region, welche die Nucleotidfolge U- (A/G) -C-U-(A/G) -A enthält. Im Outron, oberhalb der Branch-A-Region findet sich zudem eine Folge von - aus Gründen der Selektionsspezifität (s.v., Anwendungsfall 1) - mindestens 18 Nucleotiden, die in Antisens zu einer bestimmten tumorspezifischen pre-mRNA als Transspleißpartner paaren. Durch die Antisenshybridisierung ist insbesondere eine Blockierung der 3' Spleißstelle (in deren Polypyrimidinbasenstretch) vor dem zweiten codierenden Exon der tumorzellspezifischen RNA anzustreben. Die Antisenspaarung und damit der stabile Kontakt zwischen den beiden tranzuspleißenden RNA-Molekülen erhöht zudem die Transsspleißeffiziens wesentlich.

Damit eine hohe zelluläre Konzentration der unvollständigen Zelltod-pre-mRNA und damit eine hohe Transspleißeffiziens erreicht wird, soll die die RNA codierende DNA einen starken Promotor (Fig. K1), wie z.B. aus SV40, tragen. Weiterhin soll diese codierende DNA einen starken Replikations-Origin (z.B. auch aus SV40) aufweisen, damit sich die DNA in den Zellen selbständig replizieren kann.

Im speziellen Fall des Einsatzes einer unvollständigen Zelltod-RNA zum selektiven Abtöten von Tumorzellen sollte diese Zelltod-pre-mRNA im Exon und im Outron jedoch über die obigen Strukturelemente hinaus noch weitere Strukturelemente aufweisen, die bestimmte Funktionen erfüllen.
i) Das codierende Exon 1 der tumorzellenspezifischen RNA enthält den benötigten Startcodon AUG sowie in der Regel in Folge daran bis zum Spleißstellenende des Exons noch die Codons für weitere Aminosäuren, wie dies beispielhaft in Fig. M angegeben ist; bei der AFP-RNA etwa codiert das Exon 1 für 28 Aminosäuren, die letzte ist Isoleucin (s. Fig. M1). Diese Aminosäurenfolge aus dem Exon 1 einer tumorzellenspezifischen RNA ist also Bestandteil der transgespleißten Hybrid-RNA und damit auch des HybridProteins und könnte deshalb unter Umständen die Funktion des Zelltodproteins beeinträchtigen. Sofern solches durch entsprechende Experimente nachgewiesen wurde, müssen geeignete Lösungswege zur Beseitigung dieser störenden Aminosäuren ergriffen werden. Eine praktikable Möglichkeit besteht darin, den störenden Peptid-Anteil aus dem tumorzellenspezifischen Protein im Hybridprotein nach Synthese des Hybridproteins abzuspalten. Dies geschieht dadurch, indem durch einen gentechnologischen Eingriff auf der unvollständigen Zelltod-pre-mRNA bzw. der diese RNA codierenden DNA im Exonbereich direkt oberhalb der Nucleotidfolge, die ab Aminosäure Nr. 2 des Zelltodproteins codiert, eine Folge von ca. 15 bis 45 Nucleotiden eingebaut werden, die für eine Peptidfolge von 5 bis 15 Aminosäuren codieren, welche durch bestimmte zelluläre, stets vorhandene Proteasen gespalten werden können (Protease-Erkennungsregion, s. Fig. K1 bis K4). Nach Fertigstellung des Hybridproteins wird dieses dann durch eine spezifische zelluläre Protease in der Protease-Erkennungsregion gespalten und es entsteht dann das fertige Zelltodprotein, beginnend ab Aminosäure 2 (die Aminosäure 1, also ein Methionin fehlt lediglich) , welches upstream dieser Aminosäure 2 noch einige wenige Aminosäuren aus der gespaltenen, künstlichen Protease-Erkennungsregion trägt, die aber die Zelltod-Funktion des Zeiltod-Proteins nicht beeinträchtigen sollten (s. Fig. K8, M3).
ii) Wie oben beschrieben, enthält die transgespleißte RNA das Translationsstartcodon AUG aus dem codierenden Exon 1 der tumorzellspezifischen RNA. Dieses Startcodon muss jedoch nun nicht unbedingt nach einem RNA-Transspleiß mit dem Leserahmen der Nucleotide der Aminosäuren des Zelltodproteins übereinstimmen. Sofern dies nicht der Fall ist, muss ein Shift des Leserahmens durch ein Einfügen von 1 oder 2 Nucleotiden ustream des Codons der zweiten Aminosäure des Zelltodproteins und der Proteaserkennungsregion erfolgen. Diese 1 oder 2 zusätzlichen Nucleotide bilden also eine Frameschift-Region bzw. einen Reading-Frame-Linker und liegen direkt upstream der Proteaseerkennungsregion direkt am vorderen Ende des Exons (s. Fig. K1 bis K5). Die 1-2-Basen-Frameshift-Sequenz muss zudem so gewählt sein, dass in Kombination mit den zum Codon überzähligen 1 oder 2 Nucleotiden der 5' Spleißstelle der Tumorzellen-RNA hierdurch keine Translationstermination (Folge UAG etc.) entsteht; zudem sollte durch das neue Basentriplett eine einfache Aminosäure (Gly, Ala) codiert werden (Fig. M2). Im angegebenen Beispiel aus der Fig. M1 liegt der Startcodon AUG im Exon 1 der AFP-RNA, als Beipiel einer Tumorzellen spezifischen RNA so, dass der exonische Anteil der 5' Spleißstelle nicht mit einem kompletten Codon-Triplett abschließt. Es bleibt hier ein einzelnes Nucleotid (hier ein G) übrig, welches zur Aufrechterhaltung des Leserahmens durch 2 weitere Nucleotide (hier CU) im Exonanteil der 3' Spleißstelle der transzuspleißenden Zelltod-RNA ergänzt werden muß (s. Fig. M1, M2).
iii) Eine letzte wesentliche Voraussetzung an die unvollständige Zelltod-pre-mRNA ist, dass diese für sich alleine, d.h. ohne Transspleißreaktion zu einer tumorzellspezifischen RNA, nicht für ein N-terminal verkürztes Protein codiert, was dennoch auch noch den Zelltod auslösen könnte. Die unvollständige Zelltodprotein-RNA hat zwar nicht mehr das authentische AUG zum Tanslationsstart und zur Synthese der ersten Aminosäure (Methionin) des Zelltodproteins, jedoch mit hoher Wahrscheinlichkeit weitere Codons AUG," die downstream des Codons AUG für die erste Aminosäure Methionin liegen. Diese nachfolgenden AUG-Codons auf dieser Zelltod-RNA können dann theoretisch ebenfalls als Translationsstart genommen werden. Sofern ein AUG-Codon außerhalb des Readingframes des Zelltodproteins als Translationsstart genommen wird, entsteht ein Nonsens-Protein, welches inaktiv ist. Sofern jedoch ein AUG-Codon downstream des urprünglichen AUG für Met 1 im Leserahmen des Zelltod-Proteins als Translationsstart verwendet wird, entsteht ein N-terminal verkürztes Zelltodprotein. Dieses könnte unter Umständen noch voll aktiv sein, sofern der N-terminal fehlende Aminosäuren-Anteil für die Zelltod-Funktion unerheblich ist. Dieses ist in präklinischen Zellexperimenten entsprechend zu prüfen. Bei einer Bestätigung dieser Annahme muss die unvollständige Zelltod-pre-mRNA dann so geschaffen sein, dass eine Verwendung des AUG, codierend für das zweite Met (vgl. Fig. L2) als Translationsstart ausgeschlossen wird. Dies wird, wie hier in einem Ausführungsbeispiel angegeben, einfach dadurch erreicht, dass oberhalb dieses AUG (für Met 2) ein anderes AUG eingefügt wird, welches bei der nichttrans-gespleißten pre-mRNA als AUG-Translations-Startcodon dient, so dass die Benutzung des AUG für das zweite Methionin als Translationsstart des Zelltodproteins unmöglich wird. Dieses AUG-Startcodon liegt - bedingt durch einen gentechnischen Eingriff - im vorderen Outron oberhalb der der 3' Spleißstelle der Zelltod-pre-mRNA (Fig. L2) ; zudem sollte dieses AUG-Startcodon außerhalb des Leserahmens des AUG für das 2. Met des Zelltodproteins liegen. In diesem Fall wird aus der nicht-transgespleißten Zelltod-pre-mRNA nach dem Translationsstart aus diesem vorderen AUG-Startcodon ein unschädliches Nonsenseprotein gebildet (Fig. L9) , wodurch eine Benutzung des Translätionsstartcodons AUG für das 2. Met des Zelltodproteins ausgeschlossen wird. Das Terminationssignal (UAG) dieses Nonsensproteins liegt im vorderen Exonteil, hinter dem AUG-Codon für das 2. Methionin des Zelltodproteins (s. Fig. L2).

In Zellkulturversuchen ist auch hier, wie im Anwendungsfall 1 bei der Reparatur-RNA beschrieben, dann nach Einbringung dieser Zelltod-pre-mRNA zu überprüfen, mit welcher Effiziens der gewünschte RNA-TransspleiB zwischen der tumorzellspezifischen RNA und der unvollständigen Zelltod-pre-mRNA abläuft, und ob die gebildete Hybrid-mRNA dann letztlich den Tod dieser Tumorzellen auslöst. Die Menge an gebildeter Hybrid-RNA, und damit die Effiziens der Transspleißreaktion, kann über eine einfache cDNA-PCR nachgeweisen werden. Durch Auswahl entsprechender 2 Primer, die zum einem zum Exon 1 der Tumorzellen-spezifischen-RNA und zum anderen zum Exon der unvollständigen Zelltod-RNA binden, werden in der nachfolgenden cDNA-PCR nur entsprechend transgespleißte mRNAs erfasst (s. Fig. K5 und K6). In weiteren Untersuchungen ist zudem zu überprüfen, dass die unvollständige Zelltod-pre-mRNA ausschließlich zur Tumorspezifischen RNA, nicht jedoch unerwünschterweise zu einer anderen zellulären RNA transspleißt (zur Vorgehensweise s. Beschreibung zum Anwendungsfall 1 zur Reparatur-RNA) . Insgesamt gesehen soll also die eingebrachte unvollständige Zelltod-pre-mRNA die Tumorzellen selektiv abtöten, ohne jedoch die Normalzellen zu schädigen, was über entsprechende Zellkulturexperimente mit Tumorzellen und Normalzellen überprüft werden kann.

Die DNA, welche diese unvollständige Zelltod-pre-m-RNA codiert, die danach über einen spezifischen Transspleiß zu einer Tumorzellen-RNA vervollständigt wird, kann unter Berücksichtigung der zuvor beschriebenen Bedingungen folgende Struktur (von 5' nach 3') aufweisen:
a) Am vorderen DNA-Ende liegt ein starker Replikationsorigin (z.B. aus SV40).
b) Es folgt ein starker Promotor mit Enhancer (z.B. aus SV40) (s. Fig. K1).
c) 10-30 Nucleotide hinter dem Promotor liegt eine Folge von 18 oder mehr Nucleotiden, die - durch einen gentechnologischen Eingriff- zur Tumorzellenspezifischen pre-mRNA in Antisens exakt zum PolyPyrimidinbasen-Stretch und dessen Umgebung der 3' Spleißstelle des zweiten Exons paart, welches dem ersten Exon mit dem AUG-Startcodon folgt (s. Fig. K3 , M3).
d) 40-60 Nucleotide hinter dem Promotorende (bzw. einige Nucleotide hinter der obigen Antisenspaarungsregion) liegt ein Codon ATG out-of-frame zum Leserahmen des Zelltodproteins im nachfolgenden Exon, welcher im Falle eines nicht stattfindenden Transspleißes ein unschädliches kurzes Nonsensprotein initiiert (vgl. Fig. L2).
e) 10-30 Nucleotide hinter dieser Antisensregion liegt eine starke Branch-A-Region einer 3 ' Spleißstelle mit der Sequenz T-A/G-C/T-T-A/G-A-C/T-A/G.
f) 10-30 Nucleotide hinter dieser Branch-A-Region liegt ein durchgehender Polypyrimidinbasenstretch (Poly-T/C) von 15-18 mer Länge gefolgt vom Dinucleotid A-G der 3' Spleißstelle (= hinteres Ende des vorderen Outrons) .
g) Das folgende Exon beginnt mit einem Readingframe-Linker aus 0, 1 oder 2 Nucleotiden zum Ausgleich eines evtl. verschiedenen Leserahmens zwischen dem nachfolgenden Zelltodprotein in diesem Exon und dem Exon 1 aus der transgespleißten, tumorzellenspezifischen RNA.
h) Auf diese sehr kurze Region (0 - 2 nt) folgt eine Region von 15 bis 45 Nucleotiden, welche für eine Folge von 5-15 Aminosäuren codiert, die durch zelluläre Proteasen gespalten werden kann.
i) An diese Proteaseerkennungsregion schließt sich eine cDNA, codierend für die Aminosäuren 2 bis zur letzten Aminosäure des Zelltodproteins, gefolgt von einem Stopcodon, z.B. TAG, an.
j) Im Bereich dieser cDNA-Region liegt downstream des ATG-Codons für das 2. Methionin des Zelltodproteins und out-of-frame zu diesem ein Stopcodon für das gebildete Nonsensprotein, das aus der nicht transgespleißten unvollständigen Zelltodprotein pre-mRNA entsteht (vgl. Fig. L2) .
k) 10 - 50 Nucleotide hinter dem Translationsstopcodon (TAG etc.) des Zelltodproteins liegt bedingt durch einen gentechnischen Eingriff die Polyadenylierungserkennungsstelle A-A-T-A-A-A gefolgt von einer downstream G/T-reichen Region (2. Signal zur Erkennung der Polyadenylierung) (s. Fig. K1) (z.B. die komplette Polyadenylierungstelle aus SV40) .

Der Offenbarungsgehalt von Dokument D1 [PCT) ist mangelhaft, da die noch nicht transgespleißte pre-mRNA des Toxins (diphtheria toxin subunit A; DT-A) bereits für ein vollständiges aktives Toxin ab Aminosäure 1 kodiert.

In Anbetracht des Dokuments D1 wird festgestellt. dass ein Bestandteil dieses Dokuments (09/941,492) nach dem Anmeldetag des Deutschen Patents (13.08.2001) am 29.08.2001 beim US-Patentamt eingereicht wurde. Außerdem wurde das Dokument D1 am 11.07.2002 international publiziert.

In Dokument 2 (PCT) ist der Offenbarungsgehalt in Bezug auf die spezifische Abtötung von Zielzellen mittels DT-A so mangelhaft wie in Dokument D1. Außerdem wird in D1 die Reparatur, aber nicht die spezifische Abtötung pathogener Zellen, für die Cystische Fibrose (Exon 10) mittels RNA-Transsplicing beschrieben. Für den allgemeinen Nachweis der RNA-Transspleißreaktion werden ein β-Gal-Konstrukt, ein LacZ-Konstrukt und ein ß HCG-Konstrukt als Targets verwendet. Die spezifische Abtötung von Zielzellen mittels RNA-Transspleißing war nicht das Ziel dieser experimentellen Ansätze.

In Dokument D3 (PCT) ist der Offenbarungsgehalt in Bezug auf die Verwendung der diphtheria toxin subunits ebenso mangelhaft wie in den Dokumenten D1 und D2. Die Funktionsweise in Bezug auf eine spezifische Abtötung von Zielzellen ist für den Fachmannn nicht erkennbar!

Im Dokument 4 (Puttaraju M. et al., Nature Biotechnology, Vol 17, March 1999) wird die cDNA des DT-A Toxins beschrieben. Die Funktionsweise dieses prätherapeutischen Moleküls (PTM) ist für den Fachmann hier ebenfalls nicht nachvollziehbar! Die ATG-DNA-Sequenz ist hier Bestandteil des PTMs

Anmerkung: In der hier vorgelegten Patentanmeldung (02746846.8-2403) ist die ATG-DNA-Sequenz nicht Bestandteil des PTMs!

Wie im Anwendungsfall 1 und 2 beschrieben wurde, können mit Hilfe extern gewollter RNA-Transspleißprozesse über eine künstlich geschaffenen RNA, die nur aus einem Exon und einem Outron besteht, monogenetisch bedingte Krankheiten und Krebs therapiert werden. Andererseits ist es aber auch möglich und sogar sehr wahrscheinlich, dass durch in der Zelle von selbst vorkommende, jedoch abberante Spleißprozesse Erkrankungen oder speziell auch Krebs ausgelöst werden; diese Prozesse können sowohl "gewöhnliche" Cis-Spleißprozesse als prinzipiell auch Trans-Spleißprozesse sein. Im dritten, letzten hier vorgestellten Anwendungsfall fungiert diese extern eingebrachte transspleißfähige RNA, bestehend wiederum aus einem Exon und einem Outron, nicht als ein therapeutisches Mittel sondern als ein diagnostisches Instrument, um derartige abberante zelluläre Spleißprozesse aufzudecken.

Es ist bekannt, dass durch abberante Cis-Spleißprozesse sehr viele Krankheiten ausgelöst werden, wie z.B. Cystische Fibröse, Ehelers Danlos Syndrom, Leukodystrophie, Haemophilie A und B, Sichelzellenkrankheit, Phenylketonurie, Leukodystrophie, Retinoblastomdefekte usw. Bei diesen Krankheiten liegt stets eine Mutation in einer authentischen 5' oder 3' Speißstelle vor. Dies führt dazu, dass die betreffende authentische Spleißstelle nicht mehr als (Cis) Spleißpartner benutzt werden kann, und dass andere authentische Spleißstellen oder sogenannte kryptische Spleißstellen auf dem selben pre-mRNA-Molekül statt dieser mutierten Spleißstelle als Spleißpartner reagieren. Durch diese abberanten Spleißprozesse kommt es dann zur Bildung von anderen mRNA-Molekülen, die oft defekte und nicht mehr funktionsfähige Proteine codieren, wodurch die entsprechende Krankheit ausgelöst wird.

Es gibt zahlreiche Fallbeispiele, in denen nach Mutation einer authentischen Spleißstelle eine Ersatzspleißstelle
auf dem selben RNA-Molekül für einen gewöhnlichen intramolekularen Cis-Spleißprozess nicht mehr zur Verfügung steht, in diesem Fall kann dann als Ersatzspleißstelle eine (kryptische) Spleißstelle auf einem anderen! RNA-Molekül dienen, sodass nach einem inter-molekularen Transspleiß eine völlig neuartige Hybrid-RNA entsteht. Ein konkreter Fall hierzu wäre z.B., wenn die upstream 5' Spleißstelle im ersten Intron einer pre-mRNA mutiert ist, und es für die downstream liegende, nicht mutierte 3' Spleißstelle keinen kryptischen 5' -Spleißstellen-Ersatz für einen Cis-Spleiß auf demselben RNA-Molekül gibt (s. Fig. N1, pre-mRNA A, obere Darstellung). Die zu dieser mutierten 5' Spleißstelle zugehörige downstream 3' Spleiß stelle im ersten Intron kann einen Spleißprozess in diesem Falle prinzipiell nur dann ausführen, wenn diese mit der 5' Spleißstelle auf einem anderen, zweiten pre-mRNA-Molekül über einen Transspleiß interagiert (s. Fig. N1, N3).

Ein umgekehrter Fall wäre die Mutation der 3' Spleißstelle im letzten Intron: Die upstream 5' Spleißstelle dieses letzten Introns kann dann praktisch nur noch mit der 3' Spleißstelle auf einem anderen RNA-Molekül als Partnerspleißstelle interagieren (Fig. N1, pre-mRNA B, untere Darstellung) .

Transspleißprozesse können aber auch ausgelöst werden, wenn keine Mutation in einer Cis-Spleißstelle vorliegt: Alle Exone enthalten neben den authentischen Cis-Spleißstellen sehr oft noch weitere Spleißstellen (kryptische Spleißstellen), die aber aufgrund ihrer Nucleotidsequenz oder aus sterischen Gründen etc. weniger "attraktiv" sind als die authentischen Cis-Spleißstellen und deshalb nicht im (Cis) Spleißprozess genutzt werden. Eine Besonderheit liegt nun aber vor, wenn etwa eine solche kryptische 5' Spleißstelle im letzten Exon oder aber eine solche kryptische 3 ' Spleißstelle im ersten Exon vorkommt. Diese kann aufgrund ihrer terminalen Lage praktisch nicht mit einer anderen Spleißstelle innerhalb des selben RNA-Moleküls über einen gewöhnlichen Cis-Spleiß interagieren. Solche kryptische Spleißstellen in terminalen Exons stellen also schon per se sehr potente Spleißstellen ausschließlich für einen Transspleiß dar. Insbesondere kryptische 5' Spleißstellen in C-terminalen Exonen und kryptische 3' Spleißstellen in N-terminalen Exonen werden mit relativ hoher Wahrscheinlichkeit über einen Transspleiß nteragieren, sofern die beiden zugehörigen pre-mRNA-Moleküle in eine bestimmte Entfernung zueienander kommen (s. Fig. N2).

Als weitere Fälle können zudem kryptische Spleißstellen in terminalen Exons mit zum Transspleiß aktivierten Cis-Spleißstellen aus terminalen Introns durch Transspleiß interagieren (Fig. N3). Die Aktivierung der ehemaligen Cis-Spleißstelle zu einer Trans-Spleißstelle kann auch dadurch erfolgen, dass die natürliche Cis-Spleißpartnerstelle dieser Spleißstelle durch eine Mutation verloren geht (Fig. N3) und keine weitere Ersatz-Cis-Spleißstelle als Spleißpartner zur Verfügung stand. Selbstverständlich ist auch der umgekehrte Fall möglich, indem z.B. eine kryptische 3' Spleißstelle in einem N-terminalen Exon mit einer zum Transspleiß aktivierten ehemaligen 5' Cis-Spleißstelle auf einem anderen RNA-Molekül durch Transspleiß interagiert. Insgesamt gibt es also recht viele Spleißstellen-Kombinationsmöglichkeiten, die Trans-Spleißprodukte entstehen lassen.

Berücksichtigt man, dass sehr viele RNA-Moleküle transspleißfähige Spleißstellen besitzen, z.B. aufgrund einer Mutation authentischer Spleißstellen oder direkt als kryptische Spleißstellen innerhalb der terminalen Exone, und dass inter-molekulare RNA-RNA-Verbindungen über ausgeprägte Antisensstrukturen im Zellkern etwa zur Stabilisierung von Transspleißprozessen kein seltenes Ereignis darstellen, so ist anzunehmen, dass verschiedene Transspleißprozesse in einem Säugerzellkern mit an Sicherheit grenzender Wahrscheinlichkeit vorkommen. Unter Umständen können diese abberanten Transspleißprozesse dann auch die Ursache einer Krankheit oder speziell von Krebs sein, wie dies durch den Patentanmelder bereits 1995 nachgewiesen und publiziert wurde. Dass abberante Spleißprozesse per" se Krankheiten auslösen können, ist bekannt; über hundert verschiedene Krankheiten werden durch abberante Cis-Spleißprozesses ausgelöst, wie eingangs beschrieben wurde.

Alle bislang beschriebenen, natürlich vorkommenden Transspleißprozesse der Säugerzelle wurden erst entschlüsselt, nachdem z.B. die mRNA/cDNA-Struktur eines vom Molekulargewicht her zunächst unerklärlichen Proteins des Immunoblots aufgeklärt wurde. Eine andere Möglichkeit zur Identifikation von Transspleißprodukten wäre die direkte Suche auf zellulärer RNA-Ebene: Auch hier finden sich in spezifischen RNA-Northern-Blots oft Größen einer spezifischen RNA-Spezies, die von der Größe her zunächst unerklärlich sind, und die eventuell auch ein Transspleißprodukt darstellen könnten. Doch auch hier fehlt es an einem einfachen Verfahren, welches transgespleißte RNAs erkennt. Eine sehr empfindliche und spezifische Methode, die es prinzipiell erlaubt, ein einziges transgespleißtes RNA-Molekül einer Zelle nachzuweisen, ist die cDNA-PCR. Die PCR-Amplifikation und anschließende Sequenzanalyse einer cDNA setzt aber voraus, dass mindestens zwei ca. 18 mer lange Bereiche dieser RNA bzw. cDNA bekannt sein müssen, die als spezifische PCR-Primer-Bindungsstellen dienen. Bei transgespleißten RNAs braucht man also zum Nachweis einen Primer, der auf dem ersten cDNA-Hybridanteil, abstammend von der ersten RNA, und einen zweiten Primer, der auf dem zweiten cDNA-Hybridanteil, abstammend von der zweiten RNA, bei der transgespleißten mRNA bindet. Vom Prinzip her kann jede RNA-Spezies mit einer anderen RNA-Spezies ein Transspleißprodukt bilden. Um nur alle möglichen Transpleißpartner zu nur einer pre-mRNA-Spezies der Zelle über cDNA-PCR-Reaktionen zu überprüfen, wären bei angenommen 25.000 verschiedenen pre-mRNA-Spezies mindestens 25.000 verschiedene PCR-Reaktionen mit 25.000 + 1 verschiedenen spezifischen Primern notwendig. Um sytematisch alle möglichen RNA-Spezies-RNA-Spezies-Spleiß-Kombinationen aufzuspüren, wären entsprechend mindestens 24.999 x 24.998 x ... x 3 x 2 x 1 PCR-Kombinationen notwendig, was als praktisch durchführbar ausgeschlossen ist.

Es ermangelt also derzeit, gemäß dem Stand der Technik, an einem einfachen und sicheren, routinemäßigen Detektionsverfahren für die Identifizierung von transgespleißter RNA.

Im folgenden dritten Anwendungsfall wird jene in die Zellen eingebrachte, transspleißfähige RNA bestehend aus einem Exon und einem Outron dazu benutzt, natürliche Transspleißprodukte der Zelle, die eventuell Krankheiten auslösen können, zu identifizieren. Der Nachweis dies er zellulären transgepleißten RNAs erfolgt hierbei über einen zweistufigen Mechanismus, wobei im jeder Teilstufe eine spezifische cDNA-PCR eingesetzt wird.

Der Lδsungsweg sieht dabei so aus, dass in der ersten Stufe zunächst einmal Spleißstellen auf den ca. 25.000 verschiedenen pre-mRNA-Spezies gefunden werden müssen, die prinzipiell intermolekulare Transspleißreaktionen durchführen können. Wie zuvor beschreiben, sind sehr gute Kandiaten für Transspleißreaktionen hierbei insbesondere Cis-Spleißstellen aus N- oder C-terminalen Introns, deren Cis-Spleißpartner innerhalb dieses Introns durch eine Spleißstellen-Mutation verloren ging, sowie kryptische Spleißstellen in N- oder C-terminalen Exons (vgl. Fig. N) .

Diese potenten Trans-Spleißstellen werden dadurch bestimmt, dass man diese in einer Transspleißreaktion reagieren lässt. Der Transspleißpartner ist hierbei jedoch nicht eine weitere, unbekannte zelluläre RNA, sondern jene künstlich in die Zelle hineingebrachte transspleißfähige RNA, die quasi als eine Transspleißspnde fungiert. Diese RNA-Transspleißsonde ist im Prinzip eine kurze RNA von 150-300 Nucleotiden Länge, welche nur eine einzige 5' oder eine einzige 3' Spleißstelle aufweist, sie besteht also aus einem Exon und einem distalen oder proximalen Outron (Fig. O1). Diese Sonden-RNA reagiert in ihrer einzigen Spleißstelle also mit potentiellen, zum Transspleiß befähigten Spleißstellen der zellulären pre-mRNA-Moleküle. Sonden-RNAs mit einer 5' Spleißstelle dienen zur Detektion transpleißfähiger 3' Spleißstellen auf zellulären pre-mRNAs, solche mit einer 3' Spleißstelle zur Detektion transspleißfähiger 5' Spleißstellen (s. Fig. O1, links u. rechts) . Die nach dem Transspleiß entstandene Hybrid-mRNA aus Sonden-RNA und zellulärer RNA kann sodann prinzipiell mit Hilfe spezieller Primer, die u.a. zur bekannten Sonden-RNA-Sequenz passen, in eine doppelsträngige (ds) cDNA überführt, PCR-amplifiziert und sequenziert werden (s. Fig. O1, unten).

Sind mit Hilfe dieser beiden RNA-Sonden etwa eine potente 5' Transspleißstelle auf einer natürlichen RNA-Spezies und eine potente 3' Transspleißstelle auf einer anderen RNA-Spezies gefunden worden, so muss dann in der Stufe 2 abschließend nur überprüft werden, ob diese beiden Transspleißstellen in vivo in der Zelle interagieren, d.h. ob diese beiden RNAs zusammen ein neues chimeres Hybrid-RNA-Molekül nach Transspleiß ausbilden. Der Nachweis dieser mRNA erfolgt ebenfalls nach cDNA-PCR-Amplifikation mit zwei Primern, die selektiv zu den zuvor sequenzierten, transgespleißten exonischen Teilen der beiden transspleißfähigen pre-mRNA-Moleküle passen (s. Fig. O2, Primer C und D).

Die zunächst in Stufe 1 zu analysierenden transgespleißten mRNAs, (Fig. O1) bestehen also zu einem Teil aus dem Exon der RNA-Sonde und zum anderen aus exonischen Anteilen einer unbekannten zellulären RNA. Die Sequenz der RNA-Sonde ist letztlich bekannt, entsprechend lässt sich hierzu analog einer der beiden Primer für die cDNA-PCR-Amplifikation des Transspleißproduktes auswählen. Ein Problem für die nachfolgende cDNA-PCR bereitet hingegen die Wahl des Primers, der zum RNA-Anteil passt, welcher innerhalb des Transspleißproduktes aus der zellulären pre-mRNA abstammt: Die Struktur dieser RNA im vorhandenen TransSpleißprodukt ist nicht bekannt, entsprechend lässt sich zunächst kein entsprechender spezifischer cDNA-PCR-Primer bestimmen.

Die Auswahl eines bestimmten Primers, passend zu einer sehr speziellen Sequenz einer zellulären pre-mRNA, ist zudem im Rahmen des hier beschriebenen Screening-Verfahrens nicht hilfreich: Schließlich sollen durch das cDNA-PCR-Verfahren alle Kombinationsmöglichekiten der Sonden-RNA zu mehr als 25.000 verschiedenen zellulären pre-mRNAs abgedeckt werden. Das im Nachfolgenden vorgestellte erfindungsgemässe Verfahren gestattet eine sichere Identifikation aller möglichen Transspleißpartner bei Verwendung einer bekannten Sonden-RNA-Sequenz, so dass letztlich aus allen transgespleißten Sonden-RNAs cDNA-PCR Produkte amplifiziert und anschließend sequenziert werden können. Die entsprechenden zweiten Primer zur PCR-Amplifikation nutzen hierbei die uniformen Enden aller zellulären mRNAs (Poly-A-Tail am 3' Ende) bzw. eine spezielle Oligo-C-Struktur an den vorderen 5' Enden aller ss-cDNAs, welche durch eine spezifische Transferase-Aktivität der reversen Transkriptase erzeugt wird, aus. Auf die einzelenen Schritte (vgl. auch Fig. Q und R) der cDNA-Synthese, der PCR-Amplifikation und Sequenzanalyse der Transspleißprodukte aus Sonden-RNA und zellulärer RNA wird in einem späteren Teil dieser Beschreibung nochmals konkret eingegangen.

Die wesentlichen Strukturelemente der Sonden-RNA sind hier, wie schon im Anwendungsfall 1 zur Reparatur-RNA beschrieben, ein Exon und daran angefügt ein distales oder proximales Outron. Das Exon selbst muss hier jedoch nicht eine Peptidfolge codieren, da diese transspleißfähige RNA nicht zu therapeutischen sondern zu diagnostischen Zwecken dient. Analog zum Anwendungsfall 2 zur unvollständigen Zelltod-RNA könnte die Codierung einer bestimmten Peptidsequenz im Exon jedoch einige diagnostische Vorteile aufweisen: Etwa im Falle einer Sonden-RNA mit einer 3 ' Spleißstelle könnte das distale Exon die durch ein Terminationssignal (UAG etc.) abgeschlossene cDNA-Folge eines über spezifische Verfahren (Immunofloureszens etc.) einfach nachzuweisenden Proteins enthalten, wobei das Translationstartcodon AUG, codierend für Methionin 1, jedoch, wie zuvor beschrieben, wiederum fehlt. Erst wenn dieses AUG über einen Transspleiß von einer anderen, nämlich zellulären RNA enthalten wird, kann ein entsprechendes analytisch nachweisbares Protein entstehen; bei In-Situ-Nachweisen könnten dann diese Zellen z.B. eine entsprechende Fluoreszensfärbung etc. aufweisen.

Die eingebrachte transspleißfähige Sonden-RNA, bzw. die jene RNA codierende DNA enthält im distalen Bereich ferner wiederum eine Erkennungsregion zur Polyadenylierung der RNA; d.h. die codierende DNA beinhaltet die Folge AATAAA upstream und eine GT-reiche Sequenz downstream (z.B. aus SV 40) der RNA-Polyadenylierungsstelle . Das Exon sollte zudem durch gentechnologische Methoden wiederum z.B. A/G-reiche oder andere spezielle Regionen enthalten, die über Bindung von S/R-Proteinen etc. als Spleißenhancer (ESE) dienen (vgl. Fig. J). Sofern das Exon eine Peptidfolge codieren soll (s.o.), ist darauf zu achten, dass durch die geschaffenen z.B. A/G-reichen Sequenzen keine anderen Aminosäuren codiert werden; im Falle, dass das Exon kein bestimmtes Peptid codieren soll, sind an die Variationsmöglichkeiten der Nucleotide im Exon keine Grenzen gesetzt.

Im Falle einer Sonden-RNA mit einer 3' Spleißstelle enthält das proximale Outron wiederum eine hochaktive 3' Spleißstelle, die eine Folge von 15-18 U/C-Basen unmittelbar upstream des Dinucleotides AG umfasst, sowie upstream des Polypyrimidinbasenstretch.es eine starke Branch-A-Region, welche die Nucleotidfolge U- (A/G) -C-U-(A/G) -A enthält (vgl. auch Fig. J).

Im Falle einer Sonden-RNA mit einer 5' Spleißstelle enthält das distale Outron den intronischen Anteil einer hochaktiven 5' Spleißstelle mit der bevorzugten Nucleotidfolge G-U-A-G-A-A und der exonische Anteil der 5' Spleißstelle beinhaltet die Basenfolge (A/G) -A-G (vgl. Fig. I). Als eine bereits sehr gute, und durch gentechnologische Methoden in der Sonden-RNA verwendbare natürliche 5' Spleißstelle ist die Umgebung um eine kryptische 5' Spleißstelle mit der 9-mer Basenfolge AAG/GUAGAA im zweiten Exon des SV40 T-Antigens anzusehen, die upstream zur Spleißstelle u.a. einen 15 mer großen A/G-Bereich als Exonischen-Spleiß-Enhancer enthält.

Damit eine hohe zelluläre Konzentration der Sonden-pre-mRNA und damit eine hohe Zahl von Transspleißreaktionen zu zellulären RNAs erreicht wird, soll die die Sonden-RNA codierende DNA einen starken Promotor (Fig. K1), wie z.B. aus SV40, tragen. Weiterhin soll diese codierende DNA einen starken Replikations-Origin (z.B. auch aus SV40) aufweisen, damit sich die DNA in den Zellen selbständig replizieren kann. Das Einbringen dieser Sonden-RNA codierenden DNA in die auf Transspleißprodukte zu analysierenden, lebenden Zellen erfolgt nach Einbau in normale Plasmide - Adenoviren etc. sind nicht notwendig - mit Hilfe einer der üblichen Transfektionsmethoden. Pro Transfektionsansatz zur Analyse auf transspleißfähige zelluläre RNAs wird jeweils nur eine Sonden-RNA mit einer 5' Spleißstelle oder eine solche mit einer 3' Spleißstelle verwendet.

Wie im Anwendungsfall 1 zur Reparatur-RNA explizit beschrieben wurde, kann die Transspleißeffizienz zwischen einer eingebrachten RNA (hier: Sonden-RNA) und einer zellulären pre-mRNA prinzipiell noch weiter erhöht werden, wenn eine möglichst stabile Antisens-RNA-RNA-Brückenbindung erfolgt. Im Unterschied zu einer spezifischen Reparatur-RNA kann hier jedoch keine willkürliche, zielspezifische Antisensregion auf der Sonden-RNA erzeugt werden, da die Struktur des etwaigen Transspleißpartners (Target-RNA = zelluläre transspleißfähige pre-mRNA) nicht bekannt ist. Eine sehr spezielle Struktur wäre zudem auch wenig sinnvoll, da eine etwaige AntisensStruktur auf der Sonden-RNA so gewählt sein sollte, dass diese zu möglichst vielen bzw. allen zellulären pre-mRNA-Molekülen, die potentielle Transspleißpartner darstellen, in Antisens passt. Bei der Wahl dieser Nucleotid-Folge auf der Sonden-RNA, die zu möglichst vielen zellulären pre-mRNA-Molekülen passt, gibt es mehrere Möglichkeiten:
a) Antisens-Region zur zellulären RNA über eine Oligo-U-Region in der Sonden-RNA: Eine Lösung ist eine Sonden-RNA mit einer Folge von ca. 12-18 Uracil-Nucleotiden (U) im Outron-Bereich der Sonden-RNA (s. Fig. P1, bzw. Fig. Q1 oder R1). Diese U-Basen-Folge paart in Antisens intermolekular zur Poly-A-Kette der zellulären pre-mRNAs, also auch zu der gewünschten Transspleiß-pre-mRNA. Außerdem werden Purinreiche (A/G) Regionen, die häufig auf zelluären pre-mRNA-Molekülen vorkommen, durch eine U-Basen-Folge in Antisens gebunden (auf RNA-Ebene gilt die Paarungsmöglichkeit U-A, sowie U-G) . Durch diese Antisens-Regionen kann eine stabile Kopplung zwischen Sonden-pre-mRNA und zellulärer Target-pre-mRNA erreicht werden, was die Transspleißeffizienz merklich steigert. Ein Nachteil dieses Verfahrens ist aber, dass die 12-18 mer lï-Region auf der Sonden-RNA auch intra-molekular zur Poly-A-Region der Sonden-RNA selbst paart, wodurch die U-Antisensregion für diese Sonden-RNA für eine inter-molekulare Paarung zur Target-RNA blockiert würde. Bei genügend hoher Substratkonzentration der Sonden-RNA in der Zelle werden aber genug Sonden-RNA-Moleküle mit ihrer Poly-U-Region auch intermolekular zu den zellulären Poly-A-Anhängen der zellulären pre-mRNAs paaren. Zur Lösung dieses Problems soll ein äquimolarer Mix aus Sonden-RNAs mit dieser bzw. ohne dieser entsprechenden Poly-U-Region verwendet werden.

Antisens-Region zur zellulären RNA über eine Oligo-G-Region in der Sonden-RNA: Eine Möglichkeit ist die Einfügung eines Stretches von 12-15 Guanosin-Nucleotiden (G) in Sonden-RNAs, die insbesondere eine potente 5 ' -Spleißstelle (5' ss-Sonden-RNA) enthalten. Diese paaren mehr oder weniger intermolekular stringent zu den Poly- (U/C) -Regionen etwa in den 3' Spleißstellen von zellulären Target-pre-mRNAs (es gilt wiederum die Paarung auf RNA-Ebene: G-C sowie G-U). Der Nachteil dieses Verfahrens ist, dass hierdurch auch eventuell die Poly- (U/C) -Region der potenten 3' Transspleißstelle auf der zellulären pre-mRNA durch eine Antisensbindung blockiert wird, so dass kein Transspleiß zur Sonden-RNA stattfindet, wodurch eine etwaige potente Transspleißstelle auf einer zellulären pre-mRNA unentdeckt bleibt.

Keine spezifische Antisensregion auf der Sonden-RNA zur intermolekularen Paarung zur Target-pre-mRNA: Wie dargelegt, erhöhen spezifische inter-molekulare Antisens-Strukturen zwischen zwei RNA-Transspleißpartnern die Effizienz der Transspleißreaktion erheblich. Eine solche Kopplung über Antisensstrukturen der beiden pre-mRNA-Moleküle ist aber per se nicht absolut für eine Assoziation zwischen beiden, transzuspleißenden pre-mRNA-Molekülen notwendig. Wie eingangs bei der Vorstellung des Anwendungsfalles 1 zur Reparatur-RNA beschrieben, erfolgt die Assoziation der Spleißstellen im Spleißprozess auch über eine Protein-Protein-Interaktion/Assoziation von Spleiß-(hilfs) proteinen, die zuvor selektiv an die 5' Spleißstelle sowie selektiv an die 3' Spleißstelle der pre-mRNA gebunden sind.

So erfolgt im frühen E-Komplex der Spleißreaktion eine Assoziation einer pre-mRNA in ihrer 5' Spleißstelle zu der 3' Spleißstelle der selben RNA (= Cis-Spleißreaktion) oder zu einer andern RNA (= Transspleißraktion) über eine Assoziation zwischen einerseits dem U^nRNP-Proteinkomplex (gebunden zuvor an der 5' Spleißstelle) und andererseits dem U2AF-Proteinkomplex (gebunden zuvor am Poly-U/C-Stretch der 3' Spleißstelle) ggf. unter Vermittlung weiterer SR-Proteine (z.B. SC 35, SF2 etc.) (s. Fig. P2, sowie auch Fig. Al). Auch in späteren Stufen des Spleißprozesses gibt es zahlreiche Assoziationen zwischen verschiedenen Spleiß (hilfs) proteinen, die zuvor an den beiden Spleißstellen der pre-mRNA gebunden sind (s. Fig. A2).

Eine Sonden-RNA, die etwa nur eine potente 3' Spleißstelle enthält (3 ' ss-Sonden-RNA), wird in der Zelle in ihrem Poly-U/C-Anteil der 3' Spleißstelle mit U2AF-Proteinmolekülen verbunden. Die mit dem U2AF-Proteinkomplex versehene Sonden-RNA diffundiert dann solange frei im Zellkern, bis das U2AF-Protein etwa auf einen U^nRNP-SCSS-Proteinkomplex trifft, der zuvor an einer transspleißfähigen 5' Spleißstelle einer zellulären pre-mRNA gebunden war (s. Fig. P2). Danach wird eine Protein-Protein-Bindung/Assoziation eingegangen, sodass die Sonden-RNA nun in einer relativ festen Bindung zur eventuell transspleißfähigen Target-pre-mRNA steht. Der umgekehrte Fall gilt sinngemäß natürlich auch für eine Sonden-RNA mit einer 5' Spleißstelle, an die von der Zelle gebildetes U₁SnR P gebunden wird, wobei dieser Komplex dann solange durch den Zellkern diffundiert, bis er auf einen U2AF-Komplex - gebunden an eine transspleißfähige 3' Spleißstelle einer zellulären Target-pre-mRNA - stößt, um mit dieser RNA über jenen U2AF-Komplex zu assoziieren.

Im Unterschied zu einer Gentherapie über einen RNA-Transspleiß etwa (Anwendungsfall 1) ist eine hohe spezifische Rate an Transspleißreaktionen bedingt durch eine stabile Antisens-RNA-RNA-Bindung in diesem Nachweisverfahren im übrigen auch nicht unbedingt notwendig: Hier reicht prinzipiell eine einzige Transspleißreaktion zwischen einem Sonden-RNA-Molekül und einer transspleißfähigen zellulären Target-pre-mRNA, die danach ein einziges transgespleißtes mRNA-Molekül erzeugt. Durch die nachfolgende cDNA-PCR wird dieses Molekül dann selektiv millionenfach vervielfältigt und kann so leicht nachgewiesen und sequenziert werden.

Die transspleißfähige Sonden-RNA zur Detektion transspleißfähiger zellulärer RNAs ist also einfacher aufgebaut als transspleißfähige therapeutische RNAs, wie etwa eine Reparatur-RNA zur mikrochirurgischen Beseitigung von Gendefekten oder gar eine unvollständige Zelltod-RNA zum Abtöten von Tumorzellen, da hier eine selektive Anbindung an die Target-RNA über entsprechende RNA-Antisensstrukturen entfällt. Im Unterschied zu diesen therapeutischen Anwendungen sollte - wegen des nachfolgenden Nachweis-Verfahrens der transgespleißten RNAs - die diagnostische Sonden-RNA jedoch ein weiteres Strukturelement aufweisen: Eine Restriktionsenzym-Erkennungstelle für ein sehr selten schneidendes Restriktionsenzym im Outronanteil der Sonden-RNA. Die Begründung liegt darin, dass, wie nachfolgend beschrieben, in der cDNA-PCR zur Amplifikation der Transspleißprodukte auch die ungespleißte Sonden-RNA mit amplifiziert wird. Da die ungespleißte Sonden-RNA aber in sehr viel höherer Konzentration als die Transspleißprodukte aus Sonden-RNA und zellulärer Target-RNA in der Zelle vorliegt, würde das PCR-Signal aus der ungespleißten Sonden-RNA das PCR-Signal aus den Transspleißprodukten kompetetiv auslöschen, wodurch jene Transspleiß-RNAs nicht mehr in der cDNA-PCR nachzuweisen wären. Nach Bildung einer doppelsträngigen (ds) cDNA muss also vor Start der PCR-Reaktion die ds-cDNA aus der ungespleißten Sonden-RNA zum Beispiel über einen Restriktionsenzymverdau selektiv abgebaut werden, ohne dass aber die ds-cDNA der Transspleißprodukte von diesem Abbau betroffen ist. Dies ist möglich, indem die Restriktionsstelle zum enzymatischen Abbau der ds-cDNA in den Outron-Teil der Sonden-RNA gelegt wird; die ds-cDNA-Transspleißprodukte aus der Sonden-RNA haben diesen Outron-Anteil der Sonden-RNA eben nicht mehr, und werden deshalb durch das Restriktionsenzym nicht abgebaut.

Bei der Wahl der Restriktionsschnittstelle muss zudem eine sog. seltene Restriktionsschnittstelle genommen werden (Rare-Cutter) . Der Grund liegt darin, dass der Anteil aus der unbekannten zellulären Target-RNA im Transspleißprodukt diese Restriktionserkennungstelle nicht haben sollte, denn ansonsten würde das Transspleißprodukt ebenfalls enzymatisch abgebaut und könnte in der nachfolgenden PCR dann nicht nachgewiesen werden. Als sog. seltene Restriktions-schnittsteilen gelten Basenfolgen von 8 mer (oder länger) . Bei einem 8 mer ist die Wahrscheinlichkeit (W), dass diese Sequenz an einer bestimmten Stelle in einer DNA auftritt = W = 1 zu 4⁸ = 1 zu 65.536. Unterstellt man eine Nucleotidfolge von angenommen 400 Basen, abstammend aus der unbekannten zellulären Target-RNA innerhalb des Transspleißproduktes, so ist die Wahrscheinlichkeit, dass eine solche 8-mer Erkennungstelle innerhalb der 400 Basen hier vorliegt = W = 1 zu 65.536 / 400 = 1 zu 164, was zu vernachlässigen ist; bei einer 10 mer Erkennungssequenz ist W sogar nur 1 zu 2624.

Nachdem die eingebrachte codierende DNA u.a. aufgrund eines starken Promoters hunderttausende von Sonden-RNA-Molekülen pro Zelle gebildet hat, paaren im Falle einer 5'ss-RNA-Sonde einige davon sodann zu einer potenten 3' Transspleißstelle auf der unbekannten zellulären Target-pre-mRNA (s. Fig. Q1). Im Falle einer 3 ' ss-Sonden-RNA kann sich eine entsprechende Paarung zu einer potenten 5' Transspleißstelle auf der unbekannten zellulären pre-mRNA ausbilden (s. Fig. R1). Die Verbindung der beiden RNA-Moleküle kann dabei durch Antisensbereiche zwischen der Poly-U-Kette im Outron-Bereich der Sonden-RNA und dem Poly-A-Tail der Transspleißfähigen pre-mRNA unterstützt werden (s. Fig. P1 bzw. Q1 sowie R1) ; eine solche Antisens-Nucleotid-Struktur ist jedoch für einen Transspleißprozess nicht absolut notwendig (s.v.).

Im Falle der Verwendung einer 5' ss-Sonden-RNA enthält das entstandene Transspleißprodukt im N-terminalen Anteil das Exon der Sonden-RNA mit einer von der Zelle gebildeten cap-Sequenz und im größeren C-terminalen Bereich exonische Anteile der unbekannten Transspleiß-RNA. Im Falle, dass die Transspleißstelle der zellulären RNA etwa eine kryptische 3' Spleißstelle im letzten! Exon dieser RNA ist, enthält das Transspleißprodukt die RNA-Folge downstream dieser kryptischen Spleißstelle, die einen Poly-A-Tail trägt. Im Falle dass eine kryptische oder gar authentische 3' Spleißstelle in einem vorderen! Exon der zellulären RNA benutzt wird, enthält das Transpleißprodukt die gesamte mRNA-Folge jener RNA downstream dieser 3' Spleißstelle ebenfalls unter Einschluß eines terminalen Poly-A-Tails (s. Fig. Q2). Im Falle der Verwendung einer 3' ss-Sonden-RNA enthält das entstandene Transspleißprodukt im größeren N-terminalen Bereich entsprechend die exonischen Anteile upstream der 5' Transspleißstelle aus der unbekannten transgespleißten zellulären RNA mit einer cap-Sequenz und im C-terminalen Anteil das Exon aus der Sonden-RNA mit einem Poly-A-Tail (s. Abb R2).

Wie eingangs bereits erwähnt wurde, kann der Nachweis der transgespleißten RNAs bestehend aus einem zellulärem RNA-Anteil und dem Exonanteil der Sonden-RNA aus Gründen der Selektivität und der Empfindlichkeit praktisch nur über eine cDNA-PCR erfolgen. Der erste der beiden PCR-Primer ist einfach zu bestimmen, er ist analog einer Nucleotid-Folge im Exon der Sonden-RNA. Damit alle möglichen zellulären RNAs in dieser cDNA-PCR erfasst werden, muss der zweite PCR-Primer, der hier bindet, hierfür universell geschaffen sein. Der entsprechende zweite Primer zur PCR-Amplifikation nutzt hierbei die uniformen Enden aller zellulären mRNAs (Poly-A-Tail am 3' Ende) bzw. eine spezielle Oligo-C-Struktur an den vorderen 5' Enden aller ss-cDNAs, welche durch eine spezifische Transferase-Aktivität der reversen Transskriptase erzeugt wird, aus. Im ersteren Falle wird prinzipiell ein Primer gewählt, der eine Folge von ca. 15-18 Thymidinbasen (T) enthält, welche zu den Poly-A-Enden aller mRNAs paaren; auf diese Oligo-T-Folge schließt sich in 5' Richtung eine willkürliche Folge von 18 oder mehr Nucleotiden an. Im zweiten Fall wird ein Primer verwendet, der einige Guanosinbasen (G) enthält, auf diese Oligo-G-Folge schließt sich in 5' Richtung eine andere willkürliche Folge von 18 oder mehr Nucleotiden an.

In den nachfolgenden Abschnitten ist ein entsprechendes, getestetes Verfahren beschrieben, welches eine sichere Identifikation aller möglichen Transspleißpartner bei Verwendung einer bekannten Sonden-RNA-Sequenz gestattet, so dass letztlich aus allen transgespleißten Sonden-RNAs cDNA-PCR Produkte amplifiziert und anschließend sequenziert werden können.

Die DNA, welche die Sonden-RNAs codiert, wird nach Einbau in geeignete Plasmide oder durch andere geeignete Verfahren in die zu untersuchenden Säugerzellen (z.B. Tumorzellen mit unbekannter Ursache der Tumorent icklung etc . ) eingebracht .

Es werden hierbei prinzipiell zwei DNA-Transfektionsansätze durchgeführt. Ansatz 1 besteht aus einer DNA, welche Sonden-RNAs mit einer 5'-Transspleißstelle codieren; hierbei wird ein äquimolarer Transfektionsansatz an entsprechenden DNAs, welche eine Nucleotidfolge T₁₅ bis T₁₈ aufweisen (DNA-Typ A) bzw. nicht aufweisen (DNA-Typ B) eingesetzt. Ansatz 2 besteht aus einer DNA, welche Sonden-RNAs mit einer 3' -Transspleißstelle codieren; hierbei wird ebenfalls ein äquimolarer Transfektionsansatz an entsprechenden DNAs, welche eine Nucleotidfolge T₁₅ bis T₁₈ aufweisen (DNA-Typ A) bzw. nicht aufweisen (DNA-Typ B) eingesetzt. 24 Stunden bis mehrere Tage nach der DNA-Transfektion wird die Gesamt-RNA der Zellen isoliert, hitzedenaturiert, und dann zunächst in eine ss-cDNA überführt .

Die Synthese des ersten cDNA-Stranges (ss cDNA) aus der (transgespleißten) mRNA erfolgt stets mittels einer Reversen Transskription mit einer effektiven, RNAse-Aktivität-freien, reversen Transkriptase sowie mit einem Oligo-Startpimer T₁₈, der am Poly-A-Tail der transgespleißten mRNA und aller übrigen zellulären RNA-Moleküle mit Poly-A-Ende bindet (s. Fig. Q3 , Fig. R3).

Im Falle, dass eine RNA-Sonde mit einer 5' Spleißstelle im analytischen Ansatz verwendet wurde, sollte der Oligo-T-Startprimer T₁₅ an seinem vorderen 5' Ende noch eine Folge von 18 oder mehr anderen, abwechselnden Nucleotiden aufweisen, die als spätere Erkennungssequenz für den zweiten Primer in der PCR dienen (Gesamt-Primerlänge also 33 oder mehr Nucleotide) (s. Fig. Q3 , Beispielfall: 5' -AACCGGCCAACCGGCCAA-Tₗₛ-3'). Die Folge dieser 18 oder mehr Nucleotide ist anhand entsprechender Bioinformatikprogramme so zu wählen, dass kein Selbstannealing etc. auftritt. Unter Umständen sind in jenen 18 mer Sequenzen enthaltene Restriktionsschnittstellen (z.B. Eco R1, Bam HI etc.) für weitere Arbeiten (z.B. zur Klonierung) hifreich; ebenso kann eine längere Nucleotidfolge als 18 mer sinnvoll sein (z.B. 36-40 mer), um eine sog. Nested-PCR (d.h. in einer 2-phasigen PCR binden hier zwei unterschiedliche 18-20 mer Primer) zur besseren Selektion zu ermöglichen (s. sp . ) .-,

Die Synthese des zweiten Stranges der cDNA (ds-cDNA) aus der transgespleißten mRNA ist abhängig davon, welche Art von RNA-Sonde (entweder mit einer 5' oder 3' Spleißstelle) verwendet wurde :
a) Im Falle einer unbekannten transgespleißten RNA, welche den Exonanteil einer RNA-Sonde mit einer 5' Spleißstelle enthält, startet die Synthese des zweiten cDNA-Stranges in der ursprünglichen Region des Exons der Sonden RNA. Die Synthese beginnt also einfach mit einem Primer, der einer 18mer Basenfolge des Exons der Sonden-RNA entspricht (Fig. Q4, Beispielfall: 5'-AGAAGAACGGAAGAACAA-3'). Die Synthese dieses 2. Stranges aus der ss-cDNA erfolgt hierbei z.B. durch eine einzyklische PCR-Reaktion oder andere Verfahren (s. Fig. Q4).

Im Falle einer unbekannten transgespleißten RNA, welche den Exonanteil einer RNA-Sonde mit einer 3' Spleißstelle enthält, ist die Synthese des zweiten cDNA-Stranges hingegen deutlich komplizierter, da der zur Synthese erforderliche Primer quasi im Exonteil aus der unbekannten Transspleiß-RNA binden muss. Hierzu ist eine spezifische Primersequenz jedoch nicht bestimmbar; zudem sollte ein Verfahren mit einem Primer gewählt werden, welches zu allen möglichen ss-cDNA-Transspleißprodukten passt (s.o.).

Es kann hier z.B. der Umstand genutzt werden, dass die reverse Transkriptase nach Erreichung der cap-Stelle auf der (transgespleißten) mRNA an die einsträngige cDNA in einer terminalen Transferasefunktion noch bevorzugt mehrere Cytosine zu einer C-Kette anhängt (s. Fig. R3). Diese Reaktion kann zudem durch einen Überschuss an Cytosinen im Substrat im Vergleich zu den anderen drei Substrat-Nucleotiden noch intensiviert werden. Dieser N-terminale Cytosinstretch der ss cDNA dient dann als Bindungsstelle für einen speziellen Primer (cap-Primer), der in seinem downstream-B'ereich eine Sequenz von ca. 6-8 Guanosin (G) enthält, die zu den Cytosinen paaren (s. Fig. R4a). Die Cap-Primerzugäbe erfolgt ca. 30-45 min. nach Beginn der ss-cDNA-Synthese bei einer abgesenkten Temperatur auf ca. 30°C. Dieser spezielle Primer (s. Beispielfall : 5 ' -GGTTGGAAGGTTGGAAGGGGGG-3') fungiert dann in seiner 18 mer (oder längeren) upstream-Sequenz oberhalb der G-Nucleotide als Vorlage (Template) zur weiteren Auffüllung mit entsprechenden Nucleotiden, die zu dieser Primersequenz paaren (s. Fig. R4a, b). Diese Auffüllung wird hierbei durch die reverse Transkriptase oder ein andere geeignete, zugesetzte DNA-Polymerase (bei ebenfalls ca. 30-32°C) vorgenommen. Nach einer Hitzdenaturierung zur Ablösung dieses Primers von des ss-cDNA erfolgt dann die eigentliche 2. -Strang-Synthese mittels einer einzyklischen PCR unter Zuhilfenahme eines Primers, der wie der obige cap-Primer aufgebaut ist, jedoch ggf. den distalen G-Stretch nicht enthält (s. Fig. R4c, Beispielfall: Primer 5' -GGTTGGAAGGTTGGAAG-3'). Es kann auch hier sinnvoll sein, den Bereich oberhalb der Oligo-G-Sequenz nicht nur 18-20 mer (s. Fig. R3) sondern z.B. 36-40 mer lang zu machen, um eine sog. Nested-PCR zur besseren Selektion zu ermöglichen (s.o., s.sp.). Zur Klonierung etc. können auch hier wiederum spezifische Restriktionsschnittstellen (Eco R1, Bam HI etc.) innerhalb dieser Primer-Sequenz hilfreich sein.

Aus der gebildeten ds-cDNA könnte dann sogleich eine PCR-Amplifikation erfolgen. Wie eingangs beschrieben wurde, enthält der analytische Ansatz aus dem Zellextrakt neben der ds-cDNA aus den verschiedenen RNA-Transpleißprodukten aber auch noch in sehr viel höherer Konzentration ds-cDNA aus der noch ungespleißten Sonden-RNA. Letztere ds-cDNA-Fraktion würde also in einer nachfolgenden PCR bevorzugt amplifiziert werden, wodurch die ds-cDNAs aus den RNA-Transspleiß-Produkten keine gelanalytisch nachweisbaren PCR-Produkte erbringen. Die ds-cDNA aus der ungespleißten Sonden-RNA muß also vor der PCR-Amplifikation spezifisch, etwa über einen Restriktionsverdau, beseitigt (gespalten) werden. Im konkreten Beispielfall wird der Analyseansatz unter geeigneten Bedingungen mit dem selten spaltenden Restriktionsenzym Swa I versetzt, welches die ds-cDNA aus der ungespleißten Sonden-RNA in dessen Outron-Anteil in der 8-mer Erkennungsfolge ATTT/AAAT spezifisch spaltet (s. Fig. Q, Stufen 5-6 untere Darstellung), so dass eine nachfolgende PCR keine Amplifikation mehr liefert (s. Fig. Q, Stufe 7 unten) . Die ds-cDNA aus der transgespleißten RNA, welche sehr wahrscheinlich diese seltene Restriktionsschnittsteile nicht enthält (s. Struktur dieser: "kein ATTTAAAT", s. z.B. Fig. Q Stufe 5, obere Darstellung) wird jedoch durch die enzymatische Behandlung sehr wahrscheinlich nicht gespalten und bleibt intakt (s. Abb Q, Stufe 6 oben) und kann anschließend mit zwei spezifischen Primern PCR-amplifiziert und nachgewiesen werden (s. Fig. Q, Stufe 7 oben) .

Alternativ zu der obigen Methode ist es auch möglich, die ds-cDNA zunächst begrenzt nach dem untenstehenden Protokoll zu amplifizieren (ca. 5-15 PCR-Cyklen), sodann einen vollständigen Verdau z.B. mit Swa I durchzuführen, und danach eine weitere komplette PCR (30-35 PCR-Cyclen) mit den gleichen oder (besser) anderen geeigneten Primern zu vollziehen.

Nach abgeschlossener Inkubation mit dem Restriktionsenzym erfolgt dann unter geeigneten Bedingung eine polyzyklische (ca. 35 fache) PCR.

Im Falle der Ansätze mit einer Sonden-RNA mit einer 5' Transspleißstelle sind die beiden verwendeten Primer hierzu zum einen: 1) Ein erster Primer, der im Exonteil aus der Sonden-RNA bindet, und der identisch ist mit dem Primer zur Synthese des zweiten Stranges der ds-cDNA (s. willkürl. Beispielfall: 5' -AGAAGAACGGAAGAACAA-3', s. Fig. Q4 , Q7) oder aber ein Primer, der downstream jenes ds-cDNA-Primers zum Sonden-RNA-Exon bindet. Sofern eine sog. Nested PCR durchgeführt werden soll (nicht aufgeführt in Fig. Q), muss der PCR-Primer der zweiten PCR entsprechend downstream des Primers der ersten PCR im Exon der Sonden-RNA binden. 2) Der zweite Primer ist analog der terminalen (5') Folge der 18-21 N-Nucleotide des Primers, welcher den distalen Oligo-T-Anteil enthält, und welcher zur ss-cDNA-Synthese eingesetzt wurde (s. Fig. Q7, Beispielfall: 5' -AACCTTCCAACCGGCCAA-3'). Sofern bei der ss-cDNA-Synthese eine ca. 40-mer-Folge anstelle einer 18-21-mer-Folge upstream des distalen Oligo-T-Anteiles eingesetzt wurde, kann eine Nested PCR durchgeführt werden, wobei der 18-21-mer Primer der ersten PCR der 5' -terminalen Folge und der 18-21-mer-Primer der zweiten PCR der 3' -terminalen Folge jener oben genannten 40-mer-Folge entspricht.

Im Falle der Ansätze mit einer Sonden-RNA mit einer 3' Transspleißstelle sind die verwendeten Primer hierzu zum einen: 1) Ein erster Primer, welcher zur 2. -Strang-Synthese der ds-cDNA eingesetzt wurde (verkürzter Cap-Primer) (s. Fig. R7, Beispielfall: 5'-GGTTGGAAGGTTGGAAG-3'). Sofern ein Cap-Primer mit einer entsprechenden überhängenden Folge von 36-40 Nucleotiden eingesetzt wurde, kann hier anschließend eine sog. Nested PCR durchgeführt, wobei der 18-21 mer Primer der ersten Nested PCR zum 5' -terminalen Anteil der 36-40 mer Folge und der 18-21 mer Primer der zweiten Nested PCR zum 3' -terminalen Anteil dieser Folge von 36-40 Nucleotiden paart. 2) Der zweite PCR-Primer ist analog einer bekannten Sequenz von 18-24 mer im exonischen Anteil der Sonden-RNA, bzw. der diese RNA codierenden DNA (s. Fig. R7, Beispielfall: 5' -CTTGTTCTTCCGTTCTTCT-3'). Im Falle einer durchzuführenden Nested PCR ist der Gegenprimer dieser zweiten Nested PCR dann ein 18-21 mer Primer, der ebenfalls analog zu einer entsprechenden exonischen Sequenz der Sonden-RNA ist, der jedoch downstream des Primers der ersten Nested PCR zum Exon paart.

Die aus der cDNA-PCR entstandenen unterschiedlichen PCR-DNA-Produkte, die aus entsprechenden, verschiedenen zur RNA-Sonde hin transgespleißten zellulären RNAs abstammen, werden anschließend über geeignete Verfahren zunächst selektioniert (kloniert) . Die Selektionierung der unterschiedlichen PCR-Produkte kann im einfachsten Fall durch das Herausschneiden der einzelnen PCR-DNA-Fragmente aus dem analytischen Gel geschehen; günstiger ist aber eine Klonierung aller entstandenen PCR-Produkte in geeignete Plasmide und eine nachfolgende Sequenzierung der aus der PCR hineinklonierten PCR-DNA aus dem Transspleißprodukt Die klonierten bzw. selektionierten cDNA-PCR-Produkte werden dann mit einem der beiden Primer aus der obigen PCR nach standardisierten Verfahren sequenziert (s. Fig. Q8, Fig. R8).

Das sequenzierte PCR-Produkt enthält dann neben einer bekannten Sequenzfolge aus dem exonischen Anteil der Sonden-RNA noch den transgespleißten exonischen Teil aus der unbekannten zellulären Target-RNA.

Im Falle, dass eine 3' Spleißstelle auf einer zellulären Target-RNA mit einer 5' Spleißstelle der Sonden-RNA interagierte, liegt der unbekannte Anteil aus der Target-RNA im hinteren sequenzierten Abschnitt des PCR-Produktes und beginnt mit dem Exon-Anfang der 3' Spleißstelle (unbekannte Sequenz) und endet nach 10-1000 Nucleotiden (Fig. Q8) mit der Folge A_{1S} und der reversen Folge des mind. 18 mer langen Anhanges zum T₁₅-Primer bei der Erststrang-cDNA-Synthese (Fig. Q5, Q6). Im Falle, dass eine 5' Spleißstelle auf einer zellulären Target-RNA mit einer 3' Spleißstelle einer Sonden-RNA interagierte, liegt der unbekannte Anteil aus der Target-RNA im vorderen sequenzierten Abschnitt des PCR-Produktes und beginnt nach der Sequenz des terminalen cap-Primers. Nach 10-1000 Nucleotiden aus der unbekannten zellulären RNA folgt dann die 3-Basenfolge aus dem Exonanteil der 5' Spleißstelle dieser Target-RNA (z.B. die Folge GAG, s. Fig. R), gefolgt von der exonischen Sequenz der Sonden-RNA bis zur Bindungstelle des Primers aus der vorherigen cDNA-PCR.

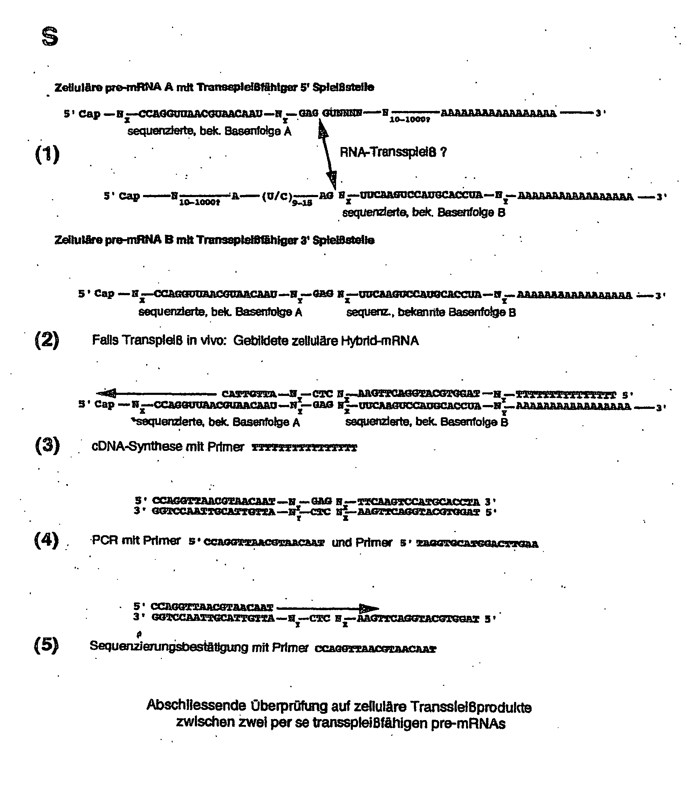

Nach Sequenzermittlung der exonischen Anteile der zur RNA-Sonde hin transgespleißten zellulären RNAs (vgl. Fig. O1, unterer Teil) ist der erste, aufwändigere Teil des Nachweises zellulärer natürlicher RNA-Transspleißprodukte abgeschlossen. Es muss dann im zweiten Teil abschließend überprüft werden, ob die entsprechenden per se transspleißfähigen zellulären RNAs in vivo in der Zelle zu daraus möglicherweise resultierenden RNA-TransspleißProdukten (Hybrid-RNAs) interagieren (s. Fig. 02, bzw. S1).

Konkret ist also nur noch zu untersuchen, ob diese prinzipiell zu einem Transpleiß befähigten 5' Spleißstellen und 3' Spleißstellen auf den verschiedenen zellulären pre-mRNA-Spezies auch in vivo in der lebenden Zelle interagieren, um danach eine transgespleißte Hybrid-mRNA enstehen zu lassen, die also von 2 Genorten abstammt, und die unter Umständen pathogene/maligne Proteine codiert (Fig. O2, S1). Dies geschieht wiederum in einer cDNA-PCR aus der Gesamt-RNA der Zellen, wobei die beiden PCR-Primer in ihrer 18-24 mer Sequenz zum einem (= Primer C in Fig. O2) zu einer beliebigen, zuvor sequenzierten exonischen Sequenz A (s. Fig. S1) des möglichen Transspleißpartners A mit einer potenten 5' Spleißstelle, und zum anderen (= Primer D in Fig. O2) zu einer beliebigen, zuvor sequenzierten (s. Fig. S1) exonischen Sequenz B des möglichen Transpleißpartners B mit einer potenten 3' Spleißstelle 'analog sind. Im Falle, dass mit diesen beiden PCR-Primern ein PCR-Produkt entsteht, ist stringent anzunehmen, dass dieses PCR-Produkt aus einer entsprechend transgespleißten zellulären Hybrid-mRNA abstammt. Durch eine abschließende Sequenzierung des PCR-Produktes (Fig. S5) ist der zelluläre Transspleiß entsprechend zu bestätigen. Sofern mit der Sonden-RNA mit der 5' Spleißstelle X-viele, verschiedene potente 5' Transspleißstellen bzw. zugehörige RNAs als diesbezügliche cDNA-PCR-Produkte, und mit der Sonden-RNA mit der 3' Spleißstelle Y-viele, verschiedene potente 3' Transspleißstellen bzw. zugehörige RNAs als diesbezügliche cDNA-PCR-Produkte identifiziert wurden, sind dann im abschließenden Überprüfungsverfahren alle möglichen Kombinationen von potenten Transspleißstellen auf den verschiedenen zellulären pre-mRNAs zu berücksichtigen; es sind also X mal Y verschiedene PCR-Reaktionen mit den entsprechenden, verschiedenen Primerkombinationen durchzuführen.

Im Falle von nachgewiesenen, natürlichen zellulären RNA-Transspleißprodukten ist sodann die daraus abgeleitete Proteinsequenz zu bestimmen. Über Immunoblots kann dann überprüft werden, ob und in welchem Maße das aus dem Transspleißprodukt hypothetisch hergestellte Hybrid-Protein in vivo auch tatsächlich exprimiert wird. Im letzten Schritt ist dann noch eine mögliche Pathogenität des Hybridproteins zu untersuchen. Hierzu wird im einfachsten Fall etwa eine ds-cDNA, welche die bereits eine fertig transgespleißte Hybrid-mRNA codiert, in gesunde Zellen eingebracht. Aus dieser ds-cDNA bildet die Zelle dann die entsprechende mRNA mit dem zugehörigen Hybrid-Protein, das dann evtl. pathologische Prozesse bzw. eine Tumor-Transformation in der Zelle auslöst, sofern es sich um ein maligne wirkendes Protein handelt.

Die Erfindung betrifft im dritten beschriebenen Anwendungsfall also eine eingebrachte transspleißfähige RNA, die wiederum nur aus einem Exon und einem Outron besteht, und die als RNA-Sonde zur Identifikation zunächst per se transspleißfähiger zellulärer RNAs dient. Die Erfindung betrifft auch das spezielle systematische Nachweisverfahren zur Sequenzermittlung transspleißfähiger zellulärer RNAs und daraus entstandener, zellulärer transgespleißter Hybrid-mRNAs, wie dies im vorangegangenen Abschnitt beschrieben ist.

Die Erfindung im Anwendungsfall drei betrifft also auch einen Kit zur Identifikation von Transspleißstellen auf zellulären RNAs, aus denen ggf. transgespleißte, maligne Hybrid-RNAs nachweisbar sind, wobei der Kit die erfindungsgemäße Sonden-RNAs sowie weitere Primer zur gezielten cDNA-Synthese und PCR-Amplifikation und ein Anweisungsprotokoll umfasst.

Die Sonden-RNA ist eine pre-mRNA mit 150-300 Basen (sofern ein Protein codiert wird mit einigen hundert Basen) Länge, die wiederum eine sehr potente 5' oder 3' Spleißstelle enthält. Die Sonden-RNA weist ferner in einem Unterfall A eine Oligo-U-Region im Outronteil auf, im Unterfall B entfällt diese Region. Die Sonden-RNA bzw. die codierende DNA hat im jeweiligen Outron-Bereich zudem eine 8-12 mer Erkennungsnucleotidfolge für ein selten spaltendes Restriktionsenzym, etwa die Folge AUUU/AAAU auf RNA-Ebene, welche in der aus der RNA hergestellten ds-cDNA durch ein entsprechendes Restriktionsenzym, hier: Swa I, in einem späteren Analyseschritt gespalten wird. Die jene RNA codierende DNA, die in die lebenden, zu analysierenden Zellen eingebracht wird, enthält zudem einen Promotor mit Enhancer-Elementen, ein Polyadenylierungssignal und einen Replikations-Origin.

Die DNA, welche eine Sonden-RNA mit einer 5'-Trans-Spleißstelle codiert, hat folglich folgende Struktur (von 5' nach 3'):
a) Zu Beginn liegt ein Replikationsorigin (z.B. aus SV40), gefolgt von
b) einem starker Promotor mit Enhancer (z.B. aus SV40) (vgl. hierzu z.B. Fig. G1, H1).
c) In der nachfolgenden Sequenz, jedoch mind. 10 Nucleotide upstream der 5' Spleißstelle liegt eine A/G-reiche Region oder eine andere spezielle Region, die als Spleißenhancer-Region dient (s. Fig. 12, dort als RNA-Sequenz) .
d) 40-120 Nucleotide hinter dem Promotorende (Beginn der hieraus codierten RNA) liegt an der Exon/Outron-Grenze die 5' Spleißstelle mit der Sequenz (A/G) -A-G-G-T-A-(A/G) -G-T (s. Fig. Q1, dort als RNA-Sequenz), bzw. mit der bevorzugten Sequenz: A-A-G-G-T-A-A-G-T
e) 10-30 Nucleotide downstream der obigen Sequenz liegt bei dem Typ A dieser DNA eine Folge von 15-18 Thymidin (T) (s. Fig. Q1) ; bei dem Typ B dieser DNA entfällt diese Folge.
f) 10-30 Nucleotide downstream der Poly-T-Folge (DNA-Typ A) bzw. 20-60 Nucleotide downstream der 5' Spleißstelle (DNA-Typ B) liegt eine 8-12 mer Nucleotidfolge zur Erkennung eines selten spaltenden Restriktionsenzyms (etwa die Folge ATTT/AAAT zur Erkennung von Swa I, s Fig. Q1, dort als RNA) .
g) 10-50 Nucleotide hinter dieser Region liegt die Polyadenylierungserkennungsstelle A-A-T-A-A-A gefolgt von einer downstream G/T-reichen Region (2. Polyadenylierungsstelle, z.B. aus SV40), (Fig. G1, H1).

Die DNA, welche eine Sonden-RNA mit einer 3' Trans-Spleißstelle codiert, hat folglich folgende Struktur (von 5' nach 3') :
a) Zu Beginn liegt ein Replikationsorigin (z.B. aus SV40), gefolgt von
b) einem starker Promotor mit Enhancer (z.B. aus SV40) (vgl. hierzu z.B. Fig. Gl, Hl).
c) 20-60 Nucleotide downstream der Promotorregion (RNA-Anfang, mit Cap- Site) liegt bei dem Typ A dieser DNA eine Folge von 15-18 Thymidin (T) (s. Fig. R1) ; bei dem Typ B dieser DNA entfällt diese Folge. I
d) 10-40 Nucleotide downstream der Poly-T-Folge (DNA-Typ A) bzw. 30-100 Nucleotide downstream des Promotorendes (DNA-Typ B) liegt eine 8-12 mer Nucleotidfolge zur Erkennung eines selten spaltenden Restriktionsenzyms (etwa die Folge ATTT/AAAT zur Erkennung von Swa I) (s. Fig. R1, dort als RNA-Sequenz).
e) 10-30 Nucleotide downstream dieser Restriktionsenzymserkennungsfolge liegt die Branch-A-Region der 3' Spleißstelle mit der Folge T- (A/G) - (C/T) -T- (A/G) -A-(C/T) - (A/G) (Fig. R1).
f) 10-30 Nucleotide downstream dieser Branch-A-Folge liegt eine Polypyrimidinbasenfolge aus 15-18 mer T/C gefolgt von dem Spleißstellendinucleotid AG zum Ende des Outrons (Fig. R1).
g) In der nachfolgenden Sequenz ab Beginn des Exons, jedoch mind. 10 Nucleotide downstream des AG der 3' Spleißstelle und mind. 30 Nucleotide upstream der Polyadenylierungsteile liegt eine A/G-reiche Region oder eine andere spezielle. Region, die als Spleißenhancer-Region dient (Fig. J2, dort als RNA- Sequenz).
h) Die nachfolgende Sequenz ab Beginn des Exons kann zudem als cDNA für ein kurzes, in situ leicht nachweisbares unvollständiges Protein codieren, dessen Sequenz etwa ab Aminosäure 2 im Exon beginnt, und das durch eine Terminationssignal (TAG etc.) abgeschlossen wird, welches jedoch den Translations-Startcodon ATG nicht enthält.
i) 80-140 Nucleotide bzw. , sofern ein unvollständiges Protein codiert wird, einige hundert Nucleotide hinter dem Spleißstellendinucleotid AG liegt die primäre Poly-adenylierungsstelle A-A-T-A-A-A gefolgt von einer downstream G/T-reichen Region (zweite Polyadenylierungstelle, z.B. aus SV40) (vgl. hierzu z.B. Fig. G1, H1).

Die Erfindung für den Anwendungsfall 2 soll anhand eines Beispiels näher erläutert werden.

### Beispiel

Transspleiß zwischen einer künstlich geschaffenen, unvollständigen RNA, codiernd für das HSV-TK-Protein, und einer Alpha-Feto-Protein-RNA (AFP-RNA) aus Tumorzellen zur selektiven Abtötung dieser Tumorzellen:

Die HSV-TK-Protein-codiernde RNA wird zunächst dadurch in den Zellen erzeugt, dass eine entsprechende DNA in alle Zellen (Tumorzellen und Normalzellen) eingebracht wird, welche jene RNA abschreibt. Um eine stake Transskription zu erreichen, ethält diese DNA wiederum einen starken Promotor mit ensprechenden Enhancerregionen z.B. aus SV40 (s. Fig. K 1). Damit eine am 3 '-Ende stabile pre-mRNA erzeugt wird, enthält diese DNA ferner eine stake Polyadenylierungserkennungssteile (etwa auch aus SV40) mit einer AATAAA-Region upstream und einer Poly-GT-region downstream der Polyadenylierungs-Cleavage-Site (s. Fig. K1).

Diese DNA enthält ferner in ihrem wesentlichen hinteren Teil die komplette DNA-Folge, welche ab Aminosäure 2 des Zelltod-Proteins codiert. Diese DNA-Sequenz wird dabei über eine cDNA-Folge, abgeleitet aus der mRNA, erhalten (s. Fig. K1). Im Falle des HSV-TK-DNA-Konstuktes ist dies die codierende Nucleotid-Folge für die Aminosäure 2 (= Ala) bis zur letzten Aminosäure 376 (= Val), auf welche das Terminationssignal UAG bzw. TAG folgt (s. Fig. Ml).

Upstream zur ab Aminosäure 2 der HSV-TK-Proteins codierenden Region liegt auf dieser DNA eine Region, welche für einen Peptidstretch codiert (Protease-Linker), der durch eine zellulär immer vorhandene spezifische Protease gespalten wird. Diese Folge ist je nach Proteasentyp bis zu 15 Aminosäuren lang, wird also durch bis zu 45 Nucleotide auf der DNA codiert (s. Fig. M1).

Upstream dieser Protease-Linker-Region liegt unmittelbar am Exonrand der 3 ' Spleißstelle noch ggf. ein Reading-Frame-Linker, bestehend aus 1 oder 2 Nucleotiden, um einen Frameshift zur translatierten Nucleotidfolge aus dem transgespleißten codierenden Exon 1 der Tumor-spezifischen RNA auszugleichen. Bei einem Transspleiß zum Exon 1 des AFP, wie in diesem Beispielfall, ist hier eine Korrekur um 2 Nucleotide über 2 zusätzliche Basen (hier wurde CU bzw. CT gewählt) notwendig (s. Fig. M1). Im Falle eines Transspleißes hat die transgespleißte RNA in ihrer Spleiß-Verbindungstelle in diesem speziellen Fall dann die Folge GCU (codierend für Ala) (s. Fig. M2), so dass eine Frameshift durch ein "überzähliges" Einzelnucleotid (hier ein G) aus dem Exon 1 der AFP-RNA ausgeglichen wird.

Oberhalb des Reading-frame-Linkers liegt der Outronbereich, welcher mit der 3' Spleißstelle abschließt. Diese 3' Spleißstelle soll wiederum eine sehr starke Spleißkompetenz haben. Dies bedeutet das Vorhandensein eines ausgeprägten Polypyrimidinbasen- (U/C bzw. T/C) Stretches unmittelbar vor dem Spleißstellendinucleotid AG, sowie etwa 10-30 Nucleotide upstream davon eine starke Branch-A-Region (nicht eingezeichnet in Fig. M1, vgl. Fig. J2).

Eine Transspleißreaktion von der singulären, künstlichen 3' Spleißstelle" auf der HSV-TK-pre-mRNA spezifisch und selektiv zur 5' Spleißstelle des Exons 1 der AFP-pre-mRNA wird dadurch erreicht, dass diese HSV-TK-pre-mRNA im Outron-Bereich oberhalb der Branch-A-Region wegen der geforderten Substratspezifität eine Folge von mindestens 18 Basen aufweist, die spezifisch zur AFP-pre-mRNA über Antisensstrukturen paart. Hierdurch wird eine feste Bindung zwischen beiden RNA-Molekülen eingegangen, wodurch die Transspleißeffizienz um mehrere Zehnerpotenzen erhöht wird. Diese RNA-RNA-Hybridisierung sollte zudem so liegen, dass hierdurch die dem Exon 1 nachfolgende 3 ' -Spleißstelle im Poly-U/C-Bereich und im AG-Bereich (gelegen an der Grenze zum Exon 2 hin) auf der AFP-pre-mRNA blockiert wird (s. Fig. M1). Hierdurch wird die Transpleißeffizienz nochmals erhöht, da eine konkurrierende Cis-Spleißreaktion zwischen Exon 1 und Exon 2 der AFP-RNA dann nicht mehr möglich ist.

Durch den Transspleiß zwischen dem Exon 1 der AFP-RNA der Tumorzellen und dem Exonanteil der unvollständigen, künstlich erzeugten Zelltod-RNA (= HSV-TK-RNA) wird schließlich eine mRNA erzeugt, die ein AUG-Startcodon im passenden, richtigen Leserahmen zur Aminosäure 2 (= Ala) der HSV-TK-RNA trägt. Das nach Ablesen der transgespleißten RNA (s. Fig. M2) hergestellte Hybridprotein enthält schließlich in diesem speziellen Beispielfall die Aminsäuren Met 1 bis Ile 28 aus der AFP-RNA, gefolgt von 3 Nucleotiden (GCU) in der Spleiß-Verbindungstelle, die letztlich für den Leserahmenausgleich sorgen, und gefolgt von einer Region aus bis zu 15 Aminosäuren, welche eine spezifische Proteaseerkennungsregion trägt. An diese Region schließt sich dann die eigentliche HSV-TK-Protein-Region (von Aminosäure 2 = Ala, bis Aminosäure 376 = Val) an. Das fertigte Hybridprotein wird dann in der spezifischen Protease-Erkennungsregion durch eine zelluläre Protease gespalten (s. Fig. M3), um eventuell bei der HSV-TK-Enzymfunktion störende fremde Proteinanteile (aus dem AFP-Anteil) abzuspalten. Das HSV-TK-Enzym liegt dann nur in den Krebszellen, nicht aber in den gesunden Zellen vor. (Nur in den Krebszellen ist ein entsprechender Transspleiß zur der nur hier vorliegenden AFP-RNA möglich, der letztlich dieses Protein liefert) . Dieses Enzym setzt dann in den Krebszellen das zugeführte Medikament Ganciclovir in eine phosphoryliertes Derivat um, wodurch hier letztlich der Abbruch der gesamten DNA-Replikation der Krebszelle erreicht wird, so dass diese abstirbt.

## Patentansprüche

1. DNA zu selektiven Abtöten von Tumorzellen in einer Zellpopulation, wobei diese DNA nach Einbringung in eine Zellpopulation dort für eine künstlich verkürzte Zelltod-pre-mRNA codiert, wobei diese Zelltod-pre-mRNA selektiv mittels der nur in diesen Tumorzellen vorkommenden pre-RNA-Molekülen, die ein AUG-Codon als Translationsstart enthalten, und der natürlich vorkommenden Spleißkomponenten über einen RNA-Transspleiß N-terminal verlängert und so eine vollständige Zelltod-mRNA erhalten wird, und hierdurch ein vollständiges Zelltod-Protein codiert wird, das Tumorzellen auf direktem oder indirektem Wege selektiv abtötet,
**dadurch gekennzeichnet,**
**dass** bei der verkürzten Zelltod-pre-mRNA ein vorderes 5'Outron und ein Exon umfasst wobei das 5' Ende des Exons eine Frameshift-Sequenz aus 1 oder 2 Nucleotiden aufweist.

2. Modifizierte Zelltod-RNA codierende DNA nach Anspruch 1,
**dadurch gekennzeichnet,**
**dass** die verkürzte Zelltod-pre-mRNA ein vorderes 5' Outron und ein Exon umfasst, wobei das Exon im distalen Teil eine Nucleotidfolge als cDNA aufweist, die ab Aminosäure zwei bis zur letzten Aminosäure für das Zelltod-Protein codiert.

3. Modifizierte Zelltod-RNA codierende DNA nach Anspruch 1 oder 2,
**dadurch gekennzeichnet,**
**dass** bei der verkürzten Zelltod-pre-mRNA das Exon hinter der Frameshift-Nucleotidfolge und vor der Nucleotidfolge ab Aminosäure 2 des Zelltodproteins eine Nucleotidfolge einer Protease-Erkennungsregion umfasst, die für eine Peptidfolge codiert, die durch zelluläre Proteasen gespalten werden kann.

4. Modifizierte Zelltod-RNA codierende DNA nach einem der vorhergehenden Ansprüche,
**dadurch gekennzeichnet,**
**dass** bei der verkürzten Zelltod-pre-mRNA das Exon eine Exonische Spleiß-Enhancer (ESE)-Sequenz im Bereich der Proteaseerkennungsregion und/oder im Bereich der ab Aminosäure 2 codierenden Region des Zelltodproteins aufweist, ohne dass durch diese ESE-Sequenz(en) jedoch zusätzliche oder andere Aminosäuren codiert werden.

5. Modifizierte Zelltod-RNA codierende DNA nach einem der vorhergehenden Ansprüche,
**dadurch gekennzeichnet,**
**dass** bei der verkürzten Zelltod-pre-mRNA das Outron in dem distalen Bereich zum Exon hin eine 3' Spleißstelle bestehend aus einer Branch-A-Stelle, einen Poly-Pyrimindinbasen-Stretch und einem AG-Dinucleotid an der Grenze zum Exon hin aufweist.

6. Modifizierte Zelltod-RNA codierende DNA nach einem der vorhergehenden Ansprüche,
**dadurch gekennzeichnet,**
**dass** bei der verkürzten Zelltod-pre-mRNA die Branch-A-Stelle der 3' Spleißstelle die 8-mer Sequenz U-A/G-C/U-U-A/G-A-C/U-A/G und der Poly-Pyrimidinbasen-Stretch die Sequenz von 15-18 mer (U/C) aufweist.

7. Modifizierte Zelltod-RNA codierende DNA nach einem der vorhergehenden Ansprüche,
**dadurch gekennzeichnet,**
**dass** die verkürzte Zelltod-pre-mRNA im Outron oberhalb ihrer 3' Spleißstelle eine Antisenssequenz von mindestens 18 Nucleotiden umfasst, die in Antisens spezifisch zu einer speziellen Tumor-pre-mRNA paaren.

8. Modifizierte Zelltod-RNA codierende DNA nach einem der vorhergehenden Ansprüche,
**dadurch gekennzeichnet,**
**dass** bei der verkürzten Zelltod-pre-mRNA die Antisenssequenz im 5' Outron zur spezifischen Tumorzellen-pre-mRNA in deren Polypryrimidinbasenfolge der 3' Spleißstelle up-stream des zweiten codierenden Exons dieser RNA paart.

9. Modifizierte Zelltod-RNA codierende DNA nach einem der vorhergehenden Ansprüche,
**dadurch gekennzeichnet,**
**dass** bei der verkürzten Zelltod-pre-mRNA das Outron einen Translationsstart AUG aufweist, der nicht im Leserahmen ab Aminosäure 2 des Zelltodproteins des Exons liegt, und der für ein kurzes Nonsensprotein initiiert.

## Claims

1. DNA for selective killing of tumor cells in a cell population, said DNA coding for an artificially shortened cell death m-RNA after being introduced into a cell population, said cell death pre-mRNA being extended selectively at the N terminus via trans-splicing by means of RNA components occurring only in the tumor cells and including an AUG codon as start of translation, and by means of naturally occurring splicing components, thus obtaining a complete cell death pre-mRNA and encoding a complete cell death protein that selectively destroys the tumor cells by a direct or indirect route,
**characterized in that**
the shortened cell death pre-mRNA comprises an anterior 5' outron and an exon, wherein the 5' end of the exon has a frameshift sequence of 0, 1 or 2 nucleotides.

2. The modified cell death RNA-coding DNA according to Claim 1,
**characterized in that**
the shortened cell death pre-mRNA comprises an anterior 5' outron and an exon, wherein the distal portion of the exon has a nucleotide sequence as cDNA that encodes from amino acid 2 to the last amino acid of the cell death protein.

3. The modified cell death RNA-coding DNA according to claim 1 or claim 2,
**characterized in that**
in the shortened cell death pre-mRNA, the exon downstream of the frameshift nucleotide sequence and upstream of the nucleotide sequence from amino acid 2 of the cell death protein on comprises a nucleotide sequence of a protease recognition region encoding a peptide sequence that can be cleaved by naturally occurring cellular proteases.

4. The modified cell death RNA-coding DNA according to any one of the foregoing claims,
**characterized in that**
in the shortened cell death pre-mRNA, the exon has an exonic splice enhancer (ESE) sequence in the region of the protease recognition region and/or in the region of the cell death protein region encoding from amino acid 2 on, with no additional or other amino acids being coded for by said ESE sequence(s).

5. The modified cell death RNA-coding DNA according to any one of the foregoing claims,
**characterized in that**
in the shortened cell death pre-mRNA, the outron in the distal region towards the exon has a 3' splice site comprising a branch A site, a polypyrimidine base stretch, and an AG dinucleotide at the border to the exon.

6. The modified cell death RNA-coding DNA according to any one of the foregoing claims,
**characterized in that**
in the shortened cell death pre-mRNA the branch A site of the 3' splice site has the 8-mer sequence U-A/G-C/U-U-A/G-A-C/U-A/G and the polypyrimidine base stretch has the sequence of 15 to 18-mer (U/C).

7. The modified cell death RNA-coding DNA according to any one of the foregoing claims,
**characterized in that**
the shortened cell death pre-mRNA in the outron upstream of the 3' splice site thereof comprises an antisense sequence of at least 18 nucleotides that undergo specific antisense pairing to a particular tumor pre-mRNA.

8. The modified cell death RNA-coding DNA according to any one of the foregoing claims,
**characterized in that**
the antisense sequence in the 5' outron of the shortened cell death pre-mRNA pairs to the specific tumor cell pre-mRNA in its polypyrimidine base sequence of the 3' splice site upstream from the second coding exon of this RNA.

9. The modified cell death RNA-coding DNA according to any one of the foregoing claims,
**characterized in that**
in the shortened cell death pre-mRNA the outron has an AUG translation start that is not in the reading frame of the exon for the cell death protein from amino acid 2 on, and which initiates a short nonsense protein.

## Revendications

1. ADN pour la destruction sélective de cellules tumorales dans une population de cellules, dans lequel cet ADN, après l'injection dans la population de cellules, y code un ARN pré-messager apoptotique artificiellement raccourci, sachant que cet ARN pré-messager apoptotique est prolongé de manière sélective au moyen des molécules ARN pré-messagères existant exclusivement dans ces cellules tumorales, qui contiennent un codon AUG en tant qu'initiation de la traduction, et des composants d'épissage existant de manière naturelle via un trans-épissage ARN N-terminal et un ARN messager apoptotique intégral est obtenu, et une protéine apoptotique intégrale est de ce fait codée, qui tue les cellules tumorales de manière sélective directe ou indirecte,
**caractérisé en ce que**
l'ARN pré-messager apoptotique raccourci contient un outron en 5' avant et un exon, sachant que l'extrémité en 5' de l'exon présente une séquence de décalage de cadre de lecture composée de 1 ou 2 nucléotides.

2. ADN modifié codant un ARN apoptotique selon la revendication 1,
**caractérisé en ce que**
l'ARN pré-messager apoptotique raccourci comprend un outron en 5' avant et un exon, sachant que l'exon, dans la partie distale, présente une suite de nucléotides en tant que ADNc, qui, à partir de l'aminoacide deux jusqu'au dernier aminoacide, code la protéine apoptotique.

3. ADN modifié codant un ARN apoptotique selon la revendication 1 ou 2,
**caractérisé en ce que**
dans l'ARN pré-messager apoptotique raccourci, l'exon comprend derrière la suite de nucléotides de décalage de cadre de lecture et avant la suite de nucléotides à partir de l'aminoacide 2 de la protéine apoptotique une suite de nucléotides d'un site de reconnaissance de protéase, qui code une suite de peptides, qui peut être dissociée par des protéases cellulaires.

4. ADN modifié codant un ARN apoptotique selon l'une des revendications précédentes,
**caractérisé en ce que**
dans l'ARN pré-messager apoptotique raccourci, l'exon présente une séquence d'accélérateurs exoniques d'épissage (ESE) dans la zone du site de reconnaissance de protéase et/ou dans la zone du site de la protéine apoptotique codant à partir de l'aminoacide 2, sans toutefois que cette/ces séquence(s) ESE code/codent des aminoacides supplémentaires ou différents.

5. ADN modifié codant un ARN apoptotique selon l'une des revendications précédentes,
**caractérisé en ce que**
dans l'ARN pré-messager apoptotique raccourci, l'outron dans la zone distale vers l'exon présente un site d'épissage en 3' composé d'un site branche A, d'un stretch de bases de polypyrimidine et d'un dinucléotide AG à la limite de l'exon.

6. ADN modifié codant un ARN apoptotique selon l'une des revendications précédentes,
**caractérisé en ce que**
dans l'ARN pré-messager apoptotique raccourci, le site branche A du site d'épissage en 3' présente la séquence 8-mer U-A/G-C/U-U-A/G-A-C/U-A/G et le stretch de bases de polypyrimidine la séquence de 15-18 mer (U/C).

7. ADN modifié codant un ARN apoptotique selon l'une des revendications précédentes,
**caractérisé en ce que**
l'ARN pré-messager apoptotique raccourci, dans l'outron au-dessus de son site d'épissage en 3', comprend une séquence anti-sens d'au moins 18 nucléotides, qui s'apparient en anti-sens spécifique à un ARN pré-messager tumoral spécial.

8. ADN modifié codant un ARN apoptotique selon l'une des revendications précédentes,
**caractérisé en ce que**
dans l'ARN pré-messager apoptotique raccourci, la séquence anti-sens dans l'outron en 5' s'apparie en ARN pré-messager de cellules tumorales dans leur suite de bases de polypyrimidine du site d'épissage en 3' en amont du deuxième exon codant de cet ARN.

9. ADN modifié codant un ARN apoptotique selon l'une des revendications précédentes,
**caractérisé en ce que**
dans l'ARN pré-messager apoptotique raccourci, l'outron présente une initiation de traduction AUG, qui n'est pas située dans le cadre de lecture à partir de l'aminoacide 2 de la protéine apoptotique de l'exon, et qui initie une courte protéine non-sens courte.
